# EUROPEAN PATENT APPLICATION

(11) **EP 3 095 792 A1**
(43) Date of publication of application: **23.11.2016**
(21) Application number: 15168203.6
(22) Date of filing: 19.05.2015
(51) Int. Cl.: C07K 14/725

(54) **T CELL RECEPTOR WITH SPECIFICITY FOR MYELOPEROXIDASE PEPTIDE AND USES THEREOF**

(71) Applicant: Klinikum rechts der Isar der Technischen Universität München, 81675 München (DE)
(72) Inventor: Krackhardt, Angela, 81675 Munich (DE); Klar, Richard, 81541 München (DE)
(74) Representative: Engelhard, Markus

(57) **Abstract**

The present invention relates to T cell receptors (TCRs) with specificity for a myeloperoxidase peptide. The present invention further relates to soluble TCR constructs, chimeric TCRs, bi-specific antibodies, nucleic acids, expression constructs and cells comprising said TCRs or TCR constructs. Moreover, the present invention relates to pharmaceutical compositions comprising such TCRs, soluble TCR constructs, chimeric TCRs, bi-specific antibodies, nucleic acids, expression constructs and/or cells. The present invention further relates to the use of such TCRs, soluble TCR constructs, chimeric TCRs, bi-specific antibodies, nucleic acids or expression constructs for generating genetically modified lymphocytes and to their use as medicaments, preferably in the detection, diagnosis, prognosis, prevention and/or treatment of a hematological neoplasia involving the expression of myeloperoxidase, preferably a leukemia involving the expression of myeloperoxidase. The present invention further relates to methods of detecting, diagnosing, prognosing, preventing and/or treating a hematological neoplasia involving the expression of myeloperoxidase, preferably a leukemia involving the expression of myeloperoxidase.

SEQUENCE LISTING

<110>  Klinikum rechts der Isar der Technischen Universität
       München

<120>  T cell receptor with specificity for myeloperoxidase peptide and
       uses thereof

<130>  U30643EP

<160>  13

<170>  PatentIn version 3.5

<210>  1
<211>  6
<212>  PRT
<213>  Homo sapiens

<400>  1

Tyr Gly Ala Thr Pro Tyr
1               5


<210>  2
<211>  8
<212>  PRT
<213>  Homo sapiens

<400>  2

Tyr Phe Ser Gly Asp Thr Leu Val
1               5


<210>  3
<211>  12
<212>  PRT
<213>  Homo sapiens

<400>  3

Cys Ala Gly Arg Ala Ala Gly Asn Lys Leu Thr Phe
1               5                   10


<210>  4
<211>  5
<212>  PRT
<213>  Homo sapiens

<400>  4

Met Asn His Glu Tyr
1               5


<210>  5
<211>  6
<212>  PRT
<213>  Homo sapiens

<400>  5

Ser Val Gly Glu Gly Thr
1               5


<210>  6
<211>  13
<212>  PRT
<213>  Homo sapiens

<400>  6

Cys Ala Ser Ser Tyr Ser Ser Gly Gln Pro Gln His Phe
1               5                   10


<210>  7
<211>  9
<212>  PRT
<213>  Homo sapiens

<400>  7

Asn Pro Arg Trp Asp Gly Glu Arg Leu
1               5


<210>  8
<211>  267
<212>  PRT
<213>  Artificial Sequence

<220>
<223>  Recombinant T cell receptor alpha-chain sequence

<400>  8

Leu Leu Glu Leu Ile Pro Leu Leu Gly Ile His Phe Val Leu Arg Thr
1               5                   10                  15

Ala Arg Ala Gln Ser Val Thr Gln Pro Asp Ile His Ile Thr Val Ser
            20                  25                  30

Glu Gly Ala Ser Leu Glu Leu Arg Cys Asn Tyr Ser Tyr Gly Ala Thr
        35                  40                  45

Pro Tyr Leu Phe Trp Tyr Val Gln Ser Pro Gly Gln Gly Leu Gln Leu
    50                  55                  60

Leu Leu Lys Tyr Phe Ser Gly Asp Thr Leu Val Gln Gly Ile Lys Gly
65                  70                  75                  80

Phe Glu Ala Glu Phe Lys Arg Ser Gln Ser Ser Phe Asn Leu Arg Lys
                85                  90                  95

Pro Ser Val His Trp Ser Asp Ala Ala Glu Tyr Phe Cys Ala Gly Arg
            100                 105                 110

Ala Ala Gly Asn Lys Leu Thr Phe Gly Gly Gly Thr Arg Val Leu Val
        115                 120                 125

Lys Pro Asn Ile Gln Asn Pro Glu Pro Ala Val Tyr Gln Leu Lys Asp
    130                 135                 140

Pro Arg Ser Gln Asp Ser Thr Leu Cys Leu Phe Thr Asp Phe Asp Ser
145                 150                 155                 160

Gln Ile Asn Val Pro Lys Thr Met Glu Ser Gly Thr Phe Ile Thr Asp
                165                 170                 175

Lys Thr Val Leu Asp Met Lys Ala Met Asp Ser Lys Ser Asn Gly Ala
            180                 185                 190

Ile Ala Trp Ser Asn Gln Thr Ser Phe Thr Cys Gln Asp Ile Phe Lys
        195                 200                 205

Glu Thr Asn Ala Cys Tyr Pro Ser Ser Asp Val Pro Cys Asp Ala Thr
    210                 215                 220

Leu Thr Glu Lys Ser Phe Glu Thr Asp Met Asn Leu Asn Phe Gln Asn
225                 230                 235                 240

Leu Ser Val Met Gly Leu Arg Ile Leu Leu Leu Lys Val Ala Gly Phe
                245                 250                 255

Asn Leu Leu Met Thr Leu Arg Leu Trp Ser Ser
            260                 265

<210> 9
<211> 303
<212> PRT
<213> Artificial Sequence

<220>
<223> Recombinant T cell receptor beta-chain sequence

<400> 9

Ser Leu Gly Leu Leu Cys Cys Gly Val Phe Ser Leu Leu Trp Ala Gly
1               5                   10                  15

Pro Val Asn Ala Gly Val Thr Gln Thr Pro Lys Phe Arg Val Leu Lys
            20                  25                  30

Thr Gly Gln Ser Met Thr Leu Leu Cys Ala Gln Asp Met Asn His Glu

35                  40                  45

Tyr Met Tyr Trp Tyr Arg Gln Asp Pro Gly Met Gly Leu Arg Leu Ile
    50                  55                  60

His Tyr Ser Val Gly Glu Gly Thr Thr Ala Lys Gly Glu Val Pro Asp
65                  70                  75                  80

Gly Tyr Asn Val Ser Arg Leu Lys Lys Gln Asn Phe Leu Leu Gly Leu
                85                  90                  95

Glu Ser Ala Ala Pro Ser Gln Thr Ser Val Tyr Phe Cys Ala Ser Ser
            100                 105                 110

Tyr Ser Ser Gly Gln Pro Gln His Phe Gly Asp Gly Thr Arg Leu Ser
        115                 120                 125

Ile Leu Glu Asp Leu Arg Asn Val Thr Pro Pro Lys Val Ser Leu Phe
    130                 135                 140

Glu Pro Ser Lys Ala Glu Ile Ala Asn Lys Gln Lys Ala Thr Leu Val
145                 150                 155                 160

Cys Leu Ala Arg Gly Phe Phe Pro Asp His Val Glu Leu Ser Trp Trp
                165                 170                 175

Val Asn Gly Lys Glu Val His Ser Gly Val Ser Thr Asp Pro Gln Ala
            180                 185                 190

Tyr Lys Glu Ser Asn Tyr Ser Tyr Cys Leu Ser Ser Arg Leu Arg Val
        195                 200                 205

Cys Ala Thr Phe Trp His Asn Pro Arg Asn His Phe Arg Cys Gln Val
    210                 215                 220

Gln Phe His Gly Leu Ser Glu Glu Asp Lys Trp Pro Glu Gly Ser Pro
225                 230                 235                 240

Lys Pro Val Thr Gln Asn Ile Ser Ala Glu Ala Trp Gly Arg Ala Asp
                245                 250                 255

Cys Gly Ile Thr Ser Ala Ser Tyr His Gln Gly Val Leu Ser Ala Thr
            260                 265                 270

Ile Leu Tyr Glu Ile Leu Leu Gly Lys Ala Thr Leu Tyr Ala Val Leu
        275                 280                 285

Val Ser Gly Leu Val Leu Met Ala Met Val Lys Lys Lys Asn Ser
    290                 295                 300

<210>  10
<211>  801
<212>  DNA
<213>  Artificial Sequence

<220>
<223>  T cell receptor alpha-chain

<400>  10
ctgctggaac tgatccccct gctgggaatc cacttcgtgc tgaggacagc cagagcccag      60

tctgtgaccc agcccgacat ccacatcacc gtgtctgagg gcgccagcct ggaactgcgg     120

tgcaactact cttacggcgc caccccctac ctgttttggt acgtgcagag ccccggacag     180

ggactgcagc tgctgctgaa gtactttagc ggcgacaccc tggtgcaggg catcaaggga     240

ttcgaggccg agttcaagcg gagccagagc agcttcaacc tgcggaagcc ctccgtgcat     300

tggagcgacg ccgccgagta tttctgtgcc ggcagagcag ccggcaacaa gctgacattt     360

ggcggcggaa ccagggtgct cgtgaagccc aacatccaga accccgagcc cgccgtgtac     420

cagctgaagg accctagaag ccaggacagc accctgtgcc tgttcaccga cttcgactcc     480

cagatcaacg tgcccaagac catggaaagc ggcaccttca tcaccgacaa gaccgtgctg     540

gacatgaagg ccatggacag caagagcaac ggcgccattg cctggtccaa tcagaccagc     600

ttcacatgcc aggacatctt caaagagaca aacgcctgct accccagcag cgacgtgccc     660

tgtgatgcca ccctgaccga gaagtccttc gagacagaca tgaatctgaa cttccagaac     720

ctgagcgtga tgggcctgag aatcctgctg ctgaaagtgg ccggcttcaa tctgctgatg     780

accctgagac tgtggtccag c                                               801

<210>  11
<211>  807
<212>  DNA
<213>  Artificial Sequence

<220>
<223>  T cell receptor alpha-chain with start and stop codon

<400>  11
atgctgctgg aactgatccc cctgctggga atccacttcg tgctgaggac agccagagcc      60

cagtctgtga cccagcccga catccacatc accgtgtctg agggcgccag cctggaactg     120

cggtgcaact actcttacgg cgccaccccc tacctgtttt ggtacgtgca gagccccgga     180

cagggactgc agctgctgct gaagtacttt agcggcgaca ccctggtgca gggcatcaag     240

ggattcgagg ccgagttcaa gcggagccag agcagcttca acctgcggaa gccctccgtg     300

cattggagcg acgccgccga gtatttctgt gccggcagag cagccggcaa caagctgaca     360

tttggcggcg gaaccagggt gctcgtgaag cccaacatcc agaaccccga gcccgccgtg 420

taccagctga aggaccctag aagccaggac agcaccctgt gcctgttcac cgacttcgac 480

tcccagatca acgtgcccaa gaccatggaa agcggcacct tcatcaccga caagaccgtg 540

ctggacatga aggccatgga cagcaagagc aacggcgcca ttgcctggtc caatcagacc 600

agcttcacat gccaggacat cttcaaagag acaaacgcct gctaccccag cagcgacgtg 660

ccctgtgatg ccaccctgac cgagaagtcc ttcgagacag acatgaatct gaacttccag 720

aacctgagcg tgatgggcct gagaatcctg ctgctgaaag tggccggctt caatctgctg 780

atgaccctga gactgtggtc cagctga 807

<210> 12
<211> 909
<212> DNA
<213> Artificial Sequence

<220>
<223> T cell receptor beta-chain

<400> 12
tctctgggac tgctgtgttg cggcgtgttc agcctgctgt gggccggacc tgtgaatgcc 60

ggcgtgaccc agacccccaa gttccgggtg ctgaaaaccg gccagagcat gaccctgctg 120

tgcgcccagg acatgaacca cgagtacatg tattggtaca gacaggaccc cggcatgggc 180

ctgcggctga tccactattc tgtgggcgag ggcaccaccg ccaagggcga agtgcctgat 240

ggctacaacg tgtcccggct gaagaagcag aacttcctgc tgggcctgga aagcgccgct 300

cctagccaga ccagcgtgta cttctgcgcc agcagctaca gcagcggcca gcctcagcac 360

tttggcgacg gcaccagact gagcatcctg gaagatctgc ggaacgtgac cccccccaag 420

gtgtccctgt tcgagcctag caaggccgag atcgccaaca agcagaaagc caccctcgtg 480

tgcctggcca gaggcttctt ccccgaccac gtggaactgt cttggtgggt caacggcaaa 540

gaggtgcaca gcggcgtgtc caccgatccc caggcctaca aagagagcaa ctacagctac 600

tgcctgtcca gcagactgag agtgtgcgcc accttctggc acaacccccg gaaccacttc 660

agatgccagg tgcagtttca cggcctgagc gaagaggaca agtggcccga gggcagccct 720

aagcccgtga cacagaatat cagcgccgaa gcctggggca gagccgactg tggaatcacc 780

agcgccagct accatcaggg cgtgctgagc gccaccatcc tgtacgagat tctgctgggc 840

aaggccaccc tgtacgccgt gctggtgtct ggcctggtgc tgatggccat ggtcaagaag 900

aagaacagc 909

<210> 13
<211> 915
<212> DNA
<213> Artificial Sequence

<220>
<223>  T cell receptor beta-chain with start and stop codon

<400>  13
atgtctctgg gactgctgtg ttgcggcgtg ttcagcctgc tgtgggccgg acctgtgaat        60

gccggcgtga cccagacccc caagttccgg gtgctgaaaa ccggccagag catgaccctg       120

ctgtgcgccc aggacatgaa ccacgagtac atgtattggt acagacagga ccccggcatg       180

ggcctgcggc tgatccacta ttctgtgggc gagggcacca ccgccaaggg cgaagtgcct       240

gatggctaca acgtgtcccg gctgaagaag cagaacttcc tgctgggcct ggaaagcgcc       300

gctcctagcc agaccagcgt gtacttctgc gccagcagct acagcagcgg ccagcctcag       360

cactttggcg acggcaccag actgagcatc ctggaagatc tgcggaacgt gacccccccc       420

aaggtgtccc tgttcgagcc tagcaaggcc gagatcgcca acaagcagaa agccaccctc       480

gtgtgcctgg ccagaggctt cttccccgac cacgtggaac tgtcttggtg ggtcaacggc       540

aaagaggtgc acagcggcgt gtccaccgat ccccaggcct acaaagagag caactacagc       600

tactgcctgt ccagcagact gagagtgtgc gccaccttct ggcacaaccc ccggaaccac       660

ttcagatgcc aggtgcagtt tcacggcctg agcgaagagg acaagtggcc cgagggcagc       720

cctaagcccg tgacacagaa tatcagcgcc gaagcctggg gcagagccga ctgtggaatc       780

accagcgcca gctaccatca gggcgtgctg agcgccacca tcctgtacga gattctgctg       840

ggcaaggcca ccctgtacgc cgtgctggtg tctggcctgg tgctgatggc catggtcaag       900

aagaagaaca gctga                                                        915

7

## Description

The present invention relates to T cell receptors (TCRs) with specificity for a peptide derived from the protein myeloperoxidase. The present invention further relates to soluble TCR constructs, chimeric TCRs, bi-specific antibodies, nucleic acids, expression constructs and cells comprising said TCRs or TCR constructs. Moreover, the present invention relates to pharmaceutical compositions comprising such TCRs, soluble TCR constructs, chimeric TCRs, bi-specific antibodies, nucleic acids, expression constructs and/or cells. The present invention further relates to the use of such TCRs, soluble TCR constructs, chimeric TCRs, bi-specific antibodies, nucleic acids or expression constructs for generating genetically modified lymphocytes and to their use as medicaments, preferably in the detection, diagnosis, prognosis, prevention and/or treatment of a hematological neoplasia involving the expression of myeloperoxidase, preferably a leukemia involving the expression of myeloperoxidase. The present invention further relates to methods of detecting, diagnosing, prognosing, preventing and/or treating a hematological neoplasia involving the expression of myeloperoxidase, preferably a leukemia involving the expression of myeloperoxidase.

Leukemias, and in particular myeloid neoplasias, are often very aggressive and difficult to treat diseases with high morbidity and mortality. While in recent years significant progress has been made, there is still an urgent need in the art for improved reagents and methods for diagnosing, preventing and treating such leukemias, in particular myeloproliferative neoplasia (MPN), acute myeloid leukemia (AML), chronic myeloid leukemia (CML), acute lymphoblastic leukemia (ALL), myelodysplastic syndrome and/or myeloproliferative syndrome.

In patients with myeloid leukemia having a high-risk constellation, allogeneic stem cell transplantation is often the only promising treatment option. However, even after allogeneic stem cell transplantation relapse is a common problem. Remarkably, the leukemic cells arising during such relapses can be targeted and often eradicated by T cells. A direct proof of principle of the efficacy of T cells in such treatment situations are the durable remissions induced by the administration of donor lymphocyte infusions to patients with acute and chronic myeloid leukemia (AML and CML) who have relapsed after allogeneic stem cell transplantation (SCT). Such graft versus leukemia (GvL) effects are, however, unpredictable and do not occur in all patients. Moreover, frequently the GvL effects are associated with graft versus host disease (GvHD), and currently little is known about how to target favorable structures for a GvL effect without GvHD.

The advent of technology to redirect T cells to defined leukemia antigens by expression of a transgenic T cell receptor on said T cells offers new opportunities for manipulating the GvL effect, such as by providing leukemia-reactive T cells directed against suitable targets on leukemic cells for patients that lack GvL effects or cannot undergo allogeneic SCT.

It is therefore an object of the present invention to provide for improved means and methods for detecting, preventing and/or treating hematological neoplasia, in particular leukemia, such as myeloproliferative neoplasia (MPN), acute myeloid leukemia (AML), chronic myeloid leukemia (CML), acute lymphoblastic leukemia (ALL), myelodysplastic syndrome and/or myeloproliferative syndrome, in particular via T cells specific for leukemia antigens or by immunotherapy. In particular, it is an object of the present invention to provide for improved means and methods for preventing/treating relapses in patients after treatment of hematological neoplasia, in particular leukemia (such as myeloproliferative neoplasia (MPN), acute myeloid leukemia (AML), chronic myeloid leukemia (CML)), acute lymphoblastic leukemia (ALL), myelodysplastic syndrome and/or myeloproliferative syndrome, preferably after a treatment involving (allogeneic) stem cell transplantation.

These objects are solved by the below-described aspects of the present invention and the preferable embodiments described.

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Moreover, the following embodiments can, wherever this does not lead to logical contradictions, be combined with each other without restrictions. Thus, the present disclosure shall encompass, even where not explicitly spelled out in the following, any feasible combination of the embodiments described below. Furthermore, embodiments relating to one aspect of the present invention can, wherever this does not lead to logical contradictions, be combined with another aspect of the present invention without restrictions.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "5 to 50 amino acids" should be interpreted to include not only the explicitly recited values of 5 to 50, but also include individual values and sub-ranges within the indicated range. Thus, included in this numerical range are individual values such as 5, 6, 7, 8, 9, 10, 11, 12, 13, 14, 15, 16, 17, 18, 19, 20,... 48, 49, 50 and sub-ranges such as from 9 to 50, from5 to 25, from 9 to 25, from 10 to 25, from 10 to 20 and from 15 to 25, etc. This same principle applies to ranges reciting only one numerical value, such as "at least 8 amino acids". Furthermore, such an interpretation should apply regardless of the breadth of the range or the characteristics being described.

A majority of T cells have a T cell receptor (TCR) existing as a complex of several proteins. The actual T cell receptor is composed of two (separate) peptide chains, which are produced from the independent T cell receptor alpha and beta (TCR α and TCR β) genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells.

The structure of the T cell receptor is very similar to immunoglobulin Fab fragments, which are regions defined as the combined light and heavy chain of an antibody arm. Each chain of the TCR is a member of the immunoglobulin superfamily and possesses one N-terminal immunoglobulin (Ig)-variable (V) domain, one Ig-constant (C) domain, a transmembrane/cell membrane-spanning region, and a short cytoplasmic tail at the C-terminal end.

According to the invention, the term "variable region" of the T cell receptor α-chain or β-chain relates to the N-terminal immunoglobulin variable domain of said T cell receptor α-chain or β-chain. The variable domain of both the TCR α-chain and β-chain have three hypervariable or complementarity determining regions (CDR1, CDR2, CDR3), whereas the variable region of the β-chain has an additional area of hypervariability (HV4) that does not normally contact antigen and therefore is not considered a CDR. The three CDRs differ in their function in recognition of Peptide/MHC complexes on the surface of target cells, whereas the CDR3 regions of both chains play the major role in peptide specific recognition of the complex.

According to the invention, the term "constant region" of the T cell receptor α-chain or β-chain relates to the constant domain of the TCR including the transmembrane region and the cytoplasmic tail.

The first signal in activation of T cells is provided by binding of the T cell receptor to a short peptide presented by the major histocompatibility complex (MHC) on another cell. This ensures that only a T cell with a TCR specific to that peptide is activated.

Hematological neoplasia cells like leukemia cells, in particular myeloproliferative neoplasia (MPN) cells, acute myeloid leukemia (AML) cells, chronic myeloid leukemia (CML) cells, acute lymphoblastic leukemia (ALL) cells, myelodysplastic syndrome cells and myeloproliferative syndrome cells, express, like many other tumors, tumor-associated antigens. Myeloperoxidase (MPO) is a differentiation antigen with restricted expression in myeloid precursors, mature neutrophils, monocytes and macrophages, but otherwise not in healthy human cells outside of the embryo stage. Thus, MPO can serve as a specific marker for hematological neoplasia cells showing expression of myeloperoxidase (i.e. MPO-positive hematological neoplasia cells). In particular, as a myeloid differentiation antigen, MPO can serve as a specific marker for myeloid leukemias, such as in myeloproliferative neoplasia (MPN) cells, acute myeloid leukemia (AML) cells, chronic myeloid leukemia (CML) cells, myelodysplastic syndrome cells and myeloproliferative syndrome cells. Moreover, MPO can serve as a specific marker for certain acute lymphoblastic leukemias (ALL) showing expression of myeloperoxidase.

Whether hematological neoplasia cells or leukemia cells of a certain type of leukemia express myeloperoxidase at the protein level can be determined by the skilled person by validated standard methods, such as immunohistochemistry on bone marrow smears or intracellular staining of fixed cells with a myeloperoxidase-specific antibody followed by flow cytometric analysis, preferably by immunohistochemistry on bone marrow smears. Healthy myeloid cells can be equally detected by antibody staining as flow cytometry and immunohistochemistry of blood and bone marrow. Furthermore, whether hematological neoplasia cells or leukemia cells of a certain type of leukemia express myeloperoxidase at the mRNA level can be detected by qRT-PCR or RNA in situ hybridisation, preferably by RNA in situ hybridisation.

### T cell receptor

In a first aspect, the present invention relates to a T cell receptor (TCR) or a fragment thereof comprising:
(i) a T cell receptor α-chain or a fragment thereof comprising the amino acid sequence of SEQ ID NO: 3 *(CDR3 of T cell receptor α-chain)* or an amino acid sequence that has at least 75% identity, more preferably at least 83% identity, more preferably at least 91% identity with the amino acid sequence of SEQ ID NO: 3;
   and
(ii) a T cell receptor β-chain or a fragment thereof comprising the amino acid sequence of SEQ ID NO: 6 (*CDR3 of T cell receptor β-chain*) or an amino acid sequence that has at least 76% identity, more preferably at least 84% identity, more preferably at least 92% identity with the amino acid sequence of SEQ ID NO: 6.

In some embodiments, said T cell receptor or said fragment thereof is capable of binding to a peptide having the amino acid sequence of SEQ ID NO: 7 (*MPO₄₆₆₋₄₇₄peptide*) bound to HLA-B**07:02* (Human Leukocyte Antigen, allele B**07:02)* and/or to a peptide having the amino acid sequence of SEQ ID NO: 7 bound to HLA-B **07:04* and/or to a peptide having the amino acid sequence of SEQ ID NO: 7 bound to HLA-B**42:01*, more preferably to a peptide having the amino acid sequence of SEQ ID NO: 7 bound to HLA-B**07:02*.

In some embodiments, said fragment of said T cell receptor α-chain comprises at least the variable region of said T cell receptor α-chain or comprises at least a sequence of 25, preferably at least a sequence of 50, more preferably at least a sequence of 100 amino acids of said T cell receptor α-chain.

In some embodiments, said fragment of said T cell receptor β-chain comprises at least the variable region of said T cell receptor β-chain or comprises at least a sequence of 25, preferably at least a sequence of 50, more preferably at least a sequence of 100 amino acids of said T cell receptor β-chain.

In view of sequence conservation of these regions between different T cell receptor families, the CDR1 and CDR2 regions play, compared to CDR3, only an auxiliary role for the peptide specificity of the T cell receptor.

In some embodiments, said T cell receptor α-chain or said fragment thereof further comprises
- the amino acid sequence of SEQ ID NO: 1 *(CDR1 of T cell receptor α-chain)* or an amino acid sequence that has at least 83% identity with the amino acid sequence of SEQ ID NO: 1,
   and/or
- the amino acid sequence of SEQ ID NO: 2 *(CDR2 of T cell receptor α-chain)* or an amino acid sequence that has at least 75% identity, more preferably at least 87% identity with the amino acid sequence of SEQ ID NO: 2.

In some embodiments, said T cell receptor β-chain or said fragment thereof further comprises
- the amino acid sequence of SEQ ID NO: 4 *(CDR1 of T cell receptor β-chain)* or an amino acid sequence that has at least 80% identity with the amino acid sequence of SEQ ID NO: 4,
   and/or
- the amino acid sequence of SEQ ID NO: 5 *(CDR2 of T cell receptor β-chain)* or an amino acid sequence that has at least 83% identity with the amino acid sequence of SEQ ID NO: 5.

In some embodiments, all amino acid exchanges by which said amino acid sequence that has at least 75% identity, more preferably at least 83% identity, more preferably at least 91% identity with the amino acid sequence of SEQ ID NO: 3 differs from the amino acid sequence of SEQ ID NO: 3 are conservative amino acid exchanges.

In some embodiments, all amino acid exchanges by which said amino acid sequence that has at least 76% identity, more preferably at least 84% identity, more preferably at least 92% identity with the amino acid sequence of SEQ ID NO: 6 differs from the amino acid sequence of SEQ ID NO: 6 are conservative amino acid exchanges.

In some embodiments, all amino acid exchanges by which said amino acid sequence that has at least 83% identity with the amino acid sequence of SEQ ID NO: 1 differs from the amino acid sequence of SEQ ID NO: 1 are conservative amino acid exchanges.

In some embodiments, all amino acid exchanges by which said amino acid sequence that has at least 75% identity, more preferably at least 87% identity with the amino acid sequence of SEQ ID NO: 2 differs from the amino acid sequence of SEQ ID NO: 2 are conservative amino acid exchanges.

In some embodiments, all amino acid exchanges by which said amino acid sequence that has at least 80% identity with the amino acid sequence of SEQ ID NO: 4 differs from the amino acid sequence of SEQ ID NO: 4 are conservative amino acid exchanges.

In some embodiments, all amino acid exchanges by which said amino acid sequence that has at least 83% identity with the amino acid sequence of SEQ ID NO: 5 differs from the amino acid sequence of SEQ ID NO: 5 are conservative amino acid exchanges.

In some embodiments, said T cell receptor α-chain (or said fragment thereof) is covalently linked to said T cell receptor β-chain (or said fragment thereof), preferably by a peptide bond. In some embodiments, said T cell receptor α-chain (or said fragment thereof) and said T cell receptor β-chain (or said fragment thereof) are part of the same polypeptide chain.

In some embodiments, said T cell receptor α-chain (or said fragment thereof) and said T cell receptor β-chain (or said fragment thereof) are not part of the same polypeptide chain. In some embodiments said T cell receptor α-chain (or said fragment thereof) is not covalently linked to said T cell receptor β-chain (or said fragment thereof).

Preferably, the part of said T cell receptor α-chain (or said fragment thereof) having the amino acid sequence of SEQ ID NO: 1 or an amino acid sequence that has at least 83% identity with the amino acid sequence of SEQ ID NO: 1 forms the CDR1 (Complementarity Determining Region 1) region of said T cell receptor α-chain (or said fragment thereof).

Preferably, the part of said T cell receptor α-chain (or said fragment thereof) having the amino acid sequence of SEQ ID NO: 2 or an amino acid sequence that has at least 75% identity, more preferably at least 87% identity with the amino acid sequence of SEQ ID NO: 2 forms the CDR2 (Complementarity Determining Region 2) region of said T cell receptor α-chain (or said fragment thereof).

Preferably, the part of said T cell receptor α-chain (or said fragment thereof) having the amino acid sequence of SEQ ID NO: 3 or an amino acid sequence that has at least 75% identity, more preferably at least 83% identity, more preferably at least 91% identity with the amino acid sequence of SEQ ID NO: 3 forms the CDR3 (Complementarity Determining Region 3) region of said T cell receptor α-chain (or said fragment thereof).

Preferably, the part of said T cell receptor β-chain (or said fragment thereof) having the amino acid sequence of SEQ ID NO: 4 or an amino acid sequence that has at least 80% identity with the amino acid sequence of SEQ ID NO: 4 forms the CDR1 (Complementarity Determining Region 1) region of said T cell receptor β-chain (or said fragment thereof).

Preferably, the part of said T cell receptor β-chain (or said fragment thereof) having the amino acid sequence of SEQ ID NO: 5 or an amino acid sequence that has at least 83% identity with the amino acid sequence of SEQ ID NO: 5 forms the CDR2 (Complementarity Determining Region 2) region of said T cell receptor β-chain (or said fragment thereof).

Preferably, the part of said T cell receptor β-chain (or said fragment thereof) having the amino acid sequence of SEQ ID NO: 6 or an amino acid sequence that has at least 76% identity, more preferably at least 84% identity, more preferably at least 92% identity with the amino acid sequence of SEQ ID NO: 6 forms the CDR3 (Complementarity Determining Region 3) region of said T cell receptor β-chain (or said fragment thereof).

In some embodiments, the variable region of said T cell receptor α-chain or said fragment thereof is or comprises Vα1.1 and/or the variable region of said T cell receptor β-chain or said fragment thereof is or comprises Vβ13.2

In some embodiments,
a) said T cell receptor α-chain (or said fragment thereof) and/or said T cell receptor β-chain (or said fragment thereof) comprise or are composed of human sequences or
b) said T cell receptor α-chain (or said fragment thereof) and/or said T cell receptor β-chain (or said fragment thereof) comprise or are composed of mouse sequences or
c) said T cell receptor α-chain (or said fragment thereof) and/or said T cell receptor β-chain (or said fragment thereof) are each composed of a combination of human and mouse sequences, wherein, preferably, the constant region of said T cell receptor α-chain and the constant region of said T cell receptor β-chain are composed of mouse sequences and, preferably, the variable region of said T cell receptor α-chain and the variable region of said T cell receptor β-chain are composed of human sequences.

If a T cell receptor is used in human cells which T cell receptor has an α-chain and/or β-chain having a constant region composed of mouse sequences, this has the advantage that mispairing of said T cell receptor α-chain and/or β-chain with endogenous T cell receptor chains of said human cells is reduced/avoided.

In some embodiments, said T cell receptor (or said fragment thereof) comprises two disulfide bonds. Preferably, said T cell receptor α-chain (or said fragment thereof) and said T cell receptor β-chain (or said fragment thereof) are linked by said two disulfide bonds. This has the technical effect that mispairing of transgenic chains with endogenous chains is reduced/avoided.

In some embodiments, said T cell receptor comprises
(i) a T cell receptor α-chain having an amino acid sequence comprising the amino acid sequence of SEQ ID NO: 8 (*full-length sequence of* α-chain *of T cell receptor TCR2.5D6)* or an amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 8,
   and/or
(ii) a T cell receptor β-chain having an amino acid sequence comprising the amino acid sequence of SEQ ID NO: 9 *(full-length sequence of β-chain of T cell receptor TCR2.5D6)* or an amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 9.

In some embodiments, said fragment of said T cell receptor comprises
(i) a fragment of a T cell receptor α-chain having the amino acid sequence of SEQ ID NO: 8 (*full*-*length sequence of* α-chain *of T cell receptor TCR2.5D6)* or of an amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 8,
   wherein, preferably, said fragment comprises at least the variable region of said T cell receptor α-chain having the amino acid sequence of SEQ ID NO: 8 (or of said amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 8) or comprises at least a sequence of 25, preferably at least a sequence of 50, more preferably at least a sequence of 100 amino acids of said T cell receptor α-chain having the amino acid sequence of SEQ ID NO: 8 (or of said amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 8);
   and/or
(ii) a fragment of a T cell receptor having the amino acid sequence of SEQ ID NO: 9 (*full*-*length sequence of of T cell receptor TCR2.5D6)* or of an amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 9,
   wherein, preferably, said fragment comprises at least the variable region of said T cell receptor β-chain having the amino acid sequence of SEQ ID NO: 9 (or of said amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 9) or comprises at least a sequence of 25, preferably at least a sequence of 50, more preferably at least a sequence of 100 amino acids of said T cell receptor β-chain having the amino acid sequence of SEQ ID NO: 9 (or of said amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 9).

In some embodiments, said T cell receptor or said fragment thereof is a free molecule. In some embodiments, said T cell receptor or said fragment thereof is not linked to another molecule by covalent linkage. In some embodiments, said T cell receptor or said fragment thereof is not linked to another molecule. In some embodiments, said T cell receptor or said fragment thereof is not immobilized on a surface. In some embodiments, said T cell receptor or said fragment thereof is not immobilized on a cell.

In some embodiments, said T cell receptor or said fragment thereof is linked to another molecule, preferably by covalent linkage. In some embodiments, said T cell receptor or said fragment thereof is immobilized on a surface. In some embodiments, said T cell receptor or said fragment thereof is immobilized on a cell.

### Soluble T cell receptor construct

In a second aspect, the present invention relates to a soluble T cell receptor (sTCR) construct comprising
(1) a T cell receptor component comprising
   at least one T cell receptor α-chain (or fragment thereof) as defined in any of the embodiments above and
   at least one T cell receptor β-chain (or fragment thereof) as defined in any of the embodiments above,
   preferably covalently linked to each other to form a TCR heterodimer or multimer, and
(2) fusion component(s)
   preferably selected from
   - an Fc receptor,
   - an Fc domain of an antibody,
   - a cytokine, preferably IL-2 or IL-15,
   - a toxin,
   - an antibody, preferably selected from the group consisting of an anti-CD3 antibody, an anti-CD28 antibody, an anti-CD5 antibody, an anti-CD16 antibody and an anti-CD56 antibody,
   - a linker specific chimeric antigen receptor (CAR) expressed in a T cell, wherein the CAR recognizes a linker that is attached to the soluble T cell receptor construct,
   or combinations thereof,
wherein said at least one T cell receptor α-chain (or said fragment thereof) of (1) and/or said at least one T cell receptor β-chain (or that fragment thereof) of (1) is bound to the fusion component(s) of (2),
and wherein, preferably, the soluble T cell receptor construct furthermore comprises a label, preferably selected from the group consisting of a radionuclide, a fluorophore and gold particles.

Preferably, said fluorophore is fluorescein.

Preferably, said toxin is an exotoxin, more preferably pseudomonas exotoxin.

The soluble TCRs can comprise different of the fusion components (2), such as cytokines and Fc domains. TCR α-chains and TCR β-chains (or fragments thereof) can be solubilized by chemical modifications and linked covalently to each other and to other molecules such as Fc-receptors, cytokines and toxins (as described in Boulter et al., 2005; Lunde et al., 2010). These soluble TCR constructs are suitable to be directly used for the detection and destruction of MPO-positive hematological neoplasia cells, such as MPO-positive leukemia cells, for example myeloproliferative neoplasia (MPN) cells, the cells of an MPO-positive acute leukemia (such as acute myeloid leukemia (AML) cells), the cells of an MPO-positive chronic leukemia (such as chronic myeloid leukemia (CML) cells), the cells of an MPO-positive acute lymphoblastic leukemia (ALL), myelodysplastic syndrome cells or myeloproliferative syndrome cells, without the need of T cells. This can be achieved by application of soluble T cell receptor constructs to patients by intravenous, subcutaneous or intramuscular infusion or injections, respectively, or by application to the mucosa of the respiratory tract by inhalation or by spraying.

Linker specific chimeric antigen receptors (CARs) are described in Urbanska K et al., 2012 and Koristka S et al., 2014. A linker (e.g. biotin or a peptide derived from a nuclear protein) can be attached to the soluble TCRs. T cells transgenic for a CAR recognizing the linker (i.e. a linker specific CAR) can thereby be redirected to MPO-positive hematological neoplasia cells, such as MPO-positive leukemia cells, for example myeloproliferative neoplasia (MPN) cells, the cells of an MPO-positive acute leukemia (such as acute myeloid leukemia (AML) cells), the cells of an MPO-positive chronic leukemia (such as chronic myeloid leukemia (CML) cells), the cells of an MPO-positive acute lymphoblastic leukemia (ALL), myelodysplastic syndrome cells or myeloproliferative syndrome cells.

In some embodiments, the soluble TCR constructs of the present invention are used as recombinant proteins. Such recombinant proteins are expressed, preferably in *E.coli* or mammalian cells, and then purified before further use or application.

The present invention provides the expressed and purified soluble TCR constructs.

### Chimeric T cell receptor

In a third aspect, the present invention relates to a chimeric T cell receptor or fragment thereof comprising at least one T cell receptor or fragment thereof according to any of the embodiments above, wherein the T cell receptor α-chain (or fragment thereof) and/or the T cell receptor β-chain (or fragment thereof) is/are fused, preferably via a linker, to CD3-zeta chain(s) and/or one or more other T cell receptor stimulation domains, preferably selected from the group consisting of the intracellular CD28, CD137 or CD134 domains.

In some embodiments, said T cell receptor α-chain (or said fragment thereof) and said T cell receptor β-chain (or said fragment thereof) of said chimeric TCR are fused to each other via a linker.

Preferred CD3-zeta chain(s) are the zeta-chain(s) of the human CD3 complex of the T cell receptor. Preferred CD28, CD137 and CD134 intracellular domains are of human origin.

The chimeric TCRs or fragments thereof can comprise further components.

Chimeric T cell receptors and chimeric antigen receptors (CAR) are known in the art. See, for example, Han et al., 2013.

### Bi-specific antibody

In a fourth aspect, the present invention relates to a bi-specific antibody comprising
(a) a T cell receptor component comprising the T cell receptor α-chain (or fragment thereof) and the T cell receptor β-chain (or fragment thereof) of a T cell receptor (or fragment thereof) according to any of the embodiments above,
   wherein said T cell receptor α-chain (or said fragment thereof) and said T cell receptor β-chain (or said fragment thereof) are linked to each other
   and fused, preferably via a linker, to
(b) an antibody or a single chain antibody fragment (scFv) or an linker specific chimeric antigen receptor (CAR), preferably and antibody, which is directed against an antigen or epitope on the surface of lymphocytes.

In some embodiments, said T cell receptor α-chain (or said fragment thereof) and said T cell receptor β-chain (or said fragment thereof) are linked to each other covalently.

In some embodiments, the antibody or scFv or linker specific chimeric antigen receptor (CAR) is/are directed against CD3 or CD5 on the surface of T cells. In this embodiment, the bi-specific antibody will bind to T cells and redirect them to myeloperoxidase-presenting tumor cells (i.e. to tumor cells which present the peptide corresponding to myeloperoxidase, residues 466-474, bound to HLA-B**07:02*/B **07:4*/B**42:01* at their cell surface).

In another embodiment, the antibody or scFv or linker specific chimeric antigen receptor (CAR) is/are directed against the co-stimulatory CD28 receptor on T cells. In this embodiment, the bi-specific antibody will bind to and co-activate T cells and direct them to myeloperoxidase-presenting tumor cells.

In another embodiment, antibodies or scFV or linker specific chimeric antigen receptors (CARs) against CD3 or CD5 and CD28 are combined in tetravalent antibody constructs.

In some embodiments, the antibody or scFv or linker specific chimeric antigen receptor (CAR) is/are directed against the CD56 receptor of NK cells. In this embodiment, the bi-specific antibody will bind to NK cells and redirect them to myeloperoxidase-presenting tumor cells.

In another embodiment, the antibody or scFv or linker specific chimeric antigen receptor (CAR) is/are directed against the activating receptor CD16 on NK cells. In this embodiment, the bi-specific antibody will bind to and activate NK cells and direct them to myeloperoxidase-presenting tumor cells.

In another embodiment, antibodies or scFV or linker specific chimeric antigen receptors (CARs) against CD56 and CD16 are combined in tetravalent antibody constructs.

For example, a bi-specific antibody can be a bi-specific T cell engager (BiTE) which is a class of artificial bi-specific monoclonal antibodies that are investigated for their use as anti-cancer drugs. They direct a host's immune system, more specifically the T cells' cytotoxic activity, against cancer cells. *BiTE* is a registered trademark of Micromet AG. BiTEs are fusion proteins consisting of two single-chain variable fragments (scFvs) of different antibodies, or amino acid sequences from four different genes, on a single peptide chain of about 55 kilodaltons. One of the scFvs binds to T cells such as via the CD3 receptor or to CD56 for NK cell activation, and the other to a tumor cell via a tumor-specific molecule (see e.g. Csoka et al., 1996).

Preferably, the bi-specific antibodies of the present invention are used as recombinant proteins. Such recombinant proteins are expressed, preferably in *E. coli* or mammalian cells, and then purified before further use or application.

The present invention provides the expressed and purified bi-specific antibodies.

### Nucleic acid/Set of nucleic acids

In a fifth aspect, the present invention relates to a nucleic acid encoding
- the amino acid sequence of a T cell receptor or fragment thereof according to any of the embodiments above,
- the amino acid sequence of a soluble T cell receptor construct according to any of the embodiments above,
- the amino acid sequence of a chimeric T cell receptor or fragment thereof according to any of the embodiments above, or
- the amino acid sequence of the T cell receptor component of a bi-specific antibody according to any of the embodiments above;
or
- a set of nucleic acids, one of which encodes the amino acid sequence of the T cell receptor α-chain (or fragment thereof) and one of which the amino acid sequence of the T cell receptor β-chain (or fragment thereof) of a T cell receptor or fragment thereof according to any of the embodiments above;
- a set of nucleic acids, one of which encodes the amino acid sequence of the T cell receptor α-chain (or fragment thereof) and one of which the amino acid sequence of the T cell receptor β-chain (or fragment thereof) of a soluble T cell receptor construct as defined above;
- a set of nucleic acids, one of which encodes the amino acid sequence of the T cell receptor α-chain (or fragment thereof) and one of which the amino acid sequence of the T cell receptor β-chain (or fragment thereof) of a chimeric T cell receptor or fragment thereof as defined above;
or
- a set of nucleic acids, one of which encodes the amino acid sequence of the T cell receptor α-chain (or fragment thereof) and one of which the amino acid sequence of the T cell receptor β-chain (or fragment thereof) of the T cell receptor component of a bi-specific antibody as defined above.

The nucleic acids according to this invention comprise DNA (such as dsDNA, ssDNA, cDNA), RNA (such as dsRNA, ssRNA, mRNA), combinations thereof or derivatives (such as PNA) thereof.

Preferably, said nucleotide sequence is a DNA sequence. Preferably, said set of nucleotide sequences is a set of DNA sequences.

Codon-optimization refers to the exchange in a sequence of interest of codons that are generally rare in highly expressed genes of a given species by codons that are generally frequent in highly expressed genes of such species, such codons encoding the same amino acids as the codons that are being exchanged. Preferably, the nucleic acid sequences of the present invention are codon-optimized for expression in mammalian cells, preferably for expression in human cells, or comprise sequence stretches that are codon-optimized for expression in mammalian cells, preferably for expression in human cells. For generation of bi-specific antibodies, the nucleic acid sequences can also be codon-optimized for expression in bacteria, yeast or insect cells.

Within the scope of this invention are also the nucleotide sequences obtained due to the degeneration of the genetic code of the above nucleotide sequences.

Preferably, said nucleic acid or set of nucleic acids comprises
- the nucleotide sequence of SEQ ID NO: 10 or SEQ ID NO: 11 or a nucleotide sequence that has at least 80% identity, more preferably at least 85% identity, more preferably at least 90% identity, more preferably at least 95% identity, more preferably at least 98% identity, more preferably at least 99% identity, with the nucleotide sequence of SEQ ID NO: 10 or SEQ ID NO: 11,
   or a nucleotide sequence that is complementary to such a sequence;
   and/or
- the nucleotide sequence of SEQ ID NO: 12 or SEQ ID NO: 13 or a nucleotide sequence that has at least 80% identity, more preferably at least 85% identity, more preferably at least 90% identity, more preferably at least 95% identity, more preferably at least 98% identity, more preferably at least 99% identity, with the nucleotide sequence of SEQ ID NO: 12 or SEQ ID NO: 13,
   or a nucleotide sequence that is complementary to such a sequence.

Preferably, said nucleic acid or set of nucleic acids comprises
- a fragment of the nucleotide sequence of SEQ ID NO: 10 or SEQ ID NO: 11 or of a nucleotide sequence that has at least 80% identity, more preferably at least 85% identity, more preferably at least 90% identity, more preferably at least 95% identity, more preferably at least 98% identity, more preferably at least 99% identity, with the nucleotide sequence of SEQ ID NO: 10 or SEQ ID NO: 11,
   or a fragment of a nucleotide sequence that is complementary to such a sequence, wherein, preferably, said fragment encodes at least the variable region of the T cell receptor α-chain amino acid sequence encoded by the nucleotide sequence of SEQ ID NO: 10 or SEQ ID NO: 11 (or of an amino acid sequence that has at least 80% identity, more preferably at least 85% identity, more preferably at least 90% identity, more preferably at least 95% identity, more preferably at least 98% identity, more preferably at least 99% identity, with the amino acid sequence encoded by the nucleotide sequence of SEQ ID NO: 10 or SEQ ID NO: 11) or comprises at least a sequence of 30, preferably at least a sequence of 75, more preferably at least a sequence of 150 nucleotides of the nucleotide sequence of SEQ ID NO: 10 or SEQ ID NO: 11 or of a nucleotide sequence that has at least 80% identity, more preferably at least 85% identity, more preferably at least 90% identity, more preferably at least 95% identity, more preferably at least 98% identity, more preferably at least 99% identity with a nucleotide sequence that has at least 80% identity, more preferably at least 85% identity, more preferably at least 90% identity, more preferably at least 95% identity, more preferably at least 98% identity, more preferably at least 99% identity, with the nucleotide sequence of SEQ ID NO: 10 or SEQ ID NO: 11;
   and/or
- a fragment of the nucleotide sequence of SEQ ID NO: 12 or SEQ ID NO: 13 or of a nucleotide sequence that has at least 80% identity, more preferably at least 85% identity, more preferably at least 90% identity, more preferably at least 95% identity, more preferably at least 98% identity, more preferably at least 99% identity, with the nucleotide sequence of SEQ ID NO: 12 or SEQ ID NO: 13,
   or a fragment of a nucleotide sequence that is complementary to such a sequence,
   wherein, preferably, said fragment encodes at least the variable region of the T cell receptor α-chain amino acid sequence encoded by the nucleotide sequence of SEQ ID NO: 12 or SEQ ID NO: 13 (or of an amino acid sequence that has at least 80% identity, more preferably at least 85% identity, more preferably at least 90% identity, more preferably at least 95% identity, more preferably at least 98% identity, more preferably at least 99% identity, with the amino acid sequence encoded by the nucleotide sequence of SEQ ID NO: 12 or SEQ ID NO: 13) or comprises at least a sequence of 30, preferably at least a sequence of 75, more preferably at least a sequence of 150 nucleotides of the nucleotide sequence of SEQ ID NO: 12 or SEQ ID NO: 13 or of a nucleotide sequence that has at least 80% identity, more preferably at least 85% identity, more preferably at least 90% identity, more preferably at least 95% identity, more preferably at least 98% identity, more preferably at least 99% identity with a nucleotide sequence that has at least 80% identity, more preferably at least 85% identity, more preferably at least 90% identity, more preferably at least 95% identity, more preferably at least 98% identity, more preferably at least 99% identity, with the nucleotide sequence of SEQ ID NO: 12 or SEQ ID NO: 13.

The nucleotide sequences of SEQ ID NO. 10 to 13 are codon-optimized for expression in human cells.

### Expression construct

In a sixth aspect, the present invention relates to an expression construct for expressing the T cell receptor or fragment thereof according to any of the embodiments above, the soluble T cell receptor construct according to any of the embodiments above, the chimeric T cell receptor or fragment thereof according to any of the embodiments above or the bi-specific antibody according to any of the embodiments above in a host cell.

Preferably, said expression construct comprises a DNA sequence encoding
- the amino acid sequence of a T cell receptor or fragment thereof according to any of the embodiments above,
- the amino acid sequence of a soluble T cell receptor construct according to any of the embodiments above,
- the amino acid sequence of a chimeric T cell receptor or fragment thereof according to any of the embodiments above, or
- the amino acid sequence of the T cell receptor component of a bi-specific antibody according to any of the embodiments above.

Preferably, said expression construct comprises promoter and terminator sequences.

Preferably, said host cell is a mammalian cell or a bacterial cell, more preferably a human cell. Preferably, said host cell is a cultured cell.

An "expression construct" or "gene construct" (wherein both terms are used interchangeably throughout this specification) refers to a nucleic acid construct, usually an expression vector or plasmid that is used to introduce a specific gene sequence into a target cell (the host cell). Once the expression or gene construct is inside the host cell, the protein that is encoded by the gene is produced by the cellular transcription and translation machinery. The expression or gene construct is designed to contain respective regulatory sequences that act as enhancer and promoter regions and lead to efficient transcription of the gene carried on the construct, including promoter and terminator sequences. The goal of a well-designed expression or gene construct is the production of large amounts of stable mRNA, and therefore proteins. Alternatively, an RNA is synthesized or *in vitro* transcribed and introduced directly into the host cells for protein expression.

The skilled artisan can select further suitable components of expression or gene constructs.

Examples for suitable expression constructs are viral vectors, such as
- retroviral vectors, such as MP71 vectors (as described in Engels et al., 2003), or retroviral SIN vectors (as described in Yu et al., 1986);
   and
- lentiviral vectors or lentiviral SIN vectors, as described in Miyoshi et al., 1998.

Viral vectors containing TCR-encoding genes can infect lymphocytes and express functional TCR in infected lymphocytes. Preferred is a SIN vector construct.

Another example for a suitable vector is the *Sleeping Beauty* (SB) transposon transposase DNA plasmid system, SB DNA plasmid (as described in Maiti et al., 2013). Further options are the vector of the piggyBac transposition system (Manuri PV et al., 2010) or vectors for chromosomal insertion through the CRISPR/CAS9 system (Mandal PK et al., 2014).

The nucleic acids and/or in particular expression constructs of the invention can also be transferred into host cells by transient RNA transfection.

The nucleic acids and/or in particular expression constructs of the invention are capable of directing the synthesis/expression of the TCRs of the invention (or fragments thereof) in a suitable host cell.

### Cell

In a seventh aspect, the present invention relates to a cell comprising the T cell receptor (or fragment thereof) according to any of the embodiments above, the soluble T cell receptor construct according to any of the embodiments above, the chimeric T cell receptor (or fragment thereof) according to any of the embodiments above, the bi-specific antibody according to any of the embodiments above, the nucleic acid or set of nucleic acids according to any of the embodiments above or the expression construct according to any of the embodiments above.

As used herein, the term "cell" refers to a eukaryotic cell (for example a mammalian cell or yeast cell) or prokaryotic cell (for example a bacterial cell, such as an *E.coli* cell). Thus, a cell is a structural unit of living organisms, which is capable of self replicating, has genetic information and a mechanism for expressing it, and is surrounded by a membrane structure which isolates the cell from the surrounding environment.

Preferably, said cell is a transgenic cell.

Preferably, said cell comprises a transgene, wherein said transgene comprises said nucleic acid or set of nucleic acids according to any of the embodiments above.

As used herein, the term "transgenic cell" refers to a cell that has a transgene stably integrated into its genome and that transmits the transgene to its progeny during cell division. As used herein, the term "transgene" refers to a heterologous gene that is stably integrated into the genome of a cell and that is transmitted to progeny of the cell during cell division.

Preferably, said cell expresses the T cell receptor (or fragment thereof) according to the present invention. Preferably, said cell expresses the soluble T cell receptor construct according to the present invention. Preferably, said cell expresses the chimeric T cell receptor (or fragment thereof) according to the present invention. Preferably, said cell expresses the bi-specific antibody according to the present invention.

Preferably, said cell is a cell for expression of the T cell receptor (or fragment thereof) according to the present invention. Preferably, said cell is a cell for expression of the soluble T cell receptor construct according to the present invention. Preferably, said cell is a cell for expression of the chimeric T cell receptor (or fragment thereof) according to the present invention. Preferably, said cell is a cell for expression of the bi-specific antibody according to the present invention.

Preferably, said cell expresses the T cell receptor (or fragment thereof) according to any of the embodiments above, the soluble T cell receptor construct according to any of the embodiments above, the chimeric T cell receptor (or fragment thereof) according to any of the embodiments above or the bi-specific antibody according to any of the embodiments above.

Preferably, said cell is a mammalian cell, more preferably a human cell.

Preferably, said cell is a cultured cell or a cell isolated from the organism that it originates from.

Preferably, said cell is a lymphocyte (preferably a T cell or a natural killer (NK) cell). More preferably, said cell is a T cell, even more preferably a CD3⁺ T cell or CD8⁺ T cell.

The transfer of myeloperoxidase-specific TCR genes into (primary) T cells by e.g. (retro-)viral vectors, SB DNA or by transient RNA transfection represents a promising means and method to generate myeloperoxidase antigen-specific T cells. Adoptive transfer of such engineered T cells into hosts represents a promising approach for the immunotherapy of MPO-positive hematological neoplasia, such as MPO-positive leukemia, for example myeloproliferative neoplasia (MPN), MPO-positive acute leukemia (such as acute myeloid leukemia (AML)), MPO-positive chronic leukemia (such as chronic myeloid leukemia (CML)), MPO-positive acute lymphoblastic leukemia (ALL), myelodysplastic syndrome or myeloproliferative syndrome.

Preferably, said cell for expression of the T cell receptor (or fragment thereof) according to the present invention or for expression of the bi-specific antibody according to the present invention is selected from the group consisting of an *E. coli* cell, an insect cell and a mammalian cell. As the skilled person will appreciate, it is advantageous to use cells that express high amounts of recombinant protein. Thus, among mammalian cells HEK293 cells are preferred.

The application of myeloperoxidase-specific bi-specific antibodies or soluble T cell receptors represents an alternative promising means and method to target myeloperoxidase-positive tumor cells by redirected T cells or by the soluble T cell receptor construct. This represents a promising approach for the immunotherapy of leukemia, in particular myeloproliferative neoplasia (MPN), acute myeloid leukemia (AML) and chronic myeloid leukemia (CML).

### Pharmaceutical composition

In an eighth aspect, the present invention relates to a pharmaceutical composition comprising one or more of:
(i) the T cell receptor (or fragment thereof) according to any of the embodiments above;
(ii) the soluble T cell receptor construct according to any of the embodiments above;
(iii) the chimeric T cell receptor (or fragment thereof) according to any of the embodiments above;
(iv) the bi-specific antibody according to any of the embodiments above;
(v) the nucleic acid or set of nucleic acids according to any of the embodiments above or the expression construct according to any of the embodiments above; and/or
(vi) the cell according to any of the embodiments above,
and, optionally, pharmaceutical excipient(s).

### Use for generating genetically modified lymphocytes

In a ninth aspect, the present invention relates to the use of a T cell receptor (or fragment thereof) according to any of the embodiments above, a chimeric T cell receptor (or fragment thereof) according to any of the embodiments above, a bi-specific antibody according to any of the embodiments above, a nucleic acid or set of nucleic acids according to any of the embodiments above or an expression construct according to any of the embodiments above for generating genetically modified lymphocytes (preferably selected from the group consisting of T cells, NK cells and NKT cells), preferably genetically modified T cells, more preferably genetically modified CD3⁺ T cells or CD8⁺ T cells.

Preferably, said use is an *in vitro* use or an *in vivo* use.

In some embodiments, the generated genetically modified lymphocytes, preferably T cells and/or NK cells, are for use in immunotherapy or donor lymphocyte infusions or purging in the context of induction or consolidation therapy with autologous modified cells, preferably for preventing and/or treating MPO-positive hematological neoplasia, preferably MPO-positive leukemia, more preferably a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably acute myeloid leukemia (AML).

The transfer of myeloperoxidase-specific TCR genes (or parts thereof) into (primary) T cells by e.g. (retro-)viral vectors or by transient RNA transfection represents a promising means and method to generate myeloperoxidase antigen-specific T cells. Adoptive transfer of such engineered T cells into hosts represents a promising approach for the immunotherapy of MPO-positive hematological neoplasia, such as MPO-positive leukemia, for example of myeloproliferative neoplasia (MPN), MPO-positive acute leukemia (e.g. acute myeloid leukemia (AML)), MPO-positive chronic leukemia (e.g. chronic myeloid leukemia (CML)), MPO-positive acute lymphoblastic leukemia (ALL), myelodysplastic syndrome and myeloproliferative syndrome. A new therapeutic approach is the adoptive T cell therapy. The transferred T cells can recognize tumor antigen and thereby specifically eliminate tumor cells. MPO-positive hematological neoplasia, such as MPO-positive leukemia, for example of myeloproliferative neoplasia (MPN), MPO-positive acute leukemia (e.g. acute myeloid leukemia (AML)), MPO-positive chronic leukemia (e.g. chronic myeloid leukemia (CML)), MPO-positive acute lymphoblastic leukemia (ALL), myelodysplastic syndrome and myeloproliferative syndrome, are targets for T cell therapy because such tumors express, like many other tumors, tumor-associated antigens, such as myeloperoxidase.

The transfer of chimeric receptors containing the whole or parts of the myeloperoxidase-specific TCR genes linked to the CD3zeta chain into primary NK cells by e.g. (retro-)viral vectors or by transient RNA transfection represents a promising means and method to generate myeloperoxidase antigen-specific NK cells. Adoptive transfer of such engineered NK cells into hosts represents a promising approach for the immunotherapy of MPO-positive hematological neoplasia, such as MPO-positive leukemia, for example of myeloproliferative neoplasia (MPN), MPO-positive acute leukemia (e.g. acute myeloid leukemia (AML)), MPO-positive chronic leukemia (e.g. chronic myeloid leukemia (CML)), MPO-positive acute lymphoblastic leukemia (ALL), myelodysplastic syndrome and myeloproliferative syndrome.

Said genetically modified lymphocytes may be used for adoptive, target cell specific immunotherapy or genetic immunization of a patient. Preferably, said patient suffers from an MPO-positive hematological neoplasia, preferably from MPO-positive leukemia, more preferably from a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably from acute myeloid leukemia (AML). Preferably, said patient is HLA-B**07:02* positive and/or HLA-B**07:04* positive and/or HLA-B**42:01* positive, more preferably HLA-B **07:02* positive.

### Medical uses

With respect to the different aspects of the present invention described above, in some embodiments the T cell receptor (or fragment thereof) according to any of the embodiments above, the soluble T cell receptor construct according to any of the embodiments above, the chimeric T cell receptor (or fragment thereof) according to any of the embodiments above, the bi-specific antibody according to any of the embodiments above, the nucleic acid or set of nucleic acids according to any of the embodiments above, the expression construct according to any of the embodiments above, the cell according to any of the embodiments above or the pharmaceutical composition as defined above is for use as a medicament.

With respect to the different aspects of the present invention described above, in some embodiments the T cell receptor (or fragment thereof) according to any of the embodiments above, the soluble T cell receptor construct according to any of the embodiments above, the chimeric T cell receptor (or fragment thereof) according to any of the embodiments above, the bi-specific antibody according to any of the embodiments above, the nucleic acid or set of nucleic acids according to any of the embodiments above, the expression construct according to any of the embodiments above, the cell according to any of the embodiments above or the pharmaceutical composition as defined above is for use in the detection, diagnosis or prognosis of a hematological neoplasia, preferably of leukemia, more preferably of a disease selected from the group consisting of myeloproliferative neoplasia (MPN), acute leukemia (preferably acute myeloid leukemia (AML)), chronic leukemia (preferably chronic myeloid leukemia (CML)), acute lymphoblastic leukemia (ALL), myelodysplastic syndrome and myeloproliferative syndrome, even more preferably of acute myeloid leukemia (AML).

Preferably, said hematological neoplasia is an MPO-positive hematological neoplasia, preferably MPO-positive leukemia, more preferably a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably acute myeloid leukemia (AML).

Preferably, the cells of said hematological neoplasia express myeloperoxidase on the protein level, more preferably on the mRNA level and the protein level. Preferably, leukemia cells of said leukemia express myeloperoxidase on the protein level, more preferably on the mRNA level and the protein level. Preferably, cells of said myeloproliferative neoplasia (MPN), acute leukemia, acute myeloid leukemia (AML), chronic leukemia, chronic myeloid leukemia (CML), acute lymphoblastic leukemia (ALL), myelodysplastic syndrome and myeloproliferative syndrome express myeloperoxidase on the protein level, more preferably on the mRNA level and the protein level.

Preferably, said detection, diagnosis or prognosis is performed *in vitro*.

With respect to the different aspects of the present invention described above, in some embodiments the T cell receptor (or fragment thereof) according to any of the embodiments above, the soluble T cell receptor construct according to any of the embodiments above, the chimeric T cell receptor (or fragment thereof) according to any of the embodiments above, the bi-specific antibody according to any of the embodiments above, the nucleic acid or set of nucleic acids according to any of the embodiments above, the expression construct according to any of the embodiments above, the cell according to any of the embodiments above or the pharmaceutical composition as defined above is for use in the prevention and/or treatment of an MPO-positive hematological neoplasia, preferably of MPO-positive leukemia, more preferably of a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably of acute myeloid leukemia (AML).

Preferably, leukemia cells of said leukemia express myeloperoxidase on the mRNA and the protein level.

Preferably, said treatment involves administration of said T cell receptor (or fragment thereof), soluble T cell receptor construct, chimeric T cell receptor (or fragment thereof), bi-specific antibody, nucleic acid or set of nucleic acids, expression construct, cell or pharmaceutical composition to a patient in need thereof. Preferably, said patient is undergoing or has undergone treatment by chemotherapy and/or radiotherapy in combination with allogeneic stem cell transplantation (SCT) or in combination with autologous stem cell transplantation followed by deletion of transgenic T cells. Preferably, said patient is a human patient. Preferably, said patient is HLA-B**07:02* positive and/or HLA-B**07:04* positive and/or HLA-B**42:01* positive, more preferably HLA*-B*07:02* positive. Preferably, said patient is having a relapse after treatment of said MPO-positive hematological neoplasia.

Preferably, said T cell receptor (or fragment thereof), soluble T cell receptor construct, chimeric T cell receptor (or fragment thereof), bi-specific antibody, nucleic acid or set of nucleic acids, expression construct, cell or pharmaceutical composition is configured for administration to a patient in need thereof. Preferably, said patient is undergoing or has undergone treatment by chemotherapy and/or radiotherapy in combination with allogeneic stem cell transplantation (SCT) or in combination with autologous stem cell transplantation followed by deletion of transgenic T cells. Preferably, said patient is a human patient. Preferably, said patient is HLA-B**07:02* positive and/or HLA-B**07:04* positive and/or HLA-B**42:01* positive, more preferably HLA*-B*07:02* positive. Preferably, said patient is having a relapse after treatment of said MPO-positive hematological neoplasia.

Preferably, said treatment involves allogeneic stem cell transplantation (SCT) from a donor to said patient.

Preferably, said treatment also involves a combination of chemotherapy and/or radiotherapy with allogeneic stem cell transplantation (SCT) from a donor to said patient.

Preferably, said treatment involves administration of a donor lymphocyte infusion to said patient, wherein, preferably, the lymphocytes of said donor lymphocyte infusion are transgenic T cells expressing said T cell receptor, more preferably transgenic CD3⁺ or CD8⁺ T cells expressing said T cell receptor.

Preferably, said patient is HLA-B**07:02* positive and/or HLA-B**07:04* positive and/or HLA-B**42:01* positive, more preferably HLA-B**07:02* positive, whereas said donor is HLA-B**07:02* negative and/or HLA-B**07:04* negative and/or HLA-B**42:01* negative, more preferably HLA-*B*07:02* negative. Alternatively, said patient and said donor are both HLA*-B*07:02* positive and/or HLA-B**07:04* positive and/or HLA-B**42:01* positive, more preferably *HLA-B*07:02* positive, whereas this will necessitate an efficient suicide mechanism to eliminate the MPO-specific cell/substance.

Preferably said allogeneic stem cell transplantation is a haploidentical or one allele mismatch allogeneic stem cell transplantation, wherein, preferably, said patient is HLA-B**07:02* positive and/or HLA-B**07:04* positive and/or HLA-B**42:01* positive, more preferably HLA-B**07:02* positive, and said donor is HLA-B**07:02* negative and/or HLA-B**07:04* negative and/or HLA-B**42:01* negative, more preferably HLA*-B*07:02* negative. Alternatively, said allogeneic stem cell transplantation is an HLA-identical allogeneic stem cell transplantation, wherein, preferably, said patient and said donor are both HLA-B**07:02* positive and/or HLA-B**07:04* positive and/or HLA-B**42:01* positive, more preferably HLA-*B***07:02* positive.

As the skilled person will appreciate, if said patient and said donor are HLA-identical, then the transgenic T cells expressing said T cell receptor must include a mechanism that allows to reduce the number of transgenic T cells in said patient after treatment, in order to allow for regeneration of healthy (i.e. non-leukemic) myeloid cells/myeloid precursor cells in the bone marrow. This can for example be achieved by a "suicide mechanism" as described in Bonini et al., 1997.

Preferably, said treatment comprises the use of adoptive, target cell specific immunotherapy or genetic immunization.

In a tenth aspect, the present invention relates to the use of the T cell receptor (or fragment thereof) according to any of the embodiments above, the soluble T cell receptor construct according to any of the embodiments above, the chimeric T cell receptor (or fragment thereof) according to any of the embodiments above, the bi-specific antibody according to any of the embodiments above, the nucleic acid or set of nucleic acids according to any of the embodiments above, the expression construct according to any of the embodiments above, the cell according to any of the embodiments above or the pharmaceutical composition as defined above for the manufacture of a medicament, preferably a medicament for the prevention and/or treatment of an MPO-positive hematological neoplasia, preferably of MPO-positive leukemia, more preferably of a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably of acute myeloid leukemia (AML).

In an eleventh aspect, the present invention relates to a method of preventing and/or treating an MPO-positive hematological neoplasia, preferably MPO-positive leukemia, more preferably a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably acute myeloid leukemia (AML),
comprising the steps of
(a) providing lymphocytes of a patient or a (stem cell) donor;
(b) providing one or more of
   (i) a T cell receptor (or fragment thereof) according to any of the embodiments above or a chimeric T cell receptor according to any of the embodiments above,
   (ii) a nucleic acid or set of nucleic acids according to any of the embodiments above,
   (iii) an expression construct according to any of the embodiments above,
(c) *ex vivo* introduction of one or more of (i) to (iii) of step (b) into the lymphocytes of step (a) and, thereby, obtaining genetically modified lymphocytes,
(d) administering the genetically modified lymphocytes of step (c) to a subject or patient in need thereof;
(e) optionally, combining any of the steps (b) to (d) with radiation, checkpoint inhibitor or cancer chemotherapy.

In such method, said MPO-positive hematological neoplasia, said lymphocytes, said patient, said donor and said genetically modified lymphocytes are preferably as defined in the above-described aspects of the present invention or any of the embodiments referring to them.

T cells express inhibitory receptors such as cytotoxic T-lymphocyte-associated antigen 4 (CTLA-4) or programmed cell death 1 (PD-1), especially after long term exposure to the antigen. The ligands of CTLA-4 and PD-1, B7-1/B7-2 and PD-L1/PD-L2 respectively, as well as the ligand of TIM-3, Galectin 9, are frequently expressed on tumor tissue and are involved in induction of T cell tolerance and thus are called immune checkpoints. To avoid silencing of transferred myeloperoxidase specific T cells checkpoint inhibitors or immune checkpoint inhibitors, exemplified by but not restricted to anti-CTLA-4, anti-PD-L1 or anti-PD-1 antibodies, can be used to avoid inhibition of T cells within the tumor microenvironment (described in Hamid et al. (2013)).

The subjects or patients are preferably HLA-B**07:02* positive and/or HLA-B**07:04* positive and/or HLA-B**42:01* positive, more preferably HLA*-B*07:02* positive.

Preferably, the lymphocytes provided in step (a) are T cells, NK cells and/or NKT cells, more preferably T cells, even more preferably CD3⁺ T cells or CD8⁺ T cells.

The lymphocytes provided in step (a) can be obtained from the subject or patient, such as from the blood or by leukapheresis of allogeneic stem cell donors.

Preferably, the *ex vivo* introduction in step (c) is carried out via electroporation of a nucleic acid of the present invention or of an expression construct of the present invention, or by transfection reagents, such as liposomes, or by transient RNA transfection.

The expression construct is preferably, as described above, a viral construct, preferably a lentiviral vector, retroviral vector, or other state of the art viral vector.

Examples for suitable expression constructs are viral vectors, such as
- retroviral vectors, such as MP71 vectors (as described in Engels et al., 2003), or retroviral SIN vectors (as described in Yu et al., 1986);
   and
- lentiviral vectors or lentiviral SIN vectors, as described in Miyoshi et al., 1998.

Another example for a suitable expression construct is the *Sleeping Beauty* (SB) transposon transposase DNA plasmid system, SB DNA plasmid (as described in Maiti et al., 2013). Alternatively, the vector is a vector suitable for the piggyBac (as described in Manuri PV et al., 2010) transposition system or a vector for chromosomal insertion through the CRISPR/CAS9 system (Mandal PK et al., 2014).

In a twelfth aspect, the present invention relates to a method of preventing and/or treating an MPO-positive hematological neoplasia, preferably MPO-positive leukemia, more preferably a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably acute myeloid leukemia (AML),
comprising the steps of
(a) providing one or more of
   (i) a T cell receptor (or fragment thereof) according to any of the embodiments above or a soluble T cell receptor construct according to any of the embodiments above or a chimeric T cell receptor (or fragment thereof) according to any of the embodiments above or a bi-specific antibody according to any of the embodiments above,
   (ii) a nucleic acid or set of nucleic acids according to any of the embodiments above,
   (iii) an expression construct according to any of the embodiments above,
   (iv) a cell according to any of the embodiments above, and
   (v) a pharmaceutical composition as defined above;
(b) direct application, preferably via injection or infusion, of one or more of (i) to (v) of step (a) to a subject or patient in need thereof,
(c) optionally, combining step (b) with radiation, checkpoint inhibitor or cancer chemotherapy.

In such method, said MPO-positive hematological neoplasia, said patient and said expression construct are preferably as defined in the above-described aspects of the present invention or any of the embodiments referring to them.

Preferably, the bi-specific antibodies or the soluble TCR constructs of the present invention are used as recombinant proteins.

In some embodiments, in step (b) the one or more of (i) to (v) of step (a) are used as recombinant proteins.

The recombinant proteins, preferably the bi-specific antibodies or the soluble TCR constructs of the present invention, are expressed, preferably in *E.coli* or mammalian cells, and then purified before the application. In a thirteenth aspect, the present invention relates to a method of detecting, diagnosing, prognosing, preventing and/or treating a hematological neoplasia, preferably leukemia, more preferably a disease selected from the group consisting of myeloproliferative neoplasia (MPN), acute leukemia (preferably acute myeloid leukemia (AML)), chronic leukemia (preferably chronic myeloid leukemia (CML)), acute lymphoblastic leukemia (ALL), myelodysplastic syndrome and myeloproliferative syndrome, even more preferably acute myeloid leukemia (AML),
comprising the detection and/or destruction of (leukemic) cancer cells of a patient with the use of the soluble T cell receptor construct according to any of the embodiments above.

Preferably, said cancer cells express myeloperoxidase at the protein level, more preferably at the mRNA level and at the protein level.

Preferably said cancer cells are MPO-positive hematological neoplasia cells, more preferably MPO-positive leukemia, even more preferably cancer cells selected from the group consisting of myeloproliferative neoplasia (MPN) cells, MPO-positive acute leukemia cells (preferably acute myeloid leukemia (AML) cells), MPO-positive chronic leukemia cells (preferably chronic myeloid leukemia (CML) cells), MPO-positive acute lymphoblastic leukemia (ALL) cells, myelodysplastic syndrome cells and myeloproliferative syndrome cells. Even more preferably, said cancer cells are acute myeloid leukemia (AML) cells.

In such method, said MPO-positive hematological neoplasia cells and said patient are preferably as defined in the above-described aspects of the present invention or any of the embodiments referring to them.

Soluble T cell receptor constructs can be applied to patients:
- by intravenous infusions,
- by subcutaneous injection or infusion,
- by intramuscular injection,
- by inhalation or by spraying to the mucosal surfaces of the respiratory tract.

Suitable dose ranges for application of soluble T cell receptor constructs vary between 1 mg to 10 000 mg dependent on the application method and application site. Soluble T cell receptors can bind to cells presenting myeloperoxidase peptides on their HLA*-B*07:02*/*B*07:04*/*B*42:01* molecules, namely MPO-positive hematological neoplasia cells, such as more preferably MPO-positive leukemia, for example myeloproliferative neoplasia (MPN) cells, MPO-positive acute leukemia cells (e.g. acute myeloid leukemia (AML) cells), MPO-positive chronic leukemia cells (e.g. chronic myeloid leukemia (CML) cells), MPO-positive acute lymphoblastic leukemia (ALL) cells, myelodysplastic syndrome cells or myeloproliferative syndrome cells, in which a peptide of myeloperoxidase residues 466-474 is presented by HLA-*B***07:02*/*B***07:04*/*B***42:01*. Soluble T cell receptors can induce destruction of the cells via different methods, such as radiation by linked radionuclides or killing by toxins such as pseudomonas exotoxin. In addition, soluble T cell receptors with linked Fc-domains can redirect and activate NK-cells and/or monocytes for the lysis of cells of a MPO-positive hematological neoplasia, in particular cells of an MPO-positive leukemia as described above. Similarly, soluble T cell receptors linked to anti-CD3-antibodies can redirect and activate T cells for lysis of cells of a MPO-positive hematological neoplasia, in particular cells of an MPO-positive leukemia as described above. Furthermore, soluble T cell receptors combined with a linker can redirect and active T cells that are transgenic for a CAR that binds specifically to the linker.

In a fourteenth aspect, the present invention relates to a method of detecting a hematological neoplasia, preferably leukemia, more preferably a disease selected from the group consisting of myeloproliferative neoplasia (MPN), acute leukemia (preferably acute myeloid leukemia (AML)), chronic leukemia (preferably chronic myeloid leukemia (CML)), acute lymphoblastic leukemia (ALL), myelodysplastic syndrome and myeloproliferative syndrome, even more preferably acute myeloid leukemia (AML),
comprising the *in vitro* staining of cells obtained from a human subject by using the soluble T cell receptor construct of the present invention which is labelled.

Preferably, the cells of said hematological neoplasia express myeloperoxidase on the protein level, more preferably on the mRNA level and the protein level. Preferably, leukemia cells of said leukemia express myeloperoxidase on the protein level, more preferably on the mRNA level and the protein level. Preferably, cells of said myeloproliferative neoplasia (MPN), acute leukemia, acute myeloid leukemia (AML), chronic leukemia, chronic myeloid leukemia (CML), acute lymphoblastic leukemia (ALL), myelodysplastic syndrome and myeloproliferative syndrome express myeloperoxidase on the protein level, more preferably on the mRNA level and the protein level.

In such method, said hematological neoplasia is preferably as defined in the above-described aspects of the present invention or any of the embodiments referring to them.

Preferably, said human subject is HLA-B**07:02* positive and/or HLA-B**07:04* positive and/or HLA-B**42:01* positive, more preferably HLA-*B***07:02* positive.

Preferably, said cells obtained from said human subject are bone marrow aspirate cells or peripheral blood mononucleated cells cells. Preferably, said cells obtained from said human subject are cells isolated from blood or bone marrow or chloroma/myeloid sarcoma cells (leukemic manifestations outside from blood/bone marrow indicates an extramedullary myeloid tumor).

The label can be fluorophore(s), gold and/or radionuclide(s).

Soluble T cell receptors linked to fluorescent molecules such as fluorescein can be used to stain cells of a MPO-positive hematological neoplasia, in particular cells of an MPO-positive leukemia as described above *in vitro.* For this purpose, said cells , are incubated *in vitro* with the soluble T cell receptor constructs, and cells expressing HLA-*B***07:02*/myeloperoxidase peptide complexes, HLA-*B*07:04*/myeloperoxidase peptide complexes and/or HLA-*B***42:01*/myeloperoxidase peptide complexes on the cell surface can be detected according to their fluorescence by a Fluorescence activated cell sorter (FACS) or by microscope.

Alternatively, labeled soluble T cell receptor constructs (e.g. with fluorochromes, gold or radionuclides) are injected into a subject or patient and allow detection and, if the soluble T cell receptor carries an appropriate label, subsequent destruction of myeloperoxidase-positive tumor, tumor metastasis or minimal residual disease after tumor resection.

The soluble TCR constructs according to the invention are suitable to be directly used for the detection and destruction of cells of a MPO-positive hematological neoplasia, in particular cells of an MPO-positive leukemia as described above, without the need of T cells.

The term "T cell receptor", as used in the present invention, has the common meaning. Thus, a T cell receptor is a protein receptor that is capable of recognizing antigenic determinants (peptides) presented in the MHC (major histocompatibility complex) context. Typically, a T cell receptor is composed of a heterodimer of a T cell receptor alpha (α) chain and a T cell receptor beta (β) chain. For more details, see Murphy, Travers, Walport: "Janeway's Immunobiology", 7th ed., Garland Science (2008).

The T cell receptor of the HLA-class I-restricted CD8+ CTL consists of two proteins, the TCR α-chain and the TCR β-chain. Both the TCR α-chain and the TCR β-chain are formed from various gene segments, which are rearranged from a variety of variable gene segments at the TCR α locus and the TCR β locus. The TCR α-chain consists of a variable (V) gene segment, a J (Joining) gene segment and a constant (C) gene segment. The TCR consists of a variable (V) gene segment, a J (Joining) gene segment, a D (diversity) gene segment and a constant (C) gene segment.

The individual TCR of individual T cells is generated during the thymocyte development by recombination of gene segments. D-to-J recombination occurs first in the β chain of the TCR. This process can involve either the joining of the Dβ1 gene segment to one of six Jβ1 segments or the joining of the Dβ2 gene segment to one of seven Jβ2 segments. DJ recombination is followed (as above) with Vβ-to-DβJβ rearrangements. All gene segments between the Vβ-Dβ-Jβ gene segments in the newly formed complex are deleted and the primary transcript is synthesized that incorporates the constant domain gene (Vβ-Dβ-Jβ-Cβ). mRNA transcription splices out any intervening sequence and allows translation of the full length protein for the TCR Cβ chain.

The rearrangement of the alpha (α) chain of the TCR follows β chain rearrangement, and resembles V-to-J rearrangement described for Ig light chains (see above). The diversity of the somatically rearranged TCRs is enhanced further by the introduction of additional nucleotides between the V and J gene segments of rearranged TCR-alpha genes. The rearrangement of TCR gene segments provide a diverse repertoire of approximately 10¹⁸ different TCRs. The TCR-alpha-chain and the TCR-beta-chain pair after their synthesis to yield the αβ-TCR -heterodimer that is expressed on a majority of T cells.

The specificity of the recognition of viral or tumor-associated or autoimmunity-associated peptides is mainly determined by the CDR3-region of both the TCR-alpha and the TCR-beta chains. Each CTL usually expresses only a single functional T cell receptor (TCR) that allows the recognition of only a single antigenic peptide (or group of peptides with highly related sequence). Dependent on the individual TCR sequence, TCRs can exhibit a certain cross-reactivity that may allow also the recognition of peptide variants to a certain degree.

At some instances, the present invention indicates that a certain amino acid sequence A has "at least x % identity" with another amino acid sequence B [or to a certain nucleotide sequence A that has "at least x % identity" with another nucleotide sequence B]. This designates a situation where, in an optimal sequence alignment of sequence A with sequence B at least x % of the aligned amino acid residues [or nucleotides] of sequence A and B are identical. Alignment for purposes of determining percent sequence identity can be achieved in various ways that are within the skill in the art, for instance, using publicly available computer software, and those skilled in the art can determine appropriate parameters for measuring alignment, including any algorithms needed to achieve maximal alignment over the full length of the sequences being compared. Preferably, the alignment is carried out according to the algorithm of Huang and Miller (published in Adv. Appl. Math. (1991) 12:337-357), for example as global alignment with the software ALIGN (by William Pearson, available at http://www.ch.embnet.org/ software/LALIGN_form.html), with an opening gap penalty setting of -14, an extending gap penalty setting of -4, and with the scoring matrix BLOSUM80. The amino acid residues [or nucleotides] at corresponding amino acid [or nucleotide] positions are then compared. When a position in the first sequence is occupied by the same amino acid residue [or nucleotide] as the corresponding position in the second sequence, then the molecules are identical at that position.

At some instances, the present application refers to a "conservative amino acid exchange", for example in the context that all amino acid exchanges by which a certain first amino acid sequence differs from a certain second are "conservative amino acid exchanges". This is meant to designate that corresponding, but differing residue positions in the two amino acid sequences are occupied by amino acids that have residues with similar side chains. Groups of amino acids that have similar side chains are: For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids exchange groups are: valine-leucine-isoleucine, phenylalanine-tyrosine, lysine-arginine, alanine-valine, glutamic-aspartic, and asparagine-glutamine.

The terms "HLA-B**07:02" and* "HLA-B7" are used interchangeably herein and refer to a certain HLA allele and the corresponding protein that is capable of binding to the peptide MPO₄₆₆₋₄₇₄.

If the present application indicates that a certain molecule or part of a molecule (such as the T cell receptor α-chain or the constant region of the T cell receptor α-chain) "is composed of human sequences", this designates a situation where said molecule or part of a molecule comprises only sequences of human origin, but no sequences originating from other organisms. Similarly, if the present application indicates that a certain molecule or part of a molecule "is composed of mouse sequences", this designates a situation where said molecule or part of a molecule comprises only sequences that originate from mouse, but no sequences originating from other organisms. If the present application indicates that a certain molecule or part of a molecule "is composed of a combination of human and mouse sequences", this designates a situation where said molecule or part of a molecule comprises sequence(s) originating from mouse and sequence(s) of human origin, but no sequences originating from other organisms.

The terms "MPO₄₆₆₋₄₇₄" and "MPO₅" are used interchangeably herein and refer to a peptide corresponding to residues 466-474 of the protein human myeloperoxidase, i.e. a peptide with the sequence of SEQ ID NO: 7 (NPRWDGERL).

At some instances, the present application refers to an "MPO-positive" disease, for example "MPO-positive leukemia". This means that the cells of said leukemia express myeloperoxidase both at the mRNA level and at the protein level. Similarly, if the present application refers to certain "MPO-positive" cells, for example "MPO-positive leukemia cells", this means that said cells express myeloperoxidase both at the mRNA level and at the protein level.

"Hematological neoplasia", as used herein, refers to a malignant disease of the hematopoietic system involving cells of the myeloid and/or lymphoid lineage characterized by the progressive proliferation of malignant cells of said lineages.

"Leukemia" as used herein, refers to a disease involving the progressive proliferation of abnormal leukocytes found in hematopoietic tissues, other organs and in the blood, resulting in increased numbers of leukocytes. "Leukemic cells" refers to leukocytes characterized by an increased abnormal proliferation of cells. Leukemic cells may be obtained from a subject diagnosed with leukemia. The term includes, but is not limited to, acute lymphocytic leukemia (ALL), myeloproliferative neoplasias (MPN), acute myeloid leukemia (AML) and chronic myeloid leukemia (CML), myelodysplastic syndrome, myeloproliferative syndrome, hypereosinophilic syndrome (such as myeloid neoplasia with eosinophilia or chronic eosinophilic leukemia), extramedullary myeloid tumors (such as chloromas or myeloid sarcomas) and monocytic leukemia. In some embodiments, the term "leukemia" is limited to leukemias that express myeloperoxidase at the protein level, preferably at the mRNA level and at the protein level, and the term "leukemic cells" is limited to leukemic cells that express myeloperoxidase at the protein level, preferably at the mRNA level and at the protein level.

The term "treatment", as used herein, refers to the process of providing a subject with a pharmaceutical treatment, such that a disease or disease state is alleviated, reduced, minimized, halted or even healed, and/or such that the chances of a relapse into the disease state are reduced or a relapse into the disease state is even prevented.

"Pharmaceutically acceptable", as used herein for example in the context of a pharmaceutically acceptable salt of a compound, refers to a substance (or composition) that is non-toxic to the subject to which it is administered and that thus can be used in the formulation of a pharmaceutical product. If the pharmaceutically acceptable substance is part of a pharmaceutical composition, then the term also implies that the pharmaceutically acceptable substance is compatible with the other ingredients of the said pharmaceutical composition.

"Cultured cells", as used herein, refers to cells growing under conditions of *in vitro* culture. Thus cultured cells are free tissue cells that are cultivated in a system entirely apart from their normal environment in which the conditions and factors for growth can be varied at will within the boundaries tolerated by the cells.

As used herein, the term *"in vitro"* means occurring outside of a living organism. The term *in vitro* can describe processes/conditions occurring within a cell culture system. In contrast to *"in vitro",* the term *"in vivo"* means occurring within a living organism. The term *"ex vivo"* means occurring in a cell culture context or tissue culture context. For example, cells may be extracted from a subject; a therapeutic gene may be introduced into the cells in a cell culture context outside of the living organism (i.e. *ex vivo);* and subsequently the transgenic cells may be returned into the same subject.
SEQ ID NO: 1 shows the amino acid sequence of the CDR1 (Complementarity Determining Region 1) region of the α-chain of a T cell receptor according to the present invention *(CDR1 of T cell receptor α-chain):*
   YGATPY
SEQ ID NO: 2 shows the amino acid sequence of the CDR2 (Complementarity Determining Region 2) region of the α-chain of a T cell receptor according to the present invention *(CDR2 of T cell receptor α-chain):*
   YFSGDTLV
SEQ ID NO: 3 shows the amino acid sequence of the CDR3 (Complementarity Determining Region 3) region of the α-chain of a T cell receptor according to the present invention *(CDR3 of T cell receptor α-chain):*
   CAGRAAGNKLTF
SEQ ID NO: 4 shows the amino acid sequence of the CDR1 (Complementarity Determining Region 1) region of the β-chain of a T cell receptor according to the present invention *(CDR1 of T cell receptor β-chain):*
   MNHEY
SEQ ID NO: 5 shows the amino acid sequence of the CDR2 (Complementarity Determining Region 2) region of the β-chain of a T cell receptor according to the present invention *(CDR2 of T cell receptor β-chain):*
   SVGEGT
SEQ ID NO: 6 shows the amino acid sequence of the CDR3 (Complementarity Determining Region 3) region of the β-chain of a T cell receptor according to the present invention *(CDR3 of T cell receptor β-chain):*
   CASSYSSGQPQHF
SEQ ID NO: 7 shows the amino acid sequence of myeloperoxidase, residues 466 to 474 (MPO₄₆₆₋₄₇₄):
   NPRWDGERL
SEQ ID NO: 8 shows the amino acid sequence of the α-chain of a T cell receptor according to the present invention (TCR2.5D6), wherein said α-chain is composed of a combination of the variable region of a human α-chain (variable region in SEQ ID NO: 8: amino acids 1 to 130 of SEQ ID NO: 8) and the constant region of a mouse α-chain and wherein, in the constant region of the mouse α-chain, an amino acid exchange T83C has been carried out (Voss et al., 2010): 
LLELIPLLGIHFVLRTARAQSVTQPDIHITVSEGASLELRCNYSYGATPYLFWYVQSPGQG
LQLLLKYFSGDTLVQGIKGFEAEFKRSQSSFNLRKPSVHWSDAAEYFCAGRAAGNKLTF
GGGTRVLVKPNIQNPEPAVYQLKDPRSQDSTLCLFTDFDSQINVPKTMESGTFITDKTVL
DMKAMDSKSNGAIAWSNQTSFTCQDIFKETNACYPSSDVPCDATLTEKSFETDMNLNF
QNLSVMGLRILLLKVAGFNLLMTLRLWSS
 In some embodiments, the amino acid sequence of a full-length T cell receptor α-chain according to the present invention may comprise an additional N-terminal M (methionine) residue.
SEQ ID NO: 9 shows the amino acid sequence of the β-chain of a T cell receptor according to the present invention (TCR2.5D6), wherein said β-chain is composed of a combination of the variable region of a human β-chain (variable region in SEQ ID NO: 9: amino acids 1 to 130 of SEQ ID NO: 9) and the constant region of a mouse β-chain and wherein, in the constant region of the mouse β-chain, an amino acid exchange S79C has been carried out (Voss et al., 2010): 
SLGLLCCGVFSLLWAGPVNAGVTQTPKFRVLKTGQSMTLLCAQDMNHEYMYWYRQD
PGMGLRLIHYSVGEGTTAKGEVPDGYNVSRLKKQNFLLGLESAAPSQTSVYFCASSYSS
GQPQHFGDGTRLSILEDLRNVTPPKVSLFEPSKAEIANKQKATLVCLARGFFPDHVELSW
WVNGKEVHSGVSTDPQAYKESNYSYCLSSRLRVCATFWHNPRNHFRCQVQFHGLSEED
KWPEGSPKPVTQNISAEAWGRADCGITSASYHQGVLSATILYEILLGKATLYAVLVSGL
VLMAMVKKKNS
 In some embodiments, the amino acid sequence of a full-length T cell receptor β-chain according to the present invention may comprise an additional N-terminal M (methionine) residue.
SEQ ID NO: 10 shows a DNA sequence of the α-chain of a T cell receptor according to the present invention (TCR2.5D6; CDRs indicated by underline): 
CTGCTGGAACTGATCCCCCTGCTGGGAATCCACTTCGTGCTGAGGACAGCCAGAGCC
CAGTCTGTGACCCAGCCCGACATCCACATCACCGTGTCTGAGGGCGCCAGCCTGGA
ACTGCGGTGCAACTACTCTTACGGCGCCACCCCCTACCTGTTTTGGTACGTGCAGAG
CCCCGGACAGGGACTGCAGCTGCTGCTGAAGTACTTTAGCGGCGACACCCTGGTGC
AGGGCATCAAGGGATTCGAGGCCGAGTTCAAGCGGAGCCAGAGCAGCTTCAACCTG
CGGAAGCCCTCCGTGCATTGGAGCGACGCCGCCGAGTATTTCTGTGCCGGCAGAGC
AGCCGGCAACAAGCTGACATTTGGCGGCGGAACCAGGGTGCTCGTGAAGCCCAACA
TCCAGAACCCCGAGCCCGCCGTGTACCAGCTGAAGGACCCTAGAAGCCAGGACAGC
ACCCTGTGCCTGTTCACCGACTTCGACTCCCAGATCAACGTGCCCAAGACCATGGAA
AGCGGCACCTTCATCACCGACAAGACCGTGCTGGACATGAAGGCCATGGACAGCAA
GAGCAACGGCGCCATTGCCTGGTCCAATCAGACCAGCTTCACATGCCAGGACATCTT
CAAAGAGACAAACGCCTGCTACCCCAGCAGCGACGTGCCCTGTGATGCCACCCTGA
CCGAGAAGTCCTTCGAGACAGACATGAATCTGAACTTCCAGAACCTGAGCGTGATG
GGCCTGAGAATCCTGCTGCTGAAAGTGGCCGGCTTCAATCTGCTGATGACCCTGAGA
CTGTGGTCCAGC
SEQ ID NO: 11 shows a DNA sequence of the α-chain of a T cell receptor according to the present invention (TCR2.5D6) including start and stop codons (CDRs indicated by underline): 
ATGCTGCTGGAACTGATCCCCCTGCTGGGAATCCACTTCGTGCTGAGGACAGCCAGA
GCCCAGTCTGTGACCCAGCCCGACATCCACATCACCGTGTCTGAGGGCGCCAGCCTG
GAACTGCGGTGCAACTACTCTTACGGCGCCACCCCCTACCTGTTTTGGTACGTGCAG
AGCCCCGGACAGGGACTGCAGCTGCTGCTGAAGTACTTTAGCGGCGACACCCTGGT
GCAGGGCATCAAGGGATTCGAGGCCGAGTTCAAGCGGAGCCAGAGCAGCTTCAACC
TGCGGAAGCCCTCCGTGCATTGGAGCGACGCCGCCGAGTATTTCTGTGCCGGCAGA
GCAGCCGGCAACAAGCTGACATTTGGCGGCGGAACCAGGGTGCTCGTGAAGCCCAA
CATCCAGAACCCCGAGCCCGCCGTGTACCAGCTGAAGGACCCTAGAAGCCAGGACA
GCACCCTGTGCCTGTTCACCGACTTCGACTCCCAGATCAACGTGCCCAAGACCATGG
AAAGCGGCACCTTCATCACCGACAAGACCGTGCTGGACATGAAGGCCATGGACAGC
AAGAGCAACGGCGCCATTGCCTGGTCCAATCAGACCAGCTTCACATGCCAGGACAT
CTTCAAAGAGACAAACGCCTGCTACCCCAGCAGCGACGTGCCCTGTGATGCCACCCT
GACCGAGAAGTCCTTCGAGACAGACATGAATCTGAACTTCCAGAACCTGAGCGTGA
TGGGCCTGAGAATCCTGCTGCTGAAAGTGGCCGGCTTCAATCTGCTGATGACCCTGA
GACTGTGGTCCAGCTGA
SEQ ID NO: 12 shows a DNA sequence of the β-chain of a T cell receptor according to the present invention (TCR2.5D6; CDRs indicated by underline): 
TCTCTGGGACTGCTGTGTTGCGGCGTGTTCAGCCTGCTGTGGGCCGGACCTGTGAAT
GCCGGCGTGACCCAGACCCCCAAGTTCCGGGTGCTGAAAACCGGCCAGAGCATGAC
CCTGCTGTGCGCCCAGGACATGAACCACGAGTACATGTATTGGTACAGACAGGACC
CCGGCATGGGCCTGCGGCTGATCCACTATTCTGTGGGCGAGGGCACCACCGCCAAG
GGCGAAGTGCCTGATGGCTACAACGTGTCCCGGCTGAAGAAGCAGAACTTCCTGCT
GGGCCTGGAAAGCGCCGCTCCTAGCCAGACCAGCGTGTACTTCTGCGCCAGCAGCT
ACAGCAGCGGCCAGCCTCAGCACTTTGGCGACGGCACCAGACTGAGCATCCTGGAA
GATCTGCGGAACGTGACCCCCCCCAAGGTGTCCCTGTTCGAGCCTAGCAAGGCCGA
GATCGCCAACAAGCAGAAAGCCACCCTCGTGTGCCTGGCCAGAGGCTTCTTCCCCG
ACCACGTGGAACTGTCTTGGTGGGTCAACGGCAAAGAGGTGCACAGCGGCGTGTCC
ACCGATCCCCAGGCCTACAAAGAGAGCAACTACAGCTACTGCCTGTCCAGCAGACT
GAGAGTGTGCGCCACCTTCTGGCACAACCCCCGGAACCACTTCAGATGCCAGGTGC
AGTTTCACGGCCTGAGCGAAGAGGACAAGTGGCCCGAGGGCAGCCCTAAGCCCGTG
ACACAGAATATCAGCGCCGAAGCCTGGGGCAGAGCCGACTGTGGAATCACCAGCGC
 
CAGCTACCATCAGGGCGTGCTGAGCGCCACCATCCTGTACGAGATTCTGCTGGGCAA
GGCCACCCTGTACGCCGTGCTGGTGTCTGGCCTGGTGCTGATGGCCATGGTCAAGAA
GAAGAACAGC
SEQ ID NO: 13 shows a DNA sequence of the β-chain of a T cell receptor according to the present invention (TCR2.5D6) including start and stop codons (CDRs indicated by underline):

ATGTCTCTGGGACTGCTGTGTTGCGGCGTGTTCAGCCTGCTGTGGGCCGGACCTGTG
AATGCCGGCGTGACCCAGACCCCCAAGTTCCGGGTGCTGAAAACCGGCCAGAGCAT
GACCCTGCTGTGCGCCCAGGACATGAACCACGAGTACATGTATTGGTACAGACAGG
ACCCCGGCATGGGCCTGCGGCTGATCCACTATTCTGTGGGCGAGGGCACCACCGCC
AAGGGCGAAGTGCCTGATGGCTACAACGTGTCCCGGCTGAAGAAGCAGAACTTCCT
GCTGGGCCTGGAAAGCGCCGCTCCTAGCCAGACCAGCGTGTACTTCTGCGCCAGCA
GCTACAGCAGCGGCCAGCCTCAGCACTTTGGCGACGGCACCAGACTGAGCATCCTG
GAAGATCTGCGGAACGTGACCCCCCCCAAGGTGTCCCTGTTCGAGCCTAGCAAGGC
CGAGATCGCCAACAAGCAGAAAGCCACCCTCGTGTGCCTGGCCAGAGGCTTCTTCC
CCGACCACGTGGAACTGTCTTGGTGGGTCAACGGCAAAGAGGTGCACAGCGGCGTG
TCCACCGATCCCCAGGCCTACAAAGAGAGCAACTACAGCTACTGCCTGTCCAGCAG
ACTGAGAGTGTGCGCCACCTTCTGGCACAACCCCCGGAACCACTTCAGATGCCAGGT
GCAGTTTCACGGCCTGAGCGAAGAGGACAAGTGGCCCGAGGGCAGCCCTAAGCCCG
TGACACAGAATATCAGCGCCGAAGCCTGGGGCAGAGCCGACTGTGGAATCACCAGC
GCCAGCTACCATCAGGGCGTGCTGAGCGCCACCATCCTGTACGAGATTCTGCTGGGC
AAGGCCACCCTGTACGCCGTGCTGGTGTCTGGCCTGGTGCTGATGGCCATGGTCAAG
AAGAAGAACAGCTGA

In connection with their finding that myeloperoxidase (MPO) is an essential and distinct marker for myeloid cells and AML that is restrictedly expressed in the hematopoietic system (Klar et al., 2014), the inventors have identified a T cell receptor from the naïve T cell repertoire of an HLA-B7-negative (i.e. HLA*-B*07:02* negative) donor with specificity against one selected HLA-B7/B42:01-restricted MPO epitope (MPO5). This T cell receptor shows high reactivity against leukemia cells endogenously expressing myeloperoxidase (MPO), and T cells transgenic for this TCR recognize MPO⁺ leukemic cell lines and MPO⁺ primary leukemic cells from patients with AML and MPN *in vitro* and *in vivo.* On target reactivity was limited to hematopoietic cells with high MPO-mRNA expression. MPO-dependent toxicity was not observed against mature hematopoietic cells with MPO protein but low mRNA expression, and no reactivity against monocytes and healthy hematopoietic stem cells (HSC) and only very low reactivity against granulocytes were observed. Moreover, no off-target reactivity was observed when diverse *cell* lines of different origin as well as a high number of peptides that have been eluted from HLA-B7 were tested. Furthermore, the peptide motif recognized by TCR-transgenic T cells is unique for MPO and reactivity against peptides with high homology to MPO5 is lacking. This implies that adoptive T cell therapy with T cells transgenic for this TCR may be safe in the haploidentical or HLA-B7 allele mismatch allogeneic SCT setting, where donor myeloid progenitor cells lack the HLA-B7 restricting allele and thus this approach can be the base for a personalized therapeutic option for patients with relapsing leukemias by adoptive T cell therapies using a defined tumor-specific T cell receptor.

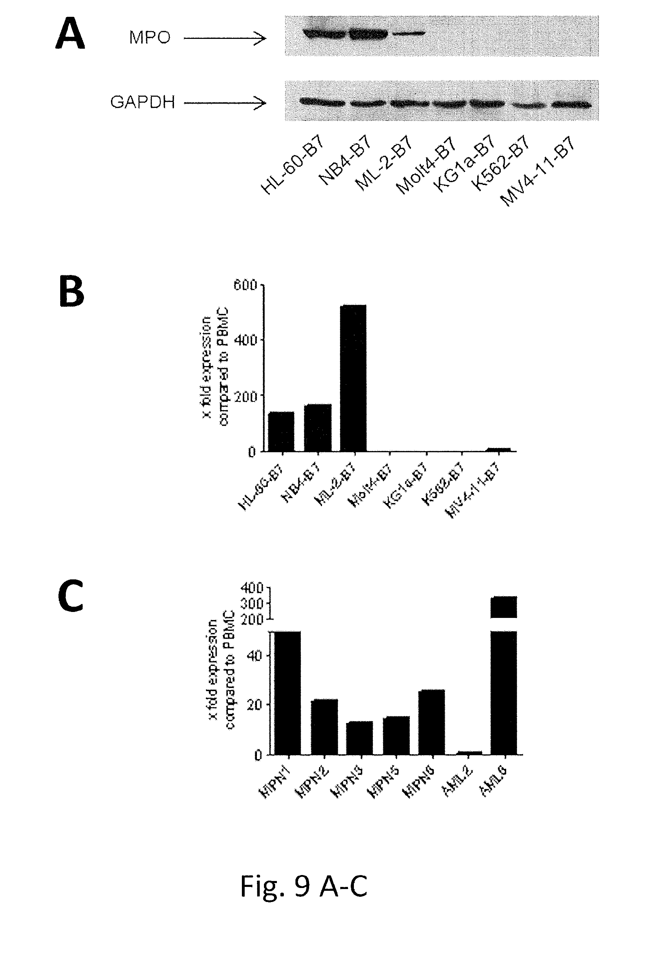

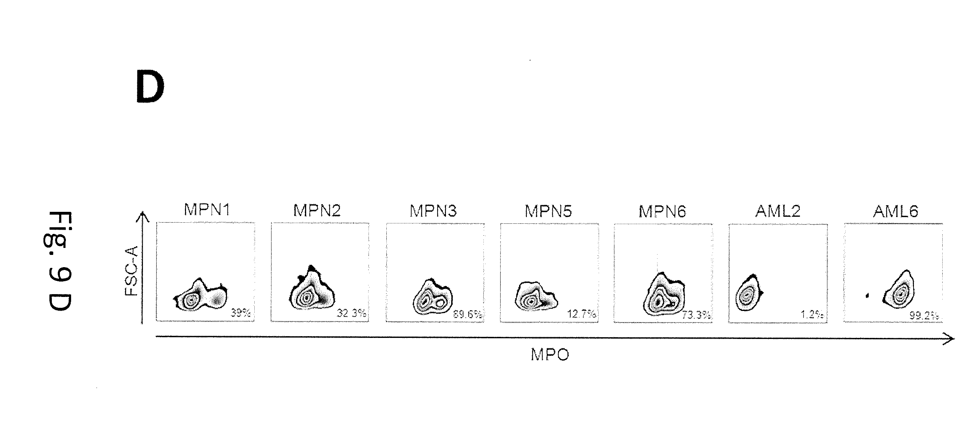

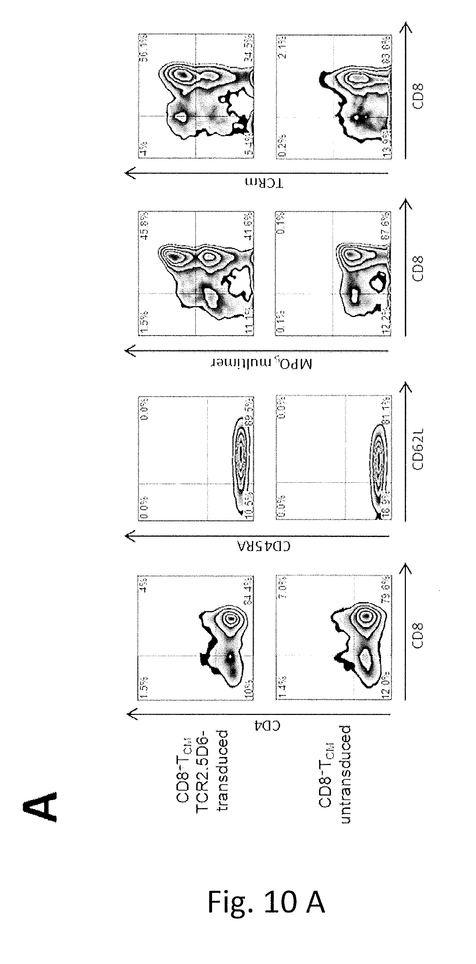

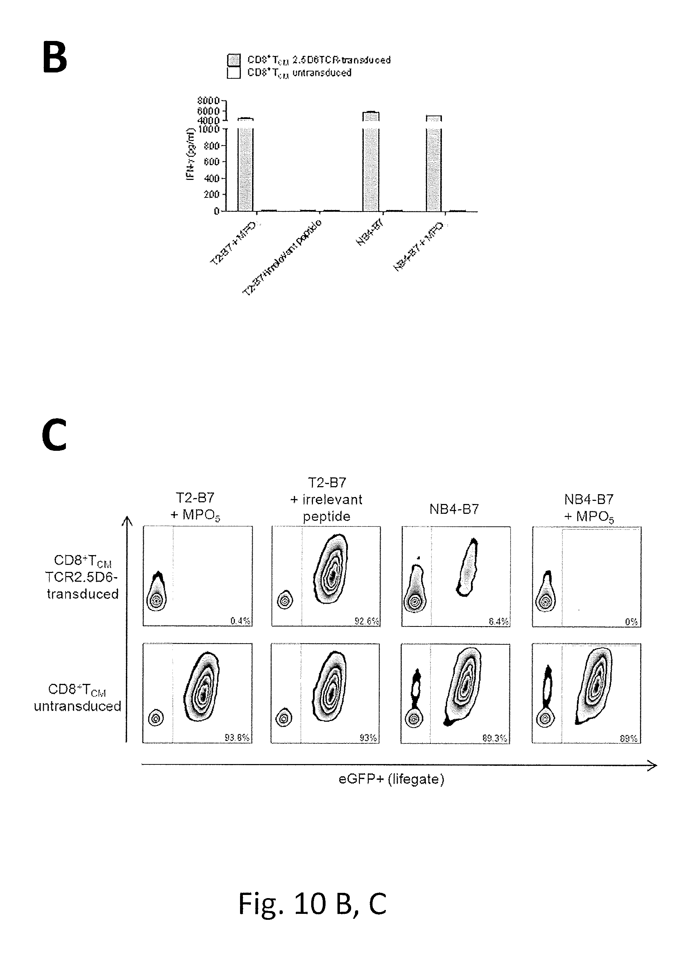

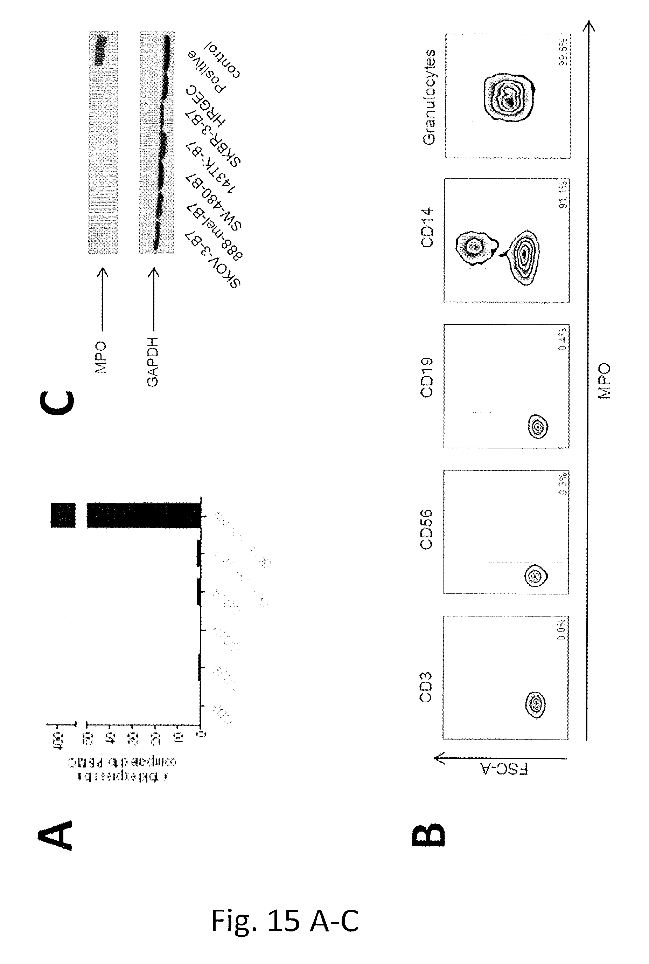

In the following, reference is made to the figures:
All methods mentioned in the figure descriptions below were carried out as described in detail in the examples.

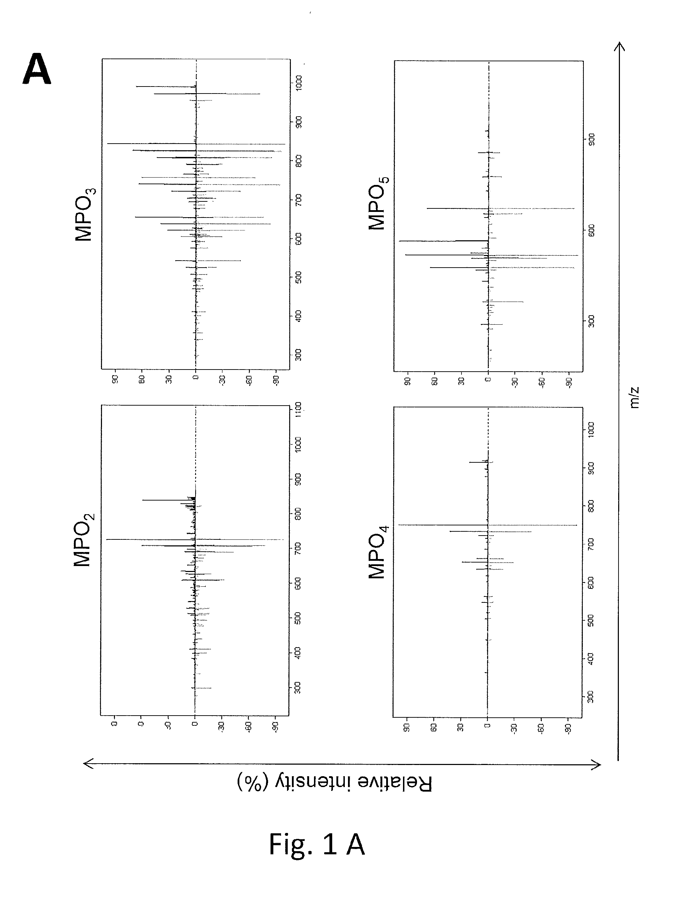

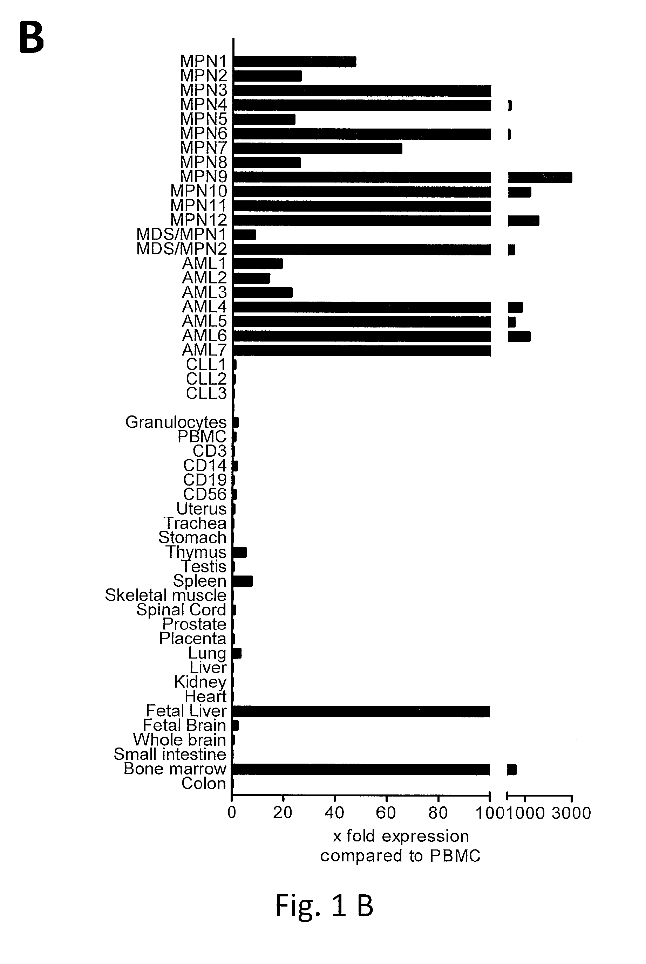

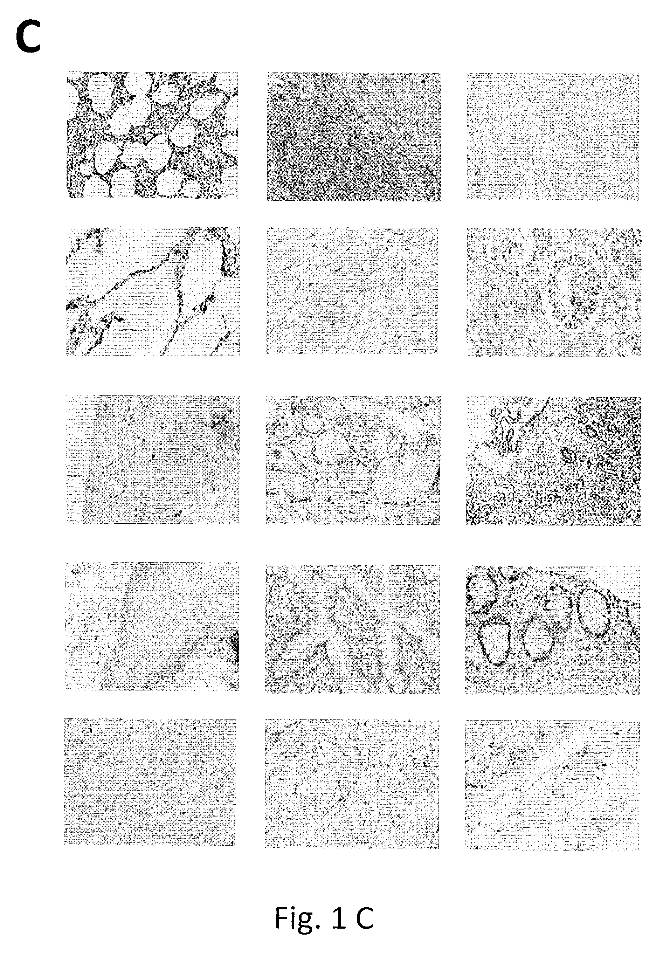

   **Figure 1** shows experimental data confirming MPO as potential T cell target antigen in myeloid leukemia.
      (A) MPO-derived HLA ligands identified by the immunopeptidomic approach were synthesized, sequenced by mass spectrometry and matched to the spectra of eluted peptides after normalization with the mMass tool for sequence validation. Spectra of synthetic peptides are shown in red (lower) and spectra of eluted peptides in black (upper).
      (B) mRNA expression of MPO in relation to PBMC was analyzed by qPCR on primary material and diverse healthy tissues. Ct values were normalized against the geometric mean of three housekeeping genes (GAPDH, HPRT1, HMBS) using the ddCT method.
      (C) MPO immunohistochemistry in diverse healthy tissue samples (from left to right and top to bottom): bone marrow, lymph node, spleen, lung, heart, kidney, brain, thyroid gland, endometrium, esophagus, intestinal mucosa, colon mucosa, liver, vessels, soft tissue.

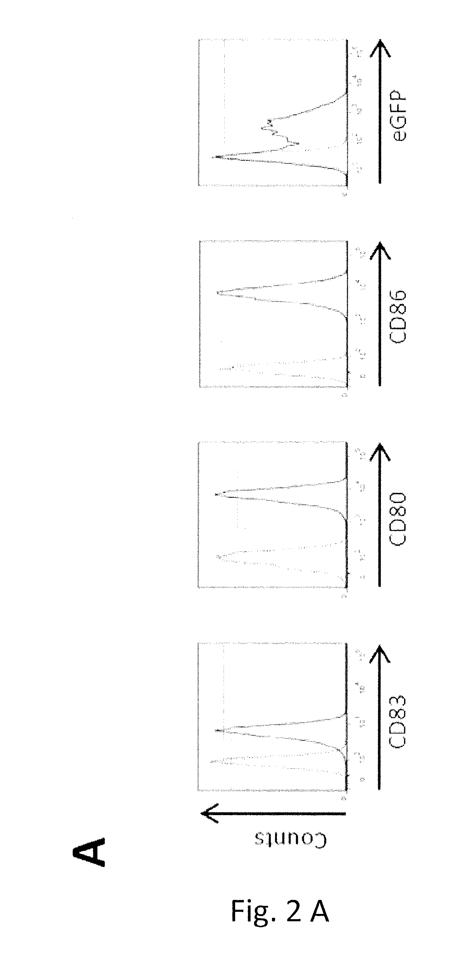

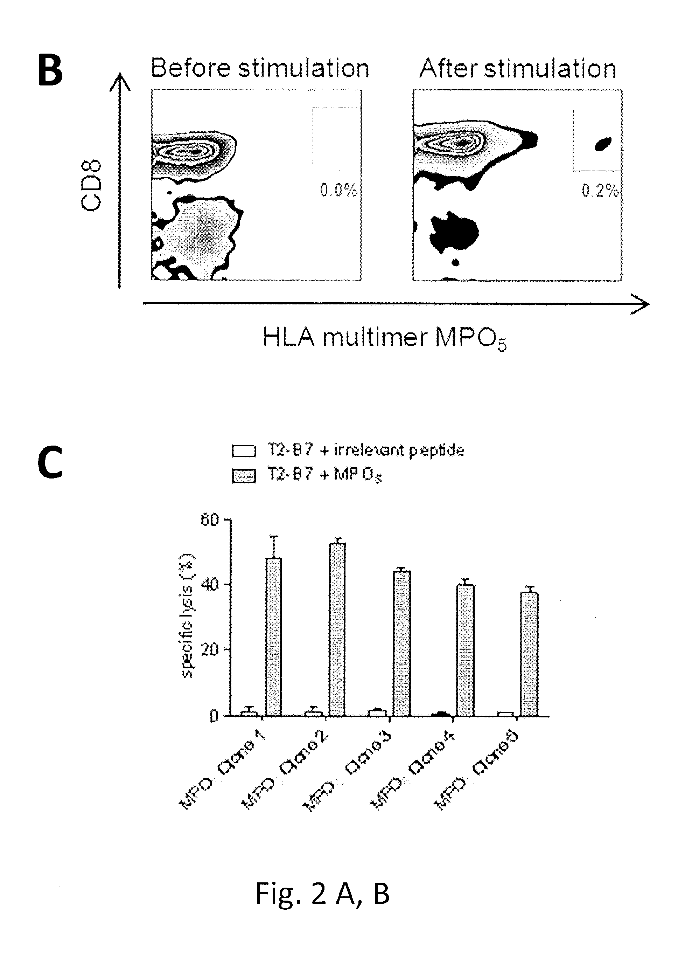

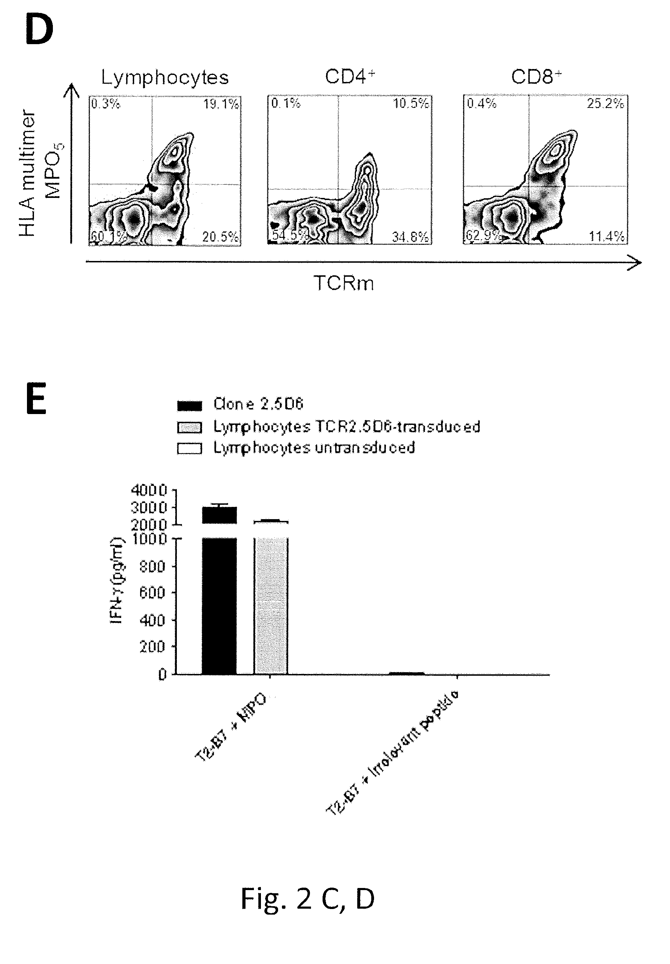

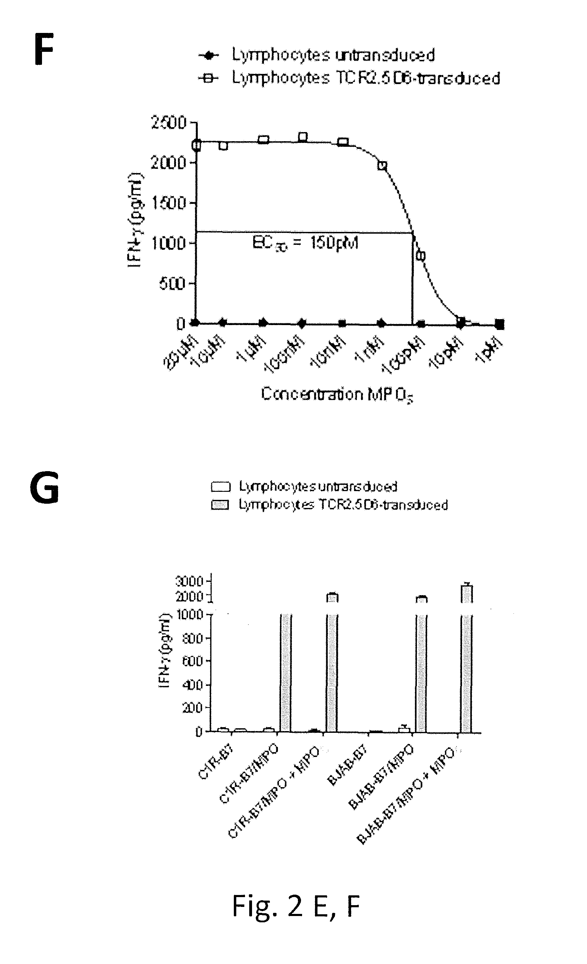

   **Figure 2** shows experimental data on the expansion of T cells specific for the HLA-B7-restricted peptide MPO₅ and the functionality of lymphocytes transgenic for an MPO₅-specific TCR.
      (A) Phenotyping of single HLA-mismatched dendritic cells (DCs) for stimulation with MPO₅. Dendritic cells from an HLA*-B*07:02* negative donor were matured and electroporated with HLA-B**07:02*P2AeGFP encoding *in vitro* transcribed RNA. The degree of maturation of the DCs was determined through the surface markers CD83, CD80 and CD86. Successful electroporation was verified by measuring eGFP expression.
      (B) MPO₅ peptide-specific T cells were expanded after stimulation of naïve T cells from an HLA-B7 negative donor with peptide-pulsed (0.1 µM) single HLA-mismatched DCs. Peptide specificity of expanded T cells was investigated with an MPO₅-specific HLA multimer by flow cytometry.
      (C) Peptide-specific, lytic capacity of cloned T cells at estimated effector to target ratios (25:1) was tested by ⁵¹Cr assay (the mean of duplicates is shown).
      (D) Stimulated peripheral lymphocytes from a healthy donor were transduced with TCR2.5D6 and stained with anti-CD4, anti-CD8, the MPO₅ specific HLA multimer and the TCRm antibody, which is directed against the murinized constant region of the optimized TCR. Binding of MPO₅ specific HLA multimer and expression of TCRm is shown in total lymphocytes as well as T cell subpopulations positive for CD4 and CD8.
      (E) For functional characterization, transduced lymphocytes were co-incubated for 24h with peptide pulsed T2-B7 cells at an effector to target ratio of 2:1. Supernatants were collected and IFN-γ secretion was measured by ELISA (standard deviation of the mean of triplicates in one of two representative experiments is shown).
      (F) For characterization of the functional avidity of the TCR2.5D6, transduced lymphocytes were co-incubated with T2-B7 cells pulsed with graded amounts of MPO₅ at effector to target ratios of 2:1. Supernatants were collected 24h later and IFN-γ secretion was measured by ELISA (standard deviation of the mean of triplicates in one of two experiments is shown).
      (G) Antigen specific IFN-γ release by TCR2.5D6-transduced lymphocytes was determined in response to the MPO-negative cell lines C1R and BJAB, transduced with HLA-B7 alone or HLA-B7 and MPO, respectively. Cell lines transduced with both constructs were additionally pulsed with MPO₅ as positive control, untransduced lymphocytes were used as negative control.

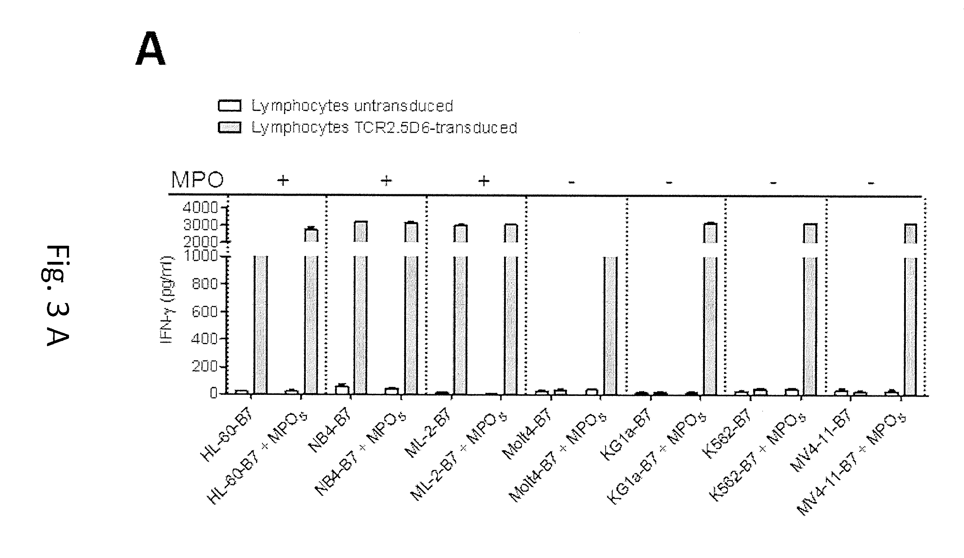

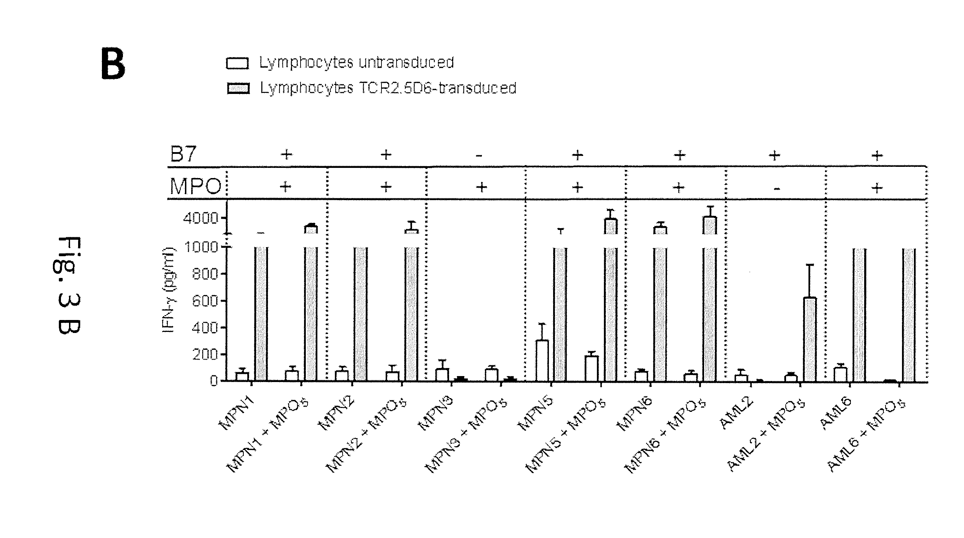

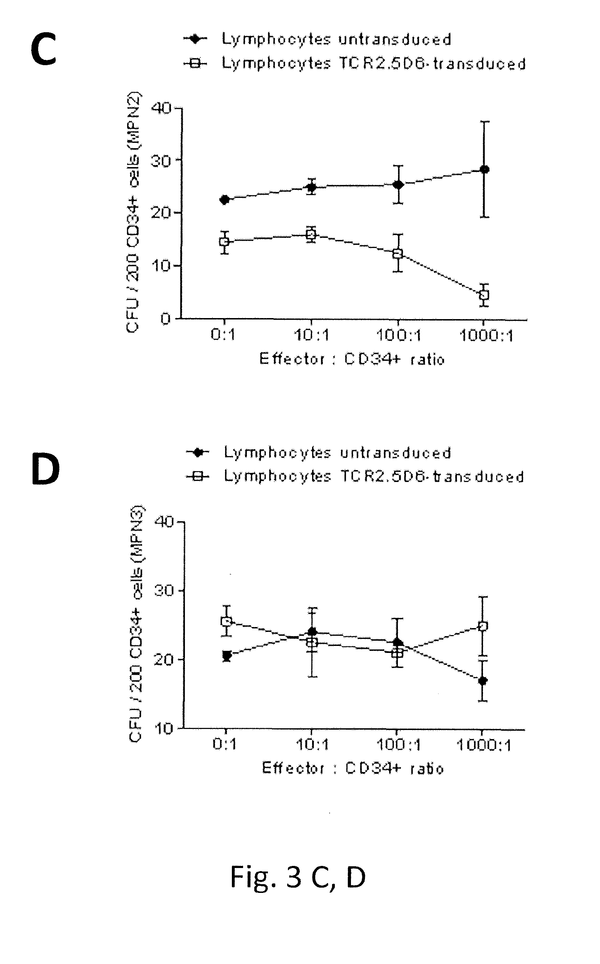

   **Figure 3** shows experimental data characterizing the reactivity of TCR2.5D6-transduced lymphocytes in response to leukemic cell lines and primary leukemia samples.
      Lymphocytes either untransduced or transduced with the TCR2.5D6 (transduction rate: 19%, Figure 2D), were stimulated with target cells for 24h at an effector to target ratio of 2:1. Supernatants were collected and IFN-γ secretion was analyzed by ELISA. Standard deviation of the mean of triplicates in one out of at least two representative experiments is shown.
      (A) Myeloid leukemia cell lines with different MPO expression (Figure 9A and B) were transduced with the retroviral construct *HLA-*B7-P2A-eGFP. Cell lines were additionally pulsed with MPO₅ at a concentration of 1µM as positive controls.
      (B) Primary samples of patients with MPN and AML were pretreated as described in Example 2. MPN1, MPN2, MPN5, MPN6 and AML6 express MPO and HLA-B7, MPN3 expresses MPO but not HLA-B7 and AML2 does express HLA-*B*07:04* but not MPO (Figure 9C and D). As positive control, cells were pulsed with MPO₅ at a concentration of 1µM. Reactivity of TCR2.5D6-transduced lymphocytes in response to colony forming leukemic cells was analyzed by CFU assays. Therefore, CD34⁺ cells (500 cells within a PBMC bulk population) of the leukemic sample MPN2 (MPO⁺, HLA-B7⁺) (C) and MPN3 (MPO⁺, HLA-B7⁻) (D) were incubated with untransduced (black circles) or TCR2.5D6-transduced (open squares) at different effector to target ratios for 15 minutes and plated in duplicates in methylcellulose medium. Colonies were counted 14 days later under a light microscope. The mean of duplicates in one out of two representative experiments is shown.

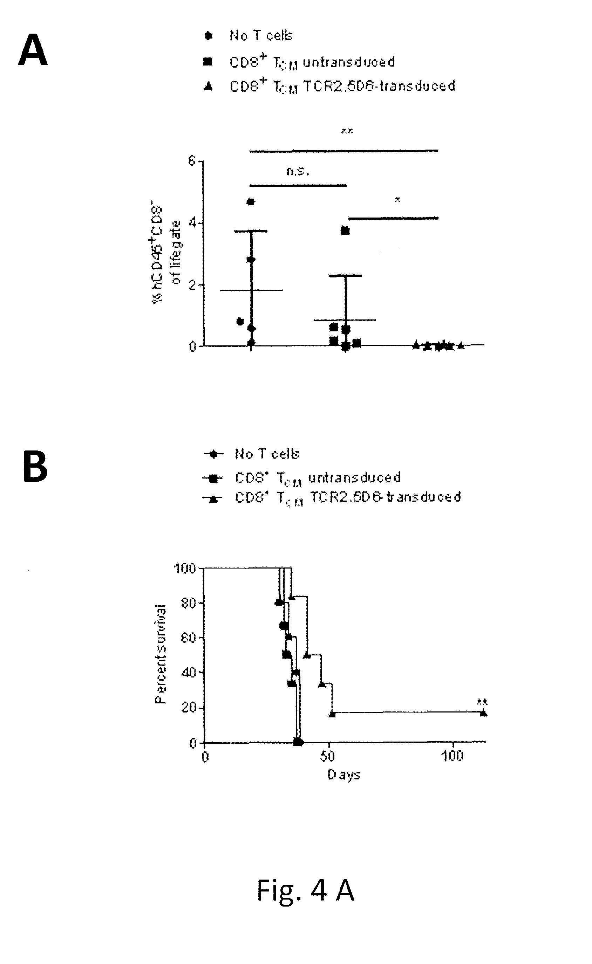

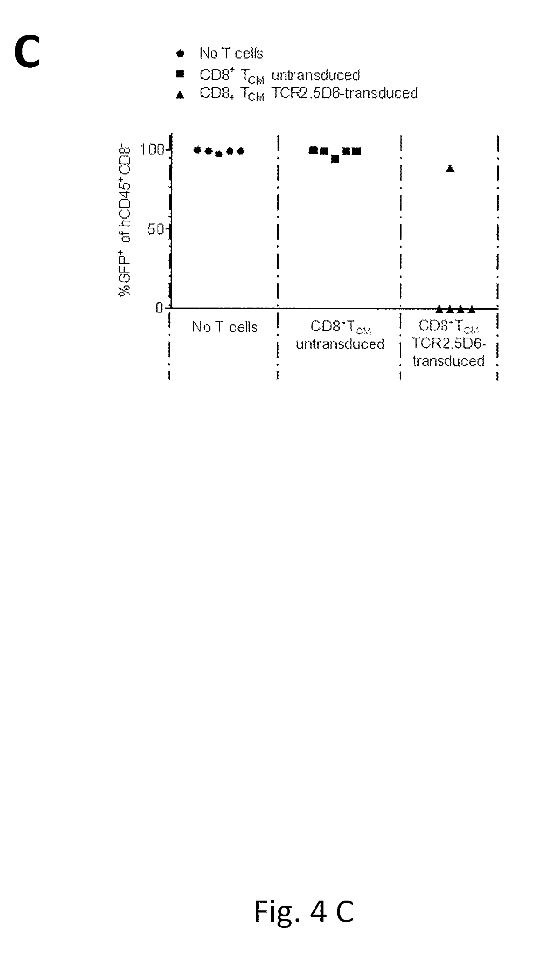

   **Figure 4** shows experimental data indicating that TCR2.5D6-transduced CD8⁺T_{CM} (T_{CM}: central memory T cells) increase survival in a xenogeneic murine AML model.
      BRG mice were irradiated with 3.5 Gy and inoculated intravenously with 1x10⁶ NB4-B7 leukemia cells 24 hours later. T cells were transferred 24h thereafter by intravenous injection of 10x10⁶ untransduced (n = 6) or TCR2.5D6-transduced (n = 6) CD8⁺T_{CM} (Phenotype and functionality: Figure 10). Control mice (n = 5) received 200µl PBS only. Continuous support of human IL-15 was provided by biweekly intraperitoneal injection of 15x10⁶ IL-15 producing γ-irradiated NSO cells.
      (A) Infiltration by human CD45⁺CD3⁻CD8⁻ tumor cells in the bone marrow was analyzed using flow cytometry. Statistical analysis was done using Mann-Whitney test (*P ≤ 0.0247, **P = 0.0043).
      (B) Kaplan-Meier curves are shown for all three groups. Statistical analysis was done between animals receiving untransduced CD8⁺T_{CM} and CD8⁺T_{CM} transduced with TCR2.5D6 using the Mantel-Cox test (**P = 0.0048).
      (C) Diseased mice were sacrificed and the percentage of eGFP-expressing, human CD45⁺ cells was analyzed in resected tumors.

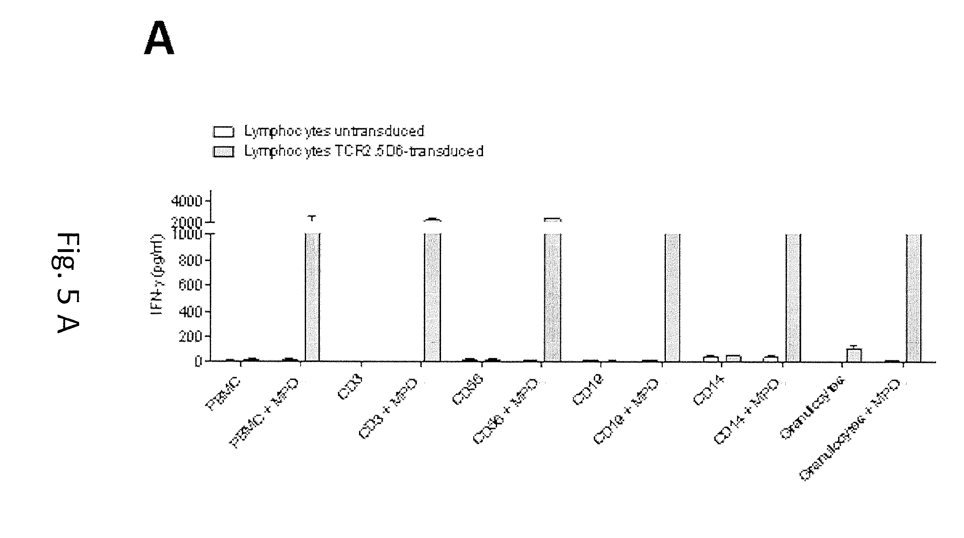

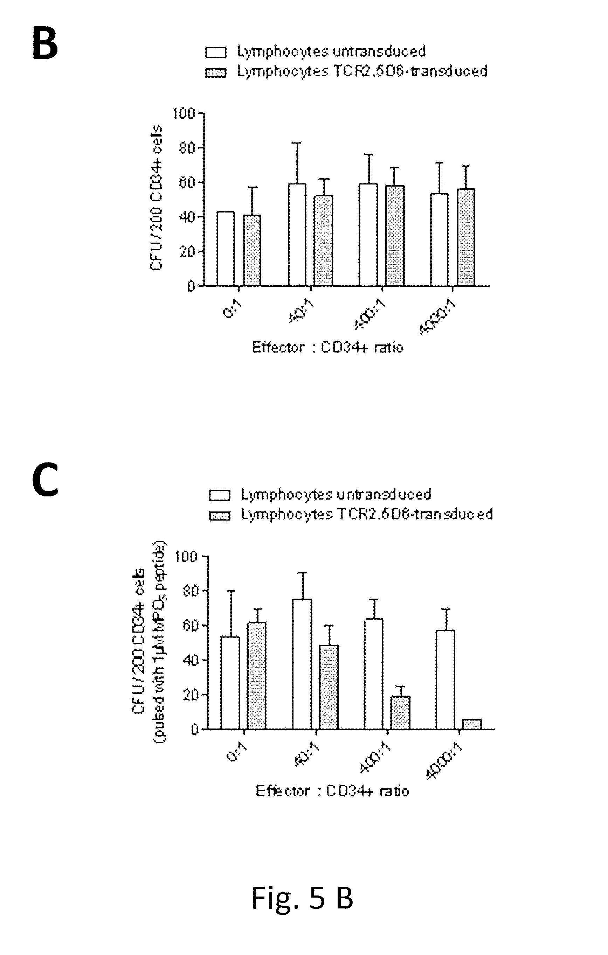

   **Figure 5** shows experimental data examining the hematotoxicity of lymphocytes transduced with the TCR2.5D6.
      (A) Lymphocytes transduced with TCR2.5D6 (transduction rate: 19%, Figure 2D), were tested for potential on-target toxicity. IFN-γ secretion was measured by ELISA after stimulation with different target cells at effector to target ratios of 2:1 for 20h. Standard deviation of the mean of triplicates in one out of at least two representative experiments is shown. Recognition of PBMC subsets of an HLA-B7 positive healthy donor was analyzed by incubation of TCR2.5D6-transduced or untransduced lymphocytes with isolated CD3⁺, CD56⁺, CD19⁺, CD14⁺ cells and granulocytes (MPO-expression data: Figure 15A and B). As positive control, cells were pulsed with 1µM MPO₅.
      (B) Reactivity of TCR2.5D6-transduced lymphocytes against HLA-B7⁺, CD34⁺ hematopoietic stem cells was assessed in CFU assays. CD34⁺ cells (500 cells within a PBMC bulk population) were incubated at different effector to target ratios with untransduced or TCR2.5D6-transduced lymphocytes for 1h. Cells were plated in duplicates in methylcellulose medium. Colonies were counted 14 days later under a light microscope. The mean of duplicates in one out of two representative experiments is shown.
      (C) As positive control, target cells were pulsed with 1µM MPO₅.

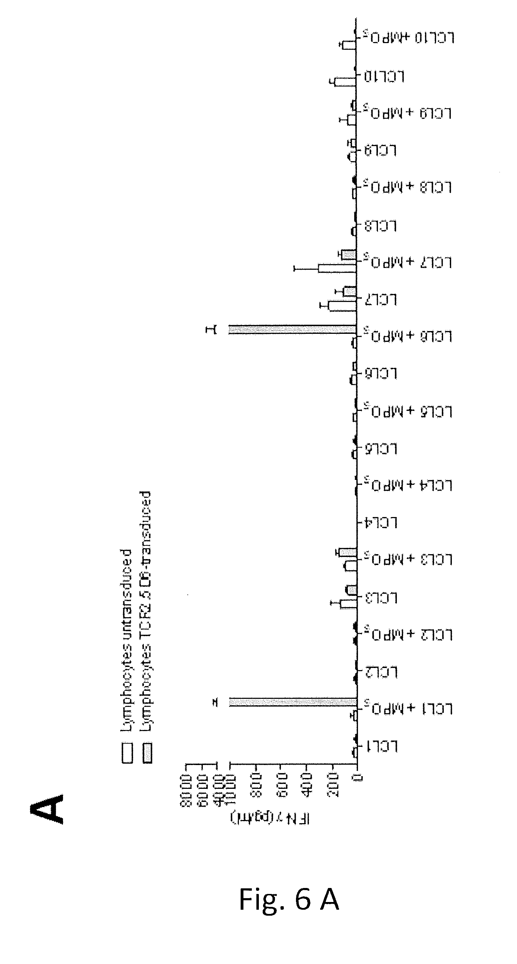

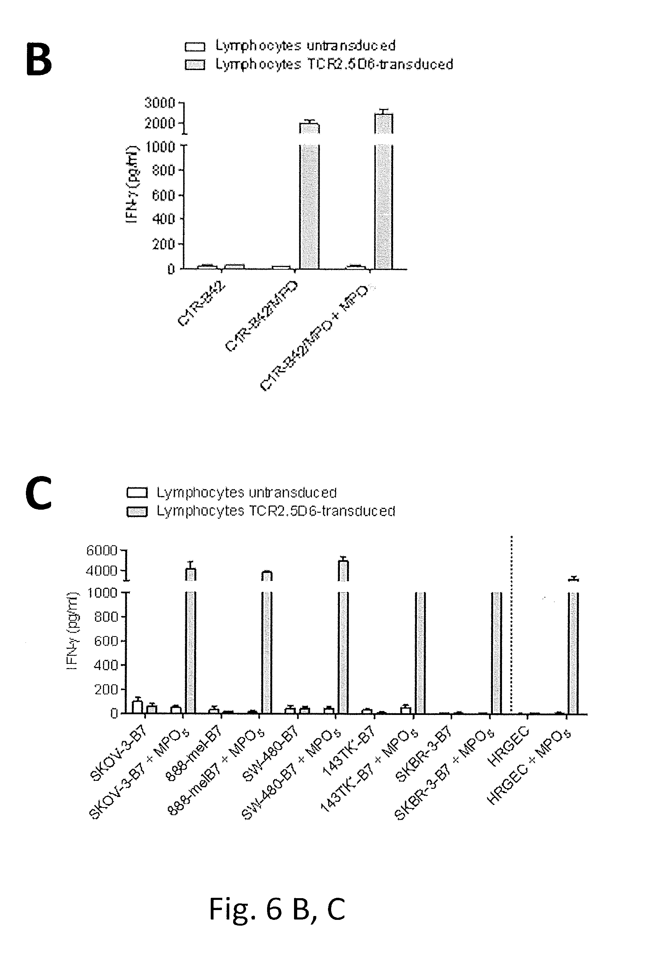

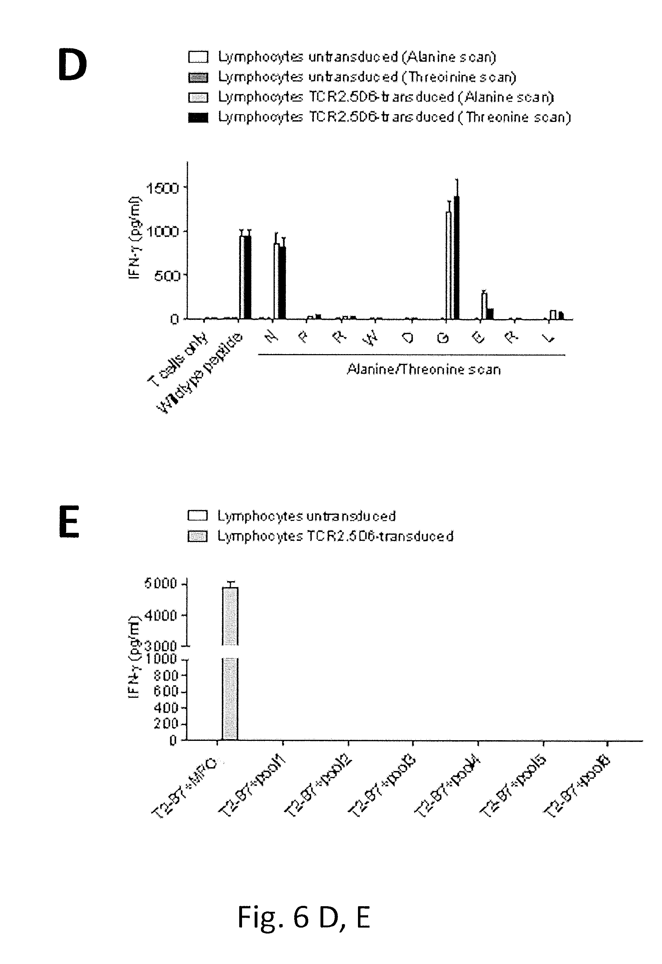

   **Figure 6** shows experimental data studying off-target reactivity of lymphocytes transduced with the TCR2.5D6
      Lymphocytes transduced with TCR2.5D6 (transduction rate: 19%, Figure 2D), were tested for potential off-target toxicity. IFN-γ secretion was measured by ELISA after stimulation with different target cells at effector to target ratios of 2:1 for 20h. Standard deviation of the mean of triplicates in one out of at least two representative experiments is shown.
      (A) Peptide dependent and independent allo-HLA reactivity was measured by IFN-γ secretion after stimulation of TCR2.5D6-transduced or untransduced lymphocytes with LCL expressing common HLA alleles (Table 4). Binding of MPO₅ to diverse HLA alleles was investigated by pulsing LCL with 1 µM MPO₅. LCL1 is HLA-B7⁺ and served as a positive control.
      (B) Lymphocytes untransduced or transduced with TCR2.5D6 were stimulated with C1R cells transduced with HLA*-B*42:01.* Target cells were additionally transduced with MPO and pulsed with MPO₅ peptide.
      (C) Recognition of HLA-B7-transduced, non-myeloid cell lines SKOV-3, 888mel, SW480, 143TK⁻, SKBR-3 (transduction rate 25-50% without sorting and cloning) and primary HRGEC (HLA-B7⁺) by TCR2.5D6-transduced lymphocytes was tested (MPO protein expression: Figure 15 C).
      (D) To determine the MPO₅ amino acid residues critical for recognition by TCR2.5D6, alanine-and threonine-variants of MPO₅ were loaded onto the HLA-B7⁺ cell line LCL1 at a concentration of 1µM. The wildtype MPO₅ peptide was used as a positive control, unpulsed target cells as a negative control.
      (E) 65 naturally presented HLA-B7 ligands were divided into 6 peptide pools. The pools were loaded onto T2-B7 cells at a final concentration of 1µM for each peptide. T2-B7 cells pulsed with MPO₅ were used as a positive control.

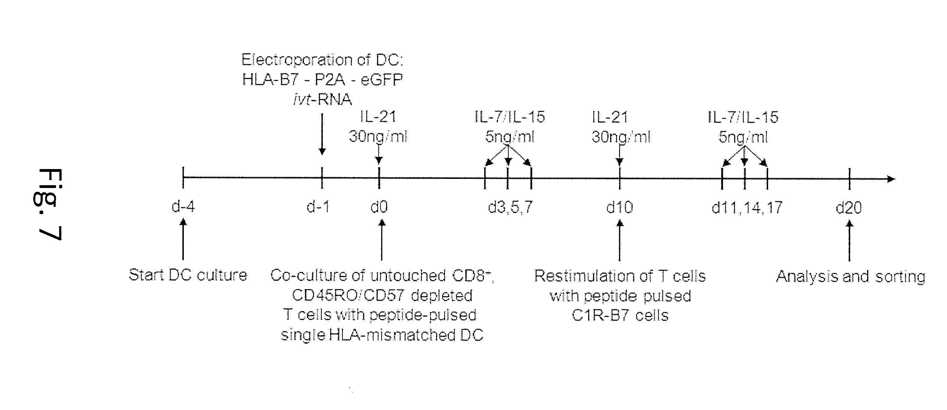

   **Figure 7** shows a schematic overview of the stimulation procedure for MPO-specific T cells in the single HLA-mismatched setting.
      Schematic overview of the single HLA-mismatched T cell priming protocol for expansion of peptide specific T cells recognizing naturally HLA-presented MPO peptides. 3d DCs of a HLA-B7- blood donor were electroporated with HLA-B7-P2A-eGFP *in vitro* transcribed (ivt)-RNA, pulsed with MPO₅ and used for priming of naive T cells from the same donor. Cells were restimulated with MPO₅ pulsed C1R-B7 cells on day ten. Ten days later, expansion of MPO₅ specific T cells was analyzed with an MPO₅ specific multimer and multimer positive cells were sorted on a MoFlo high performance cell sorter.

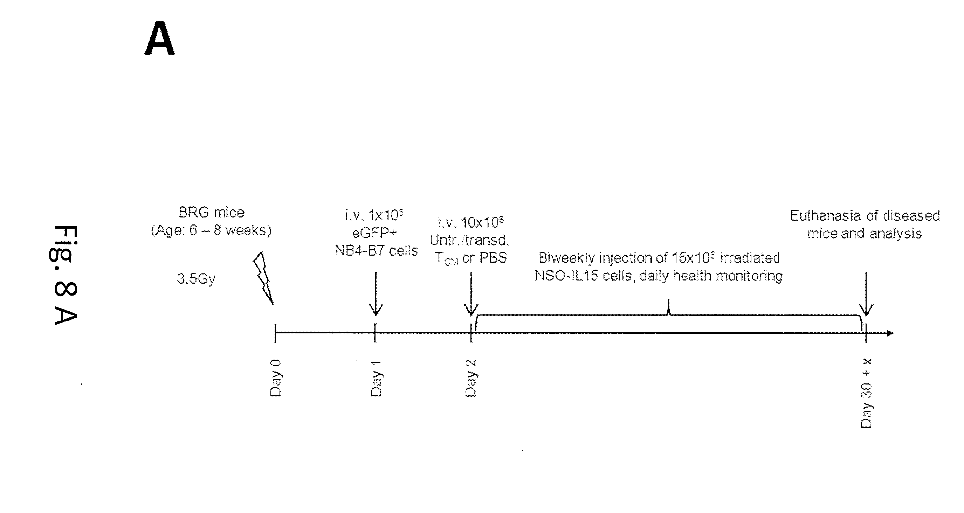

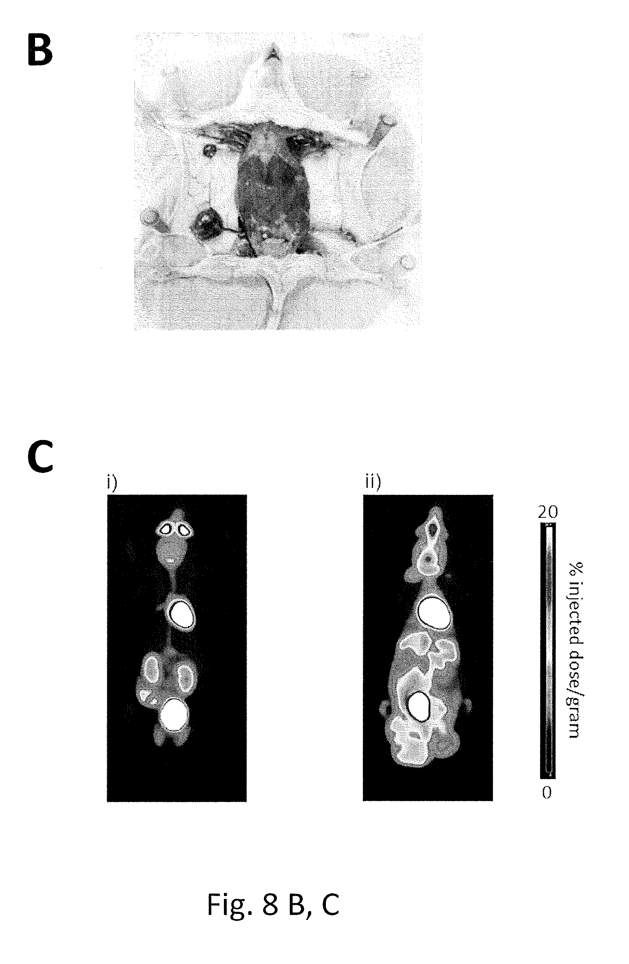

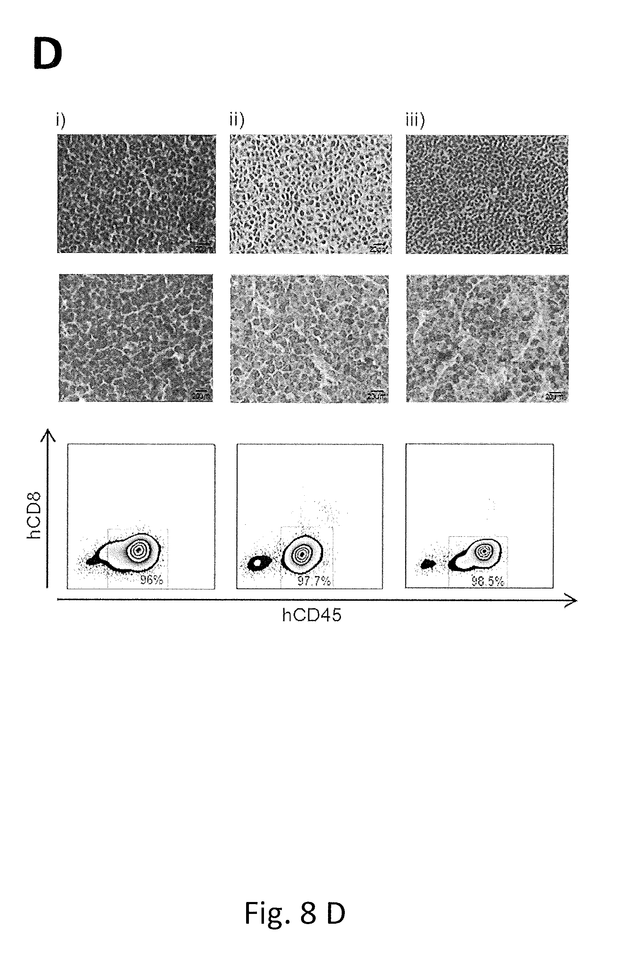

   **Figure 8** shows a detailed characterization of the mouse model used in Figure 4.
      (A) Schematic overview of the xenogenic mouse model used in Figure 4. 6 - 8 weeks old BRG mice were irradiated with 3.5Gy and inoculated i.v. with 1 x 10⁶ HLA-*B***07:02*-P2A-eGFP-transgenic NB4 cells (NB4-B7) the day after. 10 x 10⁶ TCR2.5D6-transduced CD8⁺T_{CM} were injected i.v. one day later; control mice received untransduced CD8⁺T_{CM} or PBS. In order to improve engraftment and persistence of human T cells mice were inoculated with 15 x 10⁶ human IL-15 producing NSO cells i.p. twice a week. Mice were sacrificed when suffering from leukemic disease.
      (B) Typical manifestation of the disease in the lymphnodes of a mouse sacrifized on day 30.
      (C) [₁₈F]-FDG PET of a control mouse that received 200µl PBS i.v. (i) or a mouse that received 1x10⁶NB4-B7 cells (ii) on day 30.
      (D) Representative hematoxylin and eosin, IHC-MPO and human CD45/CD8 FACS-stains of tumors from a mouse that did not receive T cells (i), untransduced (ii) or TCR2.5D6-transduced T_{CM} (iii).
   **Figure 9** shows experimental data obtained from an expression analysis of MPO in HLA-B7-transduced leukemia cell lines and primary leukemia samples used for stimulation of TCR2.5D6-transduced lymphocytes.
      (A) Expression of MPO on the protein level in leukemia cell lines was analyzed by Western Blot. mRNA expression of MPO relative to PBMC was analyzed by qPCR in leukemia cell lines
      (B) and primary leukemic samples (C). Ct values were normalized against the geometric mean of three housekeeping genes (GAPDH, HPRT1, HMBS) using the ddCT method. (D) Expression of MPO in primary leukemia samples of MPN and AML patients was determined by intracellular staining of MPO and analysis on a flow cytometer. MPN samples were treated for six days with 100ng/ml SCF and FLT3-ligand and overnight with 500IU/ml IFN-γ. Samples of patients with AML were treated overnight with 500IU/ml IFN-γ.
   **Figure 10** shows experimental data to characterize the phenotype and functionality of injected CD8⁺T_{CM} for the *in vivo* experiment shown in Figure 4.
      For mouse experiments, CD8⁺CD62L⁺CD45RA⁻ T_{CM} were isolated on day 0 by magnetic bead cell depletion and selection. Cells were activated for two days in the presence of CD3/CD28 beads and 50IU/ml IL-2 and transduced with TCR2.5D6 virus supernatant or left untreated.
      (A) Cells were analyzed for expression of the surface markers CD4, CD8, CD45RA and CD62L. Expression of the transgenic TCR chains was analyzed by staining with the MPO₅ specific HLA multimer and an antibody directed against the murinized regions of the TCR.
      (B) Functionality of the TCR2.5D6-transduced (transduction rate: 47%) or the untransduced CD8⁺T_{CM} cells was tested. Lymphocytes were incubated with T2-B7 cells pulsed with MPO₅ or an irrelevant peptide and NB4-B7 leukemia cells as well as NB4-B7 cells were pulsed with 1µM MPO₅, as positive control, at an effector to target ration of 2:1. Cells were incubated for 20h and IFN-γ secretion was measured in the supernatant by ELISA. Standard deviation of the mean of triplicates is shown.
      (C) Cytotoxic activity was assessed in the same preparation as Figure 10B 52h later by determination of the percentage of eGFP-expressing target cells in the lifegate.

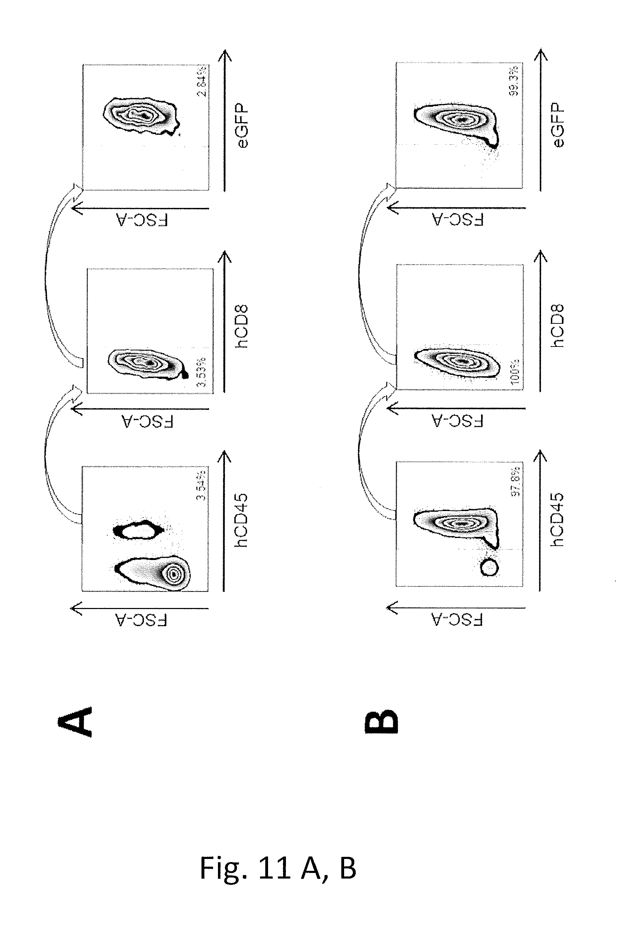

   **Figure 11** shows the gating strategy for the analysis of eGFP expression of human CD45⁺CD8⁻ cells in bone marrow and resected tumors in mice from the experiment shown in Figure 4 Single cell suspensions were stained with 7-AAD for exclusion of dead cells. Cells were further stained with an antibody specific for human CD45 and CD8 and analyzed for their eGFP expression. Percentage of cells in the lifegate is shown. (A) Bone marrow isolated out of the femur of a mouse that did not receive T cells. (B) Single cell suspensions of pooled tumors of an individual mouse that received untransduced T cells.

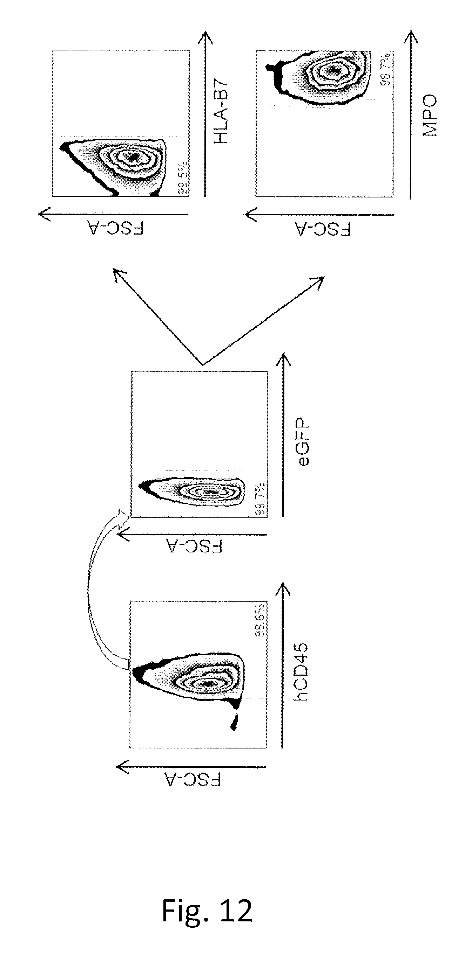

   **Figure 12** portrays experimental data showing the loss of eGFP and HLA-B7 but not MPO expression in the tumor of a mouse (Figure 4) treated with TCR2.5D6-transduced CD8⁺T_{CM}. The tumor of a mouse that was treated with TCR2.5D6-transduced CD8⁺T_{CM} was resected, a single cell suspension was obtained and cells were stained for hCD45, HLA-B7 and intracellular MPO. Viable, human CD45⁺, eGFP⁻ cells were analyzed for HLA-B7 and MPO expression by flow cytometry.

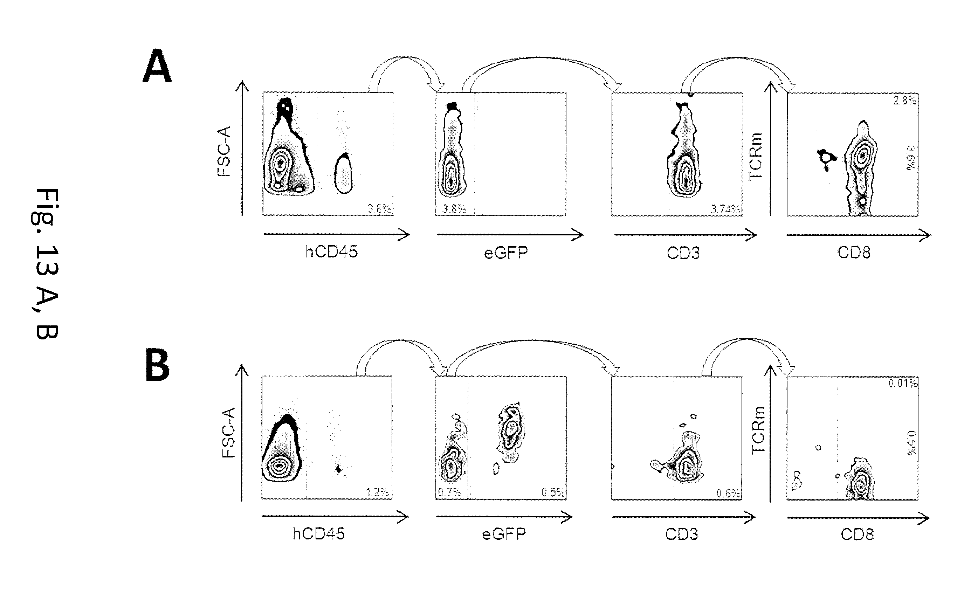

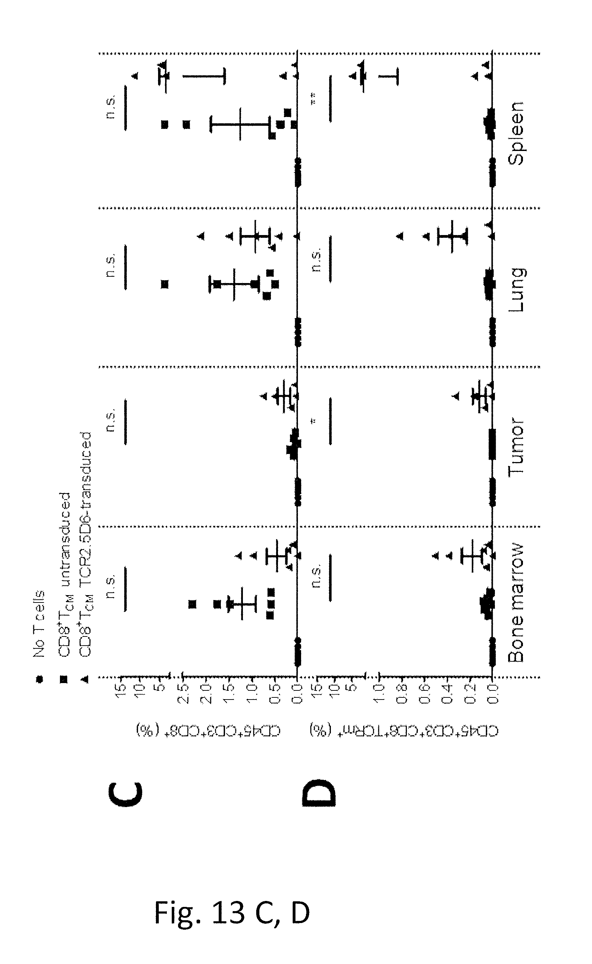

   **Figure 13** shows experimental data indicating organ-infiltration of human T cells in the xenogenic murine AML model (Figure 4).
      Diseased mice were sacrificed and organs were analyzed for T cell infiltration by flow cytometry. The gating strategy for infiltration of human T cells into the spleen of a mouse that received TCR2.5D6-transduced CD8⁺T_{CM} is shown in (A), for a mouse that received untransduced CD8⁺T_{CM} in (B). Infiltration of human CD45⁺CD3⁺CD8⁺ (C) or CD45⁺CD3⁺CD8⁺TCRm⁺ (D) T cells in bone marrow, tumor, lung and spleen was determined in all groups. Percentages of lifegate are shown. Data of groups "CD8⁺T_{CM} untransduced" and "CD8⁺T_{CM} TCR2.5D6" were compared using Mann-Whitney test (*P = 0.0117, **P < 0.0087).

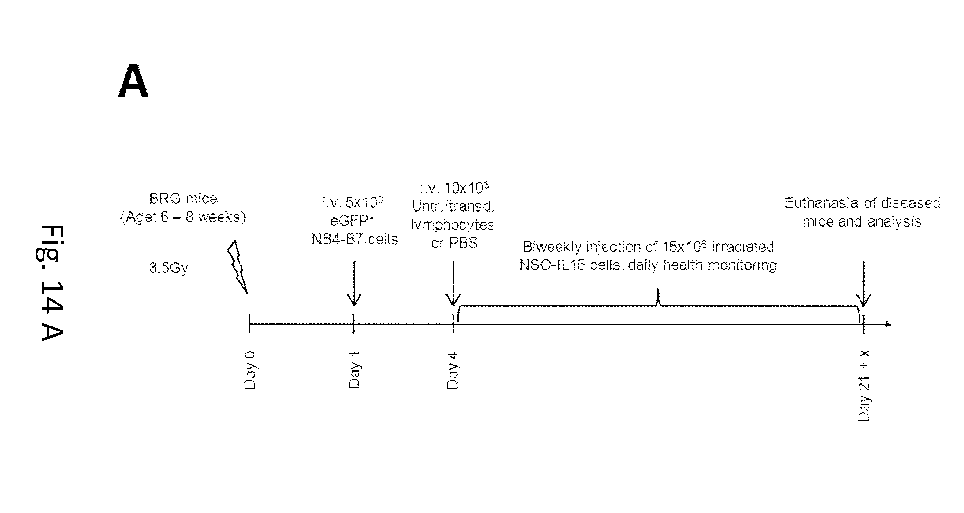

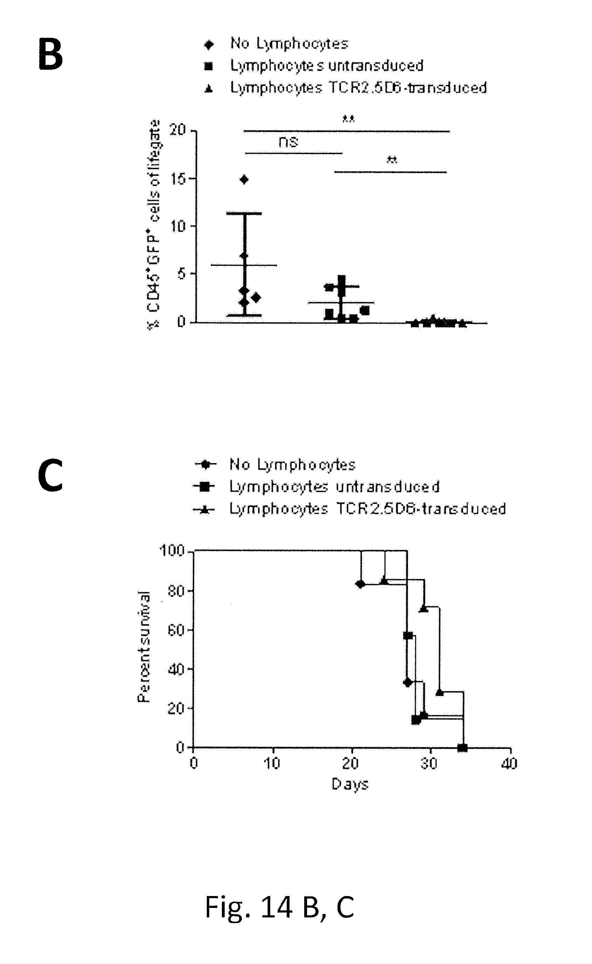

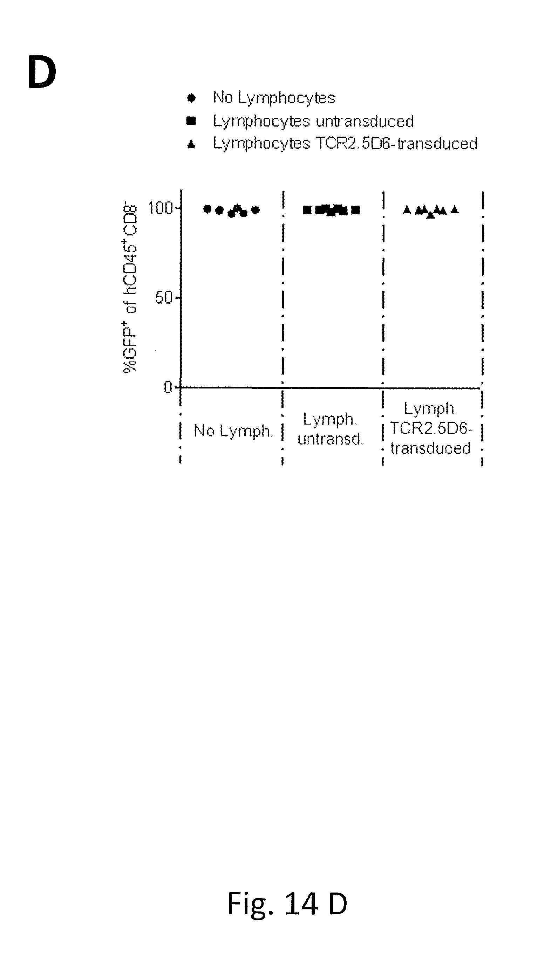

   **Figure 14** shows anti-leukemic reactivity of TCR2.5D6-transgenic unselected lymphocytes *in vivo.*
      (A) Schematic overview of the experimental process. BRG mice were irradiated with 3.5 Gy and inoculated with 5x10⁶ NB4-B7 cells via the tail vein. Three days later, no lymphocytes, 10x10⁶ untransduced or TCR2.5D6-transduced lymphocytes as depicted in Figure 2D and E were injected i.v. Systemic support of human IL-15 was applied by biweekly i.p. injection of 15x10⁶ irradiated NSO-IL15 cells. Mice were sacrificed when suffering from disease with respective criteria as written in Example 2.
      (B) Bone marrow of sacrificed mice was isolated and the percentage of CD45⁺eGFP⁺ cells was determined by flow cytometry. Statistical analysis was done using the Mann-Whitney test (**P< 0.0025).
      (C) Kaplan-Meier curves are shown for all three groups.
      (D) Diseased mice were dissected between day 21 and 34, visible tumors were resected and further prepared to obtain single cell suspensions. Pooled suspensions from each individual mouse were analyzed for their percentage of hCD45⁺CD8⁻ cells expressing eGFP by flow cytometry.
   Figure 15 shows experimental data on the expression of MPO in primary hematopoietic cells and non-hematopoietic cell lines.
      (A) mRNA expression of MPO relative to PBMC was analyzed by qPCR in PBMC subsets and granulocytes. Ct values were normalized against the geometric mean of three housekeeping genes (GAPDH, HPRT1, HMBS) using the ddCT method. Expression of MPO in the bone marrow compared to PBMC is shown as a positive control.
      (B) Expression of MPO in negatively isolated PBMC subsets and granulocytes was analyzed by intracellular staining of MPO and flow cytometry analysis. All isolated PBMC subsets were stained with an anti-human MPO antibody and a subset specific surface marker. Granulocytes were identified according to a high granularity in the side scatter.
      (C) Expression of MPO in cell lines of non-hematopoietic origin was analyzed by Western Blot. Lysates of HL-60-B7 cells were loaded as a positive control.

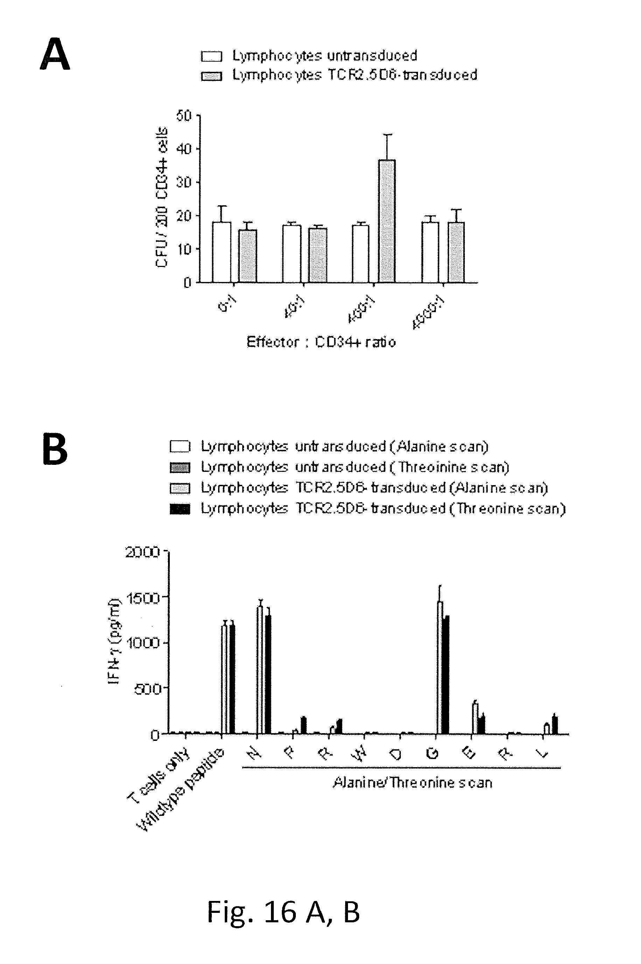

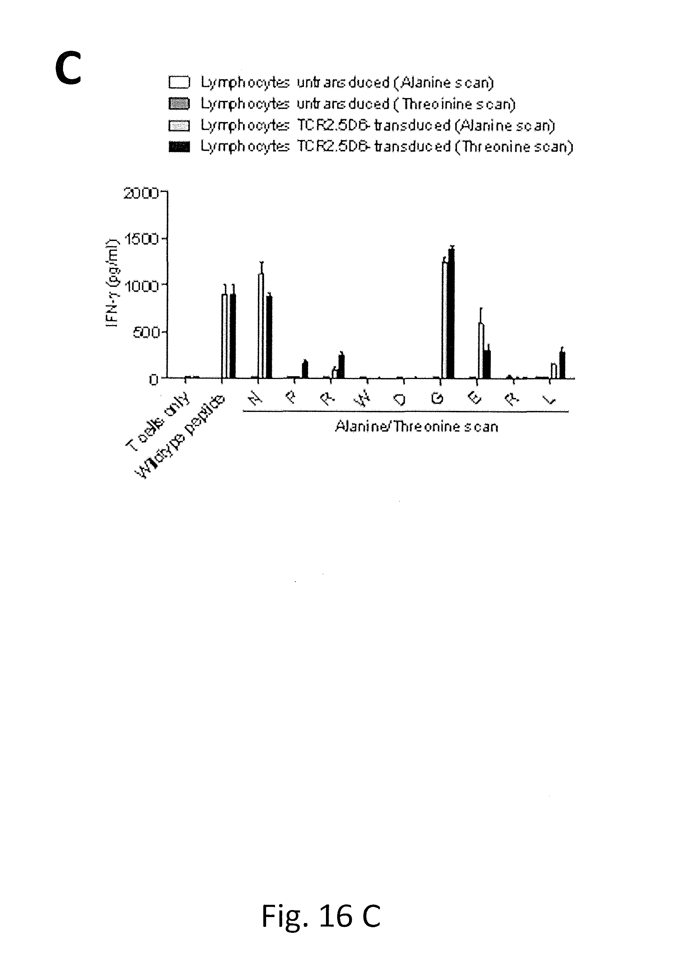

   **Figure 16** shows additional data for stem cell cytotoxicity and reactivity against alanine- and threonine-variants
      (A) Reactivity of TCR2.5D6-transduced lymphocytes against HLA-B7+, CD34+ hematopoietic stem cells was assessed in CFU assays. CD34+ cells (500 cells within a PBMC bulk population) were incubated at different effector to target ratios with untransduced or TCR2.5D6-transduced lymphocytes for 24h. Cells were plated in duplicates in methylcellulose medium. Colonies were counted 14 days later under a light microscope. The mean of duplicates is shown. Alanine and threonine exchange-variants of the MPO₅ peptide were analyzed for their recognition by TCR2.5D6-transduced lymphocytes in context with HLA-*B*07*:02. Therefore, the HLA-B7 positive cell lines LCL11 (B) and LCL12 (C) were pulsed with 1µM of the respective peptide and co-incubated with untransduced or TCR2.5D6-transduced lymphocytes at effector:target ratios of 2:1. Supernatants were analyzed for the amount of IFN-γ via ELISA 24h later. Standard deviation of the mean of triplicates in one out of three experiments is shown.

In the following, reference is made to the examples, which are given to illustrate, not to limit the present invention.

### Examples

### Example 1

### Primary material and cell lines

Blood from healthy individuals and patients with diverse hematopoietic malignancies as well as human healthy organ tissue samples obtained from diagnostic preparations were collected after informed consent following requirements of the local ethical board and principles of the Helsinki Declaration. Patient characteristics are shown in Table 3. PBMC (Peripheral Blood Mononuclear Cells) were isolated by density gradient centrifugation using Ficoll/Hypaque (Biochrom). Granulocytes were purified from whole blood by differential centrifugation as described previously (Eggleton et al., 1989). All target cells were maintained in RPMI 1640 (Invitrogen) supplemented with Penicillin/Streptomycin (PAA) and 10% fetal calf serum (Invitrogen) unless otherwise stated. Details regarding isolation of naive T cells and T_{CM} as well as the cell lines used in the present study can be found in Example 2. Transduction of cell lines with HLA-B7-P2A-eGFP is indicated by the additional specification "-B7" to the name of the cell line, transduction with MPO-P2A-dsRed by the additional specification "/MPO". For functional *in vitro* assays, CML and AML samples as well as non-lymphoid cell lines were treated as described in Example 2. Primary human renal glomerular epithelial cells (HRGEC) were purchased from ScienceCell and cultured according to the manufacturer's instructions. The hybridoma cell line HB-95 (ATCC (American Type Culture Collection)) was used for in-house production of the w6/32 antibody following standard protocols.

### Immunopeptidomic identification of MPO HLA ligands

HLA I-restricted peptides were identified and analyzed by the immunopeptidomic approach as previously described (Bassani-Sternberg et al., 2010; Falk et al., 1991). After HLA I immunoprecipitation and peptide elution, peptides were analyzed by nano-HPLC (Ultimate 3000, Dionex) coupled to a linear quadruple ion trap-Orbitrap (LTQ Orbitrap XL) mass spectrometer (Thermo Fisher) equipped with a nano-electrospray ionization (ESI) source as previously described (Hauck et al., 2010), allowing for the fragmentation of peptides with charges +1 to +3.

MS/MS data were analyzed using the software tools MASCOT, Peaks and Pep-Miner and potential HLA ligands were further identified and selected as described in Example 2. To screen for HLA ligands derived from genes with suitable expression patterns for T cell targeting, gene expression was determined by database and literature research (http://genome.ucsc.edu/cgi-bin/hgBlat, http://www.biogps.org, http://www.ncbi.nlm.nih.gov/ pubmed/). For sequence validation of selected candidate ligands, synthetic counterparts (IBA) were analyzed by mass spectrometry. After normalization, spectra were matched using mMass (Niedermeyer et al., 2012).

### MPO expression analysis by semi-quantitative real-time polymerase chain reaction, immunohistochemistry and Western Blot

To verify the expression profile of MPO in primary cells as well as diverse human tissue, semi-quantitative real-time PCR (qPCR), immunohistochemistry and Western Blot was conducted as described in Example 2.

### Expansion of T cells specific for a HLA-B7-restricted MPO epitope

To expand T cells with specificity against the selected HLA ligands, we used a single HLA mismatch priming approach as previously published (Wilde et al., 2009) with some modifications (Figure 7 and Example 2). Expanded T cells were analyzed and sorted on day 20 using HLA multimers on a high-performance cell sorter (MoFlo; Dako) and cloned by limiting dilution.

### HLA multimers and antibodies

HLA multimers were synthesized as previously reported (Knabel et al., 2002). Antibodies used for flow cytometry, western blot and immunohistochemistry are listed in the Table 5.

### Retroviral TCR transfer into PBMC

The sequence of the reactive TCR2.5D6 was determined by PCR and sequencing as previously described (Schuster et al., 2007; Weigand et al., 2012). A bi-cistronic construct separated by a P2A element consisting of beta and alpha chains containing murinized constant chains and an additional disulphide bridge was generated *in silico* and codon-optimized (Invitrogen) as previously described (Cohen et al., 2006; Kuball et al., 2007; Scholten et al., 2006). The construct was cloned into the retroviral vector pMP71 (Fehse et al. 2002), retroviral supernatants were generated and PBMC or CD8⁺T_{CM} were transduced as previously described (Weigand et al., 2012; Wang et al., 2012).

### Functional characterization of T cell clones as well as TCR-transgenic T cells

T cells were tested for their cytotoxic reactivity against ⁵¹Cr labeled target cells in a standard 4-hour ⁵¹Cr-release assay (Schuster et al., 2007) or by a flow cytometry-based cytotoxic assay. For cytokine detection, effector and target cells were incubated at a ratio of 2:1 for 24h. Supernatants were collected and cytokine levels were measured using IFN-γ enzyme linked immunosorbent assay (ELISA) (BD) according to the manufacturer's instructions. Alanine-variants of the MPO₅ peptide (Genscript) were used to define the residues critical for recognition by TCR2.5D6 as described in Liang et al., 2010. The ScanProsite tool was used for identification of proteins that contain the motif "XPRWDXXRL" as described in Linette et al., 2013. HLA-B7 ligands analyzed for off-target reactivity were synthesized on an automated peptide synthesizer EPS221 (Abimed, Langenfeld, Germany) following the 9-fluorenylmethyl-oxycarbonyl/tertbutyl (Fmoc/tBu) strategy as described (Schirle et al., 2000).

### In vivo anti-leukemic reactivity of TCR2.5D6-transduced T cells

*C.Cg-Rag2^{tm1Fwa} Il2rg^{tm1Sug}*/JicTac mice on a BALB/c background (BRG mice) were purchased from Taconic and maintained in an animal facility. Mice were sublethally irradiated with 3.5 Gy on day 0 and 1x10⁶ NB4-B7 cells were injected intravenously the day after. One day later, mice received either 200µl PBS, 1x10⁷ untransduced or 1x10⁷ 2.5D6TCR-transduced CD8⁺T_{CM} intravenously. To provide a systemic supply of human IL-15, 1.5x10⁷, irradiated NSO-IL-15 cells (kindly provided by Stanley Riddell) were injected intraperitoneally twice per week as described in Wang et al., 2011. Diseased mice (Criteria: Example 2) were sacrificed and analyzed for tumor load and T cell engraftment by flow cytometry after organ collection and preparation of single cell suspensions.

### Hematopoietic colony forming assays

All hematopoietic progenitor assays were performed according to the manufacturer's instructions (Stem Cell Technologies; Vancouver, Canada). PBMC samples containing a total of 500 CD34⁺ cells were co-incubated with either TCR2.5D6-transduced or untransduced lymphocytes. Incubation was performed in 250µl alpha MEM medium (PAA) supplemented with 2% FCS at different effector to target ratios for 15 minutes or 1h at 37°C. Subsequently, cell suspensions were mixed with 2.5ml MethoCult H4435 (Stemcell Technologies) and plated in duplicates of 1.1ml in 35mm petri dishes. After 12-14 days of incubation at 37°C/5% CO₂ in a humidified atmosphere, plates were scored for colony growth under a light microscope.

### Example 2

### Animal experiments

Health status of the mice was monitored daily by an independent, experienced research assistant in the animal facility. Mice that suffered from health problems indicated by weight loss (>10%), tumor manifestation (>1 cm), rough hair-coat, changes in breathing rate or reduced mobility were sacrificed.

### MS/MS data analysis and identification of potential HLA ligands

MS/MS data was analyzed using three different data analysis tools, namely MASCOT (Matrix Science), Peaks (Bioinformatics Solutions Inc.) (Zhang et al., 2012) and Pep-Miner (IBM Haifa Research Lab) (Beer et al., 2004). Database searches were performed using the human part of the NCBI and UniProt database and the Human Short Peptide Variation Database (http://srs.bioinformatics.nl/hspv/search.php). Error tolerance was set to 10ppm for parent ions and 0.5Da for fragment ions. Peptides with a length of 8-11 amino acids, a MASCOT score ≥ 30, a Peaks -logP10 ≥ 15 or a Pep-Miner score ≥ 70 were analyzed for described anchor residues (http://www.syfpeithi.de/) for the respective HLA type of the patient samples.

### Expression analysis of MPO

For expression analysis of MPO on the mRNA level by qPCR, total RNA was isolated or used from the Human Total RNA Master Panel II (Clontech). cDNA was synthesized by the AffinityScript Multiple Temperature Reverse Transcriptase (Agilent Technologies). qPCR was conducted on a LightCycler 480 System (Roche) using the KAPA SYBR Fast LightCycler 480 reagent (Peqlab). GAPDH, HMBS and HPRT1 were applied as control genes for relative quantification which was calculated by the delta-delta Ct method (Livak et al., 2001) using the geometric mean of GAPDH, HMBS and HPRT1 for normalization. Pooled RNA from PBMC of three different healthy donors was used as a reference in all qPCR experiments.

MPO immunohistochemistry was done using a rabbit anti-human MPO antibody (Dako) on an automated immunostainer (Ventana Medical Systems, Tucson, AZ, USA). Two independent observers analyzed MPO-stained slides for cytoplasmatic positivity. For expression analysis of MPO on the protein level, SDS-PAGE followed by Western Blot was performed using MPO specific (EPR4792, Epitomics) and GAPDH specific (6C5, Affinity BioReagents) antibodies for loading control.

### Constructs used for testing of specificity and HLA restriction of isolated T cells and clones

To transduce the restriction element HLA-B7 and the antigen MPO (NM_000250.1) into cell lines not expressing these genes, genes were cloned into the retroviral vector MP71 together with eGFP or dsRed, respectively as bi-cistronic constructs separated by a Porcine teschovirus-1-derived peptide element (P2A). For generation of *in vitro* transcribed (*ivt*)-RNA, the respective HLA molecule was cloned into the plasmid pcDNA3.1(-) (Invitrogen) together with eGFP, separated by a P2A element. eGFP and dsRed were used to follow gene-transfer efficacy and to allow untouched sorting of modified cells without the need for specific antibodies. Unless otherwise stated, transduced cells were sorted according to fluorescent dye expression and cloned by limiting dilution.

### Primary cells and cell lines

The following cell lines were used for analysis of MPO specific T cell receptors: T2 (ATCC; American Type Culture Collection, Manassas, VA), C1R (Falk et al., 1995), NB4 (Cell lines service, CLS, Germany), MV4-11 (CLS), HL-60 (CLS), ML-2 (The CABRI consortium), KGla (CLS), K562 (ATCC), BJAB (kindly provided by J. Mautner), Molt4 (CLS), SKOV-3 (ATCC), 888-mel (NIH), 143TK⁻ (kindly provided by Ralph Mocikat), SW-480 (ATCC), SKBR-3 (CLS) and different lymphoblastoid cell lines (LCL) (kindly provided by Steve Marsh; see also Table 4). 293 HEK embryonal kidney cells (ATCC) were used for production of retroviral supernatants. For functional in vitro assays, CML and AML samples as well as non-lymphoid cell lines were treated for 24h with 500 U/ml IFN-γ (Peprotech). Cryopreserved primary cells of CML patients were additionally treated for six days with stem cell factor (SCF) and FLT3 ligand (Peprotech) at a concentration of 100ng/ml (Volpe et al., 2007).

### Isolation of naïve T cells and CD8⁺T_{CM}

For isolation of untouched, naive CD8⁺ T cells, CD8⁺ T cells were negatively isolated with the Dynabeads Untouched Human CD8 T cell isolation kit (Invitrogen) according to the manufacturer's instructions. Antigen experienced T cells were further depleted with CD45RO-and CD57-microbeads (Miltenyi). For isolation of CD8⁺CD62L⁺CD45RA⁻ T_{CM}, CD4⁺ and CD45RA⁺ cells were first depleted followed by positive selection of CD62L⁺ cells using fluorescent antibodies (BD) and microbeads (Miltenyi).

### Stimulation of naïve T cells in the single HLA-mismatched setting

Monocytes of a HLA-B7 negative donor were differentiated into DC by incubation with IL-4 (20ng/ml) and GM-CSF (100ng/ml) (Peprotech) for 48h. Cells were further matured using TNFα (10ng/ml), IL-1β (10ng/ml), IFN-γ (5000IU/ml), PGE₂ (250ng/ml) (Peprotech) and CL075 (1µg/ml) (Invivogen) for 24h. DC transfected with *ivt*-RNA encoding HLA-B7 and eGFP were pulsed with the respective peptide at a concentration of 0.1µM for 2h in AIM-V medium (Invitrogen). Naïve T cells from the DC donor were isolated as described above. Priming was started at an effector to target ratio of 10:1 in the presence of IL-21 (30ng/ml) (Peprotech). IL-7 and IL-15 (5ng/ml) (Peprotech) were added every two to three days. T cells were restimulated using C1R-B7 cells, pulsed with 0.1µM of the respective peptide on day 10.

### Statistics

Statistical analysis was performed using GraphPad Prism version 5.01 (GraphPad Software, Inc.). Survival data was analyzed using the Mantel-Cox test and statistical difference in infiltration of human T cells in mouse organs was analyzed using the Mann-Whitney unpaired t test. Data was considered to be significantly different when P value < 0.05, < 0.01 or < 0.001 represented in the figures as *, ** or ***, respectively.

### Example 3

### Identification of leukemia-derived HLA ligands and selection of MPO as potential T cell target

All methods mentioned in this example were carried out as described in Examples 1 and 2 above.

Novel target antigens expressed and presented by myeloid leukemias that could be targeted by T cells were identified by analysis of the immunopeptidome of malignant cells obtained from patients with MPN. By mass spectrometry, 4386 unique peptides with adequate peptide length and proper anchor residues to serve as potential ligands of diverse HLA class-I molecules were identified. These peptides were then analyzed for their suitability as potential target structures for T cell mediated anti-tumor immunotherapies. Nineteen of those peptides are derived from 7 genes with presumably restricted expression in hematopoietic cells (Table 1). By three different data analysis tools, five naturally presented MPO-derived HLA ligands were detected which were restricted to 4 different HLA alleles, namely HLA*-A*01:01,* HLA*-B*07:02,* HLA*-B*15:01* and HLA*-B*44:02* (Table 2).

For sequence validation of MPO-derived HLA ligands, defined peptides were synthesized and reanalyzed by mass spectrometry. Resulting spectra were compared with the spectra of the naturally eluted counterpart. All peptides showed overlapping fragmentation patterns (Figure 1A). For confirmation of restricted expression of MPO to myeloid cells, qPCR and immunohistochemistry were performed. High MPO expression was observed in most MPN and AML leukemia samples as well as fetal liver and healthy bone marrow. Very low MPO gene expression was observed in mature PBMC subpopulations including CD14⁺ monocytes and granulocytes as well as solid organ tissues (Figure 1B). Immunohistochemistry was performed on healthy organ tissues demonstrating MPO protein expression only in single myeloid cells within diverse organs with the exception of bone marrow, where expression could be detected in myeloid progenitor cells (Figure 1C).

### Example 4

### Isolation and retroviral transfer of an allorestricted MPO₅-specific TCR and confirmation of MPO-specific target cell recognition

All methods mentioned in this example were carried out as described in Examples 1 and 2 above.

Focusing on the two MPO-derived peptides presented by HLA-B7 (MPO₂ and MPO₅), it was determined whether high affinity T cells specific for these two MPO peptides presented by HLA-B7 could be isolated in the single HLA-mismatched setting.

For stimulation in a single HLA-mismatched setting, dendritic cells (DCs) of an HLA*-B*07:02* negative donor were electroporated with *in vitro* transcribed RNA (ivt RNA) coding for eGFP. eGFP served as evidence for successful electroporation and is separated from the gene for HLA-*B*07:02* by a P2A element. As can be seen from Figure 2A, generation of single HLA-mismatched DCs was successful. Expression of the surface markers CD83 and CD86 provides evidence for successful maturation of the DCs, expression of eGFP shows that the electroporation with HLA-*B***07:02*P2AeGFP ivt-RNA was successful. The dendritic cells were loaded with the peptide MPO₅ and used for stimulation of naïve T cells of the same donor. The experimental procedure for priming of naïve T cells is shown in Figure 7. Staining of proliferated cells after restimulation on day 20 with an MPO₅ HLA multimer demonstrated a distinct population of peptide-specific T cells (Figure 2B). The MPO₅ HLA multimer⁺ T cells were sorted and cloned by limiting dilution. The resulting T cell clones were tested for their cytotoxic activity against T2-B7 cells, pulsed with MPO₅ or an irrelevant peptide. Five clones were selected that only lysed T2-B7 cells pulsed with MPO₅ (Figure 2C). TCR analysis revealed that all 5 T cell clones expressed the same Vα and Vβ chains, namely Vα1.1 and Vβ13.2. Murinized and optimized TCR Vα and Vβ sequences (termed TCR2.5D6) were cloned into the retroviral vector pMP71 and transduced into lymphocytes of a healthy donor. Multimer binding as well as functionality after TCR-gene transfer was confirmed (Figure 2D and E). Functional avidity of the TCR was tested using T2-B7 cells pulsed with serial dilutions of MPO₅. As shown in Figure 2F, half maximal secretion of IFN-γ was observed at concentrations of 150pM. Specific recognition of naturally processed and presented MPO₅ was analyzed using diverse B cell lines originally negative for MPO. In fact, C1R-B7 and BJAB-B7 cells were recognized when additionally transduced with the MPO gene verifying recognition of endogenously processed peptide (Figure 2G).

### Example 5

### Reactivity of TCR2.5D6-transduced lymphocytes in response to MPO-expressing leukemia cell lines as well as primary leukemia samples

All methods mentioned in this example were carried out as described in Examples 1 and 2 above.

Reactivity of TCR2.5D6-transduced lymphocytes against different leukemia cell lines and primary leukemia samples was assessed by IFN-γ ELISA. As shown in Figure 3A, only leukemia cell lines that show expression of MPO (Figure 9A, B), namely HL-60-B7, NB4-B7, ML-2-B7, were recognized by TCR2.5D6-transduced lymphocytes. MPO negative cell lines were only recognized when pulsed with MPO₅, demonstrating principal vulnerability of these cell lines by the defined TCR. No recognition by untransduced lymphocytes was observed.

Furthermore, IFN-γ production by TCR2.5D6-transduced lymphocytes in response to primary samples from patients with MPN and AML was analyzed (Figure 3B). In contrast to healthy mature myeloid cells, leukemia cells demonstrate partially high MPO-expression on both, mRNA and protein level (Figure 1 and Figure 9C, D). Reactivity of TCR2.5D6-transduced lymphocytes could only be observed against target cells expressing both HLA-B7 and MPO (MPN1, MPN2, MPN5, MPN6 and AML6). The MPO positive but HLA-B7 negative sample MPN3 and the MPO negative, HLA*-B*07:04⁺* sample AML2 were not recognized. Pulsing of AML2 with MPO₅ resulted in recognition of the sample by TCR2.5D6-transduced lymphocytes indicating a potential binding of MPO₅ also to the HLA-B7 subtype *B*07:04.*

To analyze the reactivity of TCR2.5D6 against colony forming leukemic precursor cells, either untransduced or TCR2.5D6-transduced lymphocytes were incubated with bulk PBMC of the HLA-B7 positive patient MPN2 at different effector to target ratios. A decrease in colony forming units (CFU) was observed, when cells of MPN2 (HLA-B7⁺, MPO⁺) were incubated with TCR2.5D6-transduced lymphocytes at effector to target ratios of 100:1 and 1000:1 (Figure 3C), whereas no reduction was detectable when cells were incubated with untransduced lymphocytes. As a control, untransduced and TCR2.5D6-transduced lymphocytes were incubated with cells of patient MPN3 (HLA-B7⁻, MPO⁺). No difference in the number of CFU was observed (Figure 3D). Taken together, these data demonstrate strong MPO₅-specific reactivity of TCR2.5D6-transduced lymphocytes against leukemic cells including leukemic *precursors in vitro.*

### Example 6

### TCR2.5D6-transduced CD8⁺T_{CM} prolong survival in a xenogeneic mouse model of human AML

All methods mentioned in this example were carried out as described in Examples 1 and 2 above.

*In vivo* tumor reactivity of TCR2.5D6-transduced CD8⁺T_{CM} was analyzed in a xenogeneic mouse model of human AML. 1x10⁶ NB4-B7 cells were injected into BRG mice, followed by injection of 10x10⁶ untransduced or TCR2.5D6-transduced CD8⁺T_{CM} or PBS alone. TCR expression and functionality of injected T cells is shown in Figure 10, details regarding the mouse model are shown in Figure 8. Mice were sacrificed when suffering from health problems as described in Example 2. Infiltration of bone marrow with CD45⁺CD3⁻CD8⁻ tumor cells was significantly reduced in mice that were treated with TCR2.5D6-transgenic T_{CM} compared to the untreated group (P = 0.0043) as well as the group that received untransduced T_{CM} (P = 0.0247) (Figure 4A). Furthermore, a significant prolonged overall survival of mice treated with TCR2.5D6-transduced CD8⁺T_{CM} was observed compared to mice that received untransduced CD8⁺T_{CM} (P = 0.0048) with median survival of 44 days versus 34 days, respectively. In contrast, there was no significant difference in median survival between the control group that received no T cells at all compared to the group that was treated with untransduced CD8⁺T_{CM} (37 days versus 34 days, P = 0.2229) (Figure 4B). All tumors were analyzed for expression of human CD45 and eGFP. CD45⁺, eGFP⁻ tumors were observed in 4 out of 5 mice in the group that was treated with TCR2.5D6-transduced CD8⁺T_{CM} (Figure 4C). Concomitant loss of HLA-B7 was confirmed whereas MPO expression was preserved (Figure 12). In contrast, tumors of all animals treated with untransduced T cells or no T cells showed preserved eGFP expression (Figure 4B). As shown in Figure 13, human TCRm⁺ T cells infiltrating bone marrow, tumor, lung and spleen could specifically be detected in the group that received TCR-transgenic T_{CM}. An additional experiment has been performed with clearly less favorable conditions as higher tumor load and later transfer of unselected lymphocytes with reduced TCR transduction rate (Figure 14A). Treatment still resulted in statistically significant reduction of bone marrow infiltration by leukemic cells (Figure 14B) as well a trend to improved survival (Figure 14C). In these animals, loss of GFP expression has not been observed (Figure 14D). Taken together, these data demonstrate *in vivo* efficacy of TCR2.5D6-transduced T_{CM} against leukemic cells and argue for a potent effect of this TCR against tumors with expression of both MPO and HLA-B7.

### Example 7

### On-target reactivity of lymphocytes transduced with the MPO₅ reactive TCR2.5D6

All methods mentioned in this example were carried out as described in Examples 1 and 2 above.

As it has been previously shown that on-target reactivity may induce unpredicted serious side effects, potential reactivity of TCR2.5D6-transduced lymphocytes against a number of different target cells including different HLA-B7⁺ hematopoietic cells was investigated. As shown in Figure 1 and Figure 15A and B, granulocytes as well as monocytes demonstrate relevant MPO protein but very low mRNA expression. Regarding recognition by TCR2.5D6-transduced lymphocytes, IFN-γ production was only detected at very low levels in co-cultures of healthy hematopoietic cells with TCR2.5D6-transduced lymphocytes unless the target cells were pulsed with MPO₅ (Figure 5A). In addition, the reactivity of TCR2.5D6-transduced lymphocytes against healthy, CD34⁺ hematopoietic stem cells of a HLA-B7⁺ donor was investigated in CFU assays. As shown in Figure 5B, the potential for formation of CFU by granulocyte-colony-stimulating factor (G-CSF) mobilized HSC was not reduced when HSC were incubated for 1 hour before plating in methylcellulose medium with TCR2.5D6-transduced lymphocytes compared to untransduced lymphocytes. Similar results were obtained when HSC were incubated for 24 hours with TCR2.5D6-transduced or untransduced lymphocytes (Figure 16A). In contrast, pulsing of target cells with MPO₅ resulted in a strong reduction in CFU when incubated with TCR2.5D6-transduced but not untransduced lymphocytes (Figure 5C). These data indicate the absence of reactivity of TCR2.SD6 against mature myeloid cells as well as normal HSC.

### Example 8

### Off-target reactivity of lymphocytes transduced with the MPO₅-specific TCR2.5D6

All methods mentioned in this example were carried out as described in Examples 1 and 2 above.

There have been reports in the literature of fatal side effects due to unpredicted off-target reactivity after transfer of tumor-reactive transgenic T cells containing an affinity-enhanced TCR. Although TCR2.5D6 was not affinity-enhanced, we sought to screen for peptide-dependent or independent recognition of diverse common HLA molecules. Therefore, different lymphoblastoid cell lines (LCL) were tested (Table 4) either unpulsed or pulsed with MPO₅ (Figure 6A). LCL1 is HLA-B7⁺ and served as positive control. No reactivity against cell lines was observed without pulsing of MPO₅. Following peptide pulsing, reactivity against LCL6 was observed (Figure 6A). *In silico* analysis of binding motifs for HLA molecules expressed by LCL6 revealed a potential binding of MPO₅ to HLA*-B*42:01* (HLA-B42). Therefore, the cell lines C1R-B42 as well as C1R-B42/MPO were generated. C1R cells were only detected after transduction of both, HLA-B42 and MPO, verifying on-target reactivity against MPO₅ also when presented by HLA-B42 (Figure 6B). Furthermore, off-target reactivity against different cell lines of non-hematopoietic origin as well as primary HRGEC not expressing MPO was tested (expression data: Figure 15C). As shown in Figure 6C, reactivity of TCR-transgenic lymphocytes could only be observed after pulsing of target cells with MPO₅, further strengthen the high specificity of the TCR2.5D6. In order to investigate potential peptide promiscuity of the TCR, experiments with alanine- and threonine-variants of MPO₅ loaded onto the HLA-B7⁺ cell line LCL1 were performed. As shown in Figure 6D, the residues at position 2, 3, 4, 5, 8 and 9 seem to be essential for recognition by TCR2.5D6. Similar results have been observed using two other HLA-B7⁺ LCL. *In silico* analysis using the ScanProsite tool revealed MPO being the only human protein containing the motif "XPRWDXXRL". The same has been observed when the HLA-B7⁺ cell lines LCL11 and LCL12 were used as target cells (figure 16B and C). Finally, crossreactivity was tested against 65 peptides, divided into 6 pools, which have been eluted from HLA-B7 molecules. As shown in Figure 6E, no reactivity could be observed at all, except against MPO₅. Thus, off-target reactivity could not be observed by these experiments.

The features of the present invention disclosed in the specification, the claims, and/or in the accompanying drawings may, both separately and in any combination thereof, be material for realizing the invention in various forms thereof.

### Tables

**Table 1. Identified HLA ligands derived from genes with restricted expression to the hematopoietic system**

| Gene | Number of HLA ligands | HLA Restriction |
|---|---|---|
| ELANE | 1 | *B*15:01* |
| HMHA1 | 2 | *B*07:02* |
| | | *B*15:01* |
| ITGA2B | 2 | *A*03:01* |
| | | *B*15:01* |
| LAT2 | 2 | *A*01:01* |
| | | *A*03:01* |
| MS3A3 | 2 | *B*07:02* |
| | | *A*03:01* |
| MPO | 5 | *A*01:01* |
| | | *B*07:02* |
| | | *B*15:01* |
| | | *B*44:02* |
| MYB | 5 | *B*07:02* |
| | | *B*15:01* |
| | | *B*18:01* |

**Table 2. Identified naturally presented HLA class I ligands of MPO**

| **Ligand** | **Sequence** | **HLA Restriction** | **Patient sample** | **Pep-Miner^{A}** | **Mascot^{B}** | **Peaks^{C}** |
|---|---|---|---|---|---|---|
| MPO₁ | EEAKQLVDKAY | *B *44:02* | MPN6 | √ | | √ |
| MPO₂ | TPAQLNVL | *B*07:02* | MPN1,5 | | | √ |
| MPO₃ | NQINALTSF | *B*15:01* | MPN2 | √ | √ | |
| MPO₄ | FVDASMVY | *A *01:01* | MPN3,4,5,7 | √ | | √ |
| MPO₅ | NPRWDGERL | *B*07:02* | MPN2,5 | | √ | √ |

| | | | | | | |
|---|---|---|---|---|---|---|
| ^{A}Score ≥ 70; ^{B}Score ≥ 30; ^{C}-logP10 ≥ 15 | | | | | | |

**Table 3. Patient characteristics**

| | **Age** | **Sex** | **Diagnosis** | **Molecular genetics** | **Previous therapy prior blood venipuncture** | **Stage/Type** | **Leukocytes** (x 10E9 G/L) | **HLA typing** |
|---|---|---|---|---|---|---|---|---|
| | | | | | | | **Hemoglobin** (g/dL) | |
| | | | | | | | **Platelets** (x 10E9 G/L) | |
| | | | | | | | At time of blood venipuncture | |
| MPN 1 | 74 | M | Atypical CML | JAK2, TET2, ASXL1, BCR/ABL-negative | Hydroxyurea | Blast crisis | 55 | *A*26:01, A*68:01* |
| | | | | | | | 8.7 | *B*07:02, B*58:01* |
| | | | | | | | 49 | |
| MPN 2 | 42 | M | CML | BCR/ABL, EVI, ETV | None | Blast crisis | 135 | *A*03:01, A*25:01* |
| | | | | | | | 14 | *B*07:02, B*15:01* |
| | | | | | | | 242 | |
| MPN 3 | 54 | F | CML | BCR/ABL | None | Chronic phase | 242 | *A*01:01, A*24:02* |
| | | | | | | | 8.7 | *B*08:01, B*18:01* |
| | | | | | | | 274 | |
| MPN 4 | 78 | M | CML | BCR/ABL (T315I) | Cytarabin | Blast crisis | 140 | *A*01:01, A*-:-* |
| | | | | | | | 8.6 | *B*08:01, B*-:-* |
| | | | | | | | 51 | |
| MPN 5 | 68 | M | CML | BCR/ABL | Imatinib, Nilotinib, Cytarabin, Etoposid | Blast crisis | 327 | *A*01:01, A*33:01* |
| | | | | | | | 9.4 | *B*07:02, B*14:02* |
| | | | | | | | 367 | |
| MPN 6 | 71 | M | PV | JAK2 | Hydroxyurea | Proliferative Phase | 71 | *A*24:02, A*32:01* |
| | | | | | | | 10.5 | *B*07:02, B*44:02* |
| | | | | | | | 376 | |
| MPN 7 | 68 | F | CML | BCR/ABL | None | Chronic phase | 124 | *A*01:01, A*03:01* |
| | | | | | | | 10.1 | *B*55:01, A*57:03* |
| | | | | | | | 110 | |
| MPN8 | 75 | M | CML | BCR/ABL, EVI | Litalir | Blast crisis 17% blasts | 311.5 | *ND* |
| | | | | | | | 7.4 | |
| | | | | | | | 450 | |
| MPN 9 | 80 | M | CML | BCR/ABL | None | Chronic phase | 332.4 | *ND* |
| | | | | | | | 8.9 | |
| | | | | | | | 365 | |
| MPN 10 | 61 | M | CML | BCR/ABL | None | Chronic phase | 54.9 | *ND* |
| | | | | | | | 12-1 | |
| | | | | | | | 384 | |
| MPN 11 | 61 | M | CML | BCR/ABL (T315I) | Imatinib, Dasatinib, Ponatinibm Hydroxyurea | Blast crisis | 8.25 | *A*02:01, A*03:01* |
| | | | | | | | 10.1 | *B*07:02, B*15:01* |
| | | | | | | | 26 | |
| MPN 12 | 30 | M | CML | BCR/ABL | None | Accelerated phase | 249 | *A*02:01, A*24:02* |
| | | | | | | | 8.2 | *B*15:01, B*-:-* |
| | | | | | | | 714 | |
| MDS/MPN1 | 76 | M | CMML | ASXL1, EZH2, NF 1 | Hydroxyurea | CMML | 37.5 | *ND* |
| | | | | | | | 9.6 | |
| | | | | | | | 503 | |
| MDS/MPN2 | 93 | M | CMML | ND | Hydroxyurea | CMML | 64.1 | *ND* |
| | | | | | | | 9 | |
| | | | | | | | 24 | |
| AML 1 | 37 | F | AML | NPM1 | None | M5a | 49.7 | *A*02:01, A*32:01* |
| | | | | | | | 8.4 | *B*14:02, B*27:05* |
| | | | | | | | 132 | |
| AML 2 | 33 | M | AML | 9q22, CBFB, PTEN, 1q25- | None | M5 | 207 | *A*01:01, A*03:01* |
| | | | | | | | 6.9 | *B*07:04, B*57:01* |
| | | | | | | | 42 | |
| AML 3 | 56 | M | AML | MLL-AF10 | Cytarabin, Idarubicin, Etoposid, Mitoxantron, allogeneic SCT | M2 | 2.25 | *A*01:01, A*02:01* |
| | | | | | | | 9.6 | *B*44:02, B*44:03* |
| | | | | | | | 90 | |
| AML 4 | 44 | F | AML | FLT3-ITD, MLL-PTD | None | M2 | 47.1 | *A*01:01, B*66:01* |
| | | | | | | | 8.7 | *B*08:01, B*35:02* |
| | | | | | | | 29 | |
| AML 5 | 56 | M | AML | CBFB/MY111, Inv16 | None | M4Eo | 22.6 | *ND* |
| | | | | | | | 10.6 | |
| | | | | | | | 22 | |
| AML 6 | 21 | F | AML | CBFB/MYH11, Inv16, FLT-TKD | None | M4Eo | 64.54 | *A*01:01, A*33:03* |
| | | | | | | | 7.1 | *B*07:02, B*58:01* |
| | | | | | | | 7 | |
| AML 7 | 53 | F | AML | FLT3-ITD, NPM1 | Hydroxyurea | M1 | 80.1 | *A*01:01, A*24:02* |
| | | | | | | | 8.5 | *B*08:01, B*38:01* |
| | | | | | | | 38 | |
| CLL 1 | 77 | M | B-CLL | ND | Fludarabine, Cyclophosphamide | Binet C | 303 | *ND* |
| | | | | | | | 9 | |
| | | | | | | | 357 | |
| CLL 2 | 69 | F | B-CLL | ND | None | Binet A | 93.1 | *ND* |
| | | | | | | | 13.3 | |
| | | | | | | | 213 | |
| CLL 3 | 77 | F | B-CLL | ND | Chlorambucil | Binet C | 81.7 | *ND* |
| | | | | | | | 11.5 | |
| | | | | | | | 96 | |

**Table 4. HLA type of lymphoblastoid cell lines used for stimulation experiments**

| **Cell line** | **IHW^{#} number** | **HLA-A*** | **HLA-B*** | **HLA-C*** |
|---|---|---|---|---|
| LCL1 | - | *02:01*/ *--* | *07:02*/*15:01* | *03:04*/*12:03* |
| LCL2 | IHW09005 | *03:01*/ *--* | *27:05*/ *--* | *01:02*/ *--* |
| LCL3 | IHW09037 | *29:02*/ *--* | *40:02*/ *--* | *02:02*/ *--* |
| LCL4 | IHW09064 | *02:17*/ *--* | *15:01*/ *--* | *03:03*/ *--* |
| LCL5 | IHW09216 | *02:09*/*03:01* | *35:01*/*38:01* | *04:01*/*12:03* |
| LCL6 | IHW09021 | *68:02*/*30:01* | *42:01*/ *--* | *17:01*/ *--* |
| LCL7 | IHW09213 | *02:08*/*--* | *50:01*/*08:01* | *07*/*06: 02* |
| LCL8 | IHW09079 | *33:01*/*--* | *14:02*/ *--* | *08:02*/ *--* |
| LCL9 | IHW09016 | *02:04*/*--* | *51:01*/ *--* | *15:02*/ *--* |
| LCL10 | IHW09043 | *01:01*/*--* | *41:01*/ *--* | *17:01l --* |

| | | | | |
|---|---|---|---|---|
| ^{#}International HLA workshop | | | | |

**Table 5. Antibodies used for flow cytometry**

| **Name** | **Host** | **Specificity** | **Clone** | **Labeling** | **Company** |
|---|---|---|---|---|---|
| CD3 | Mouse | Human | UCHT1 | FITC/ Alexa Fluor 700 | BD |
| CD4 | Mouse | Human | RPA-T4 | FITC/APC/V450 | BD |
| CD8 | Mouse | Human | RPA-T8 | FITC/APC/V450 | BD |
| CD14 | Mouse | Human | M5E2 | PE | BD |
| CD34 | Mouse | Human | 563 | PE | BD |
| CD45RO | Mouse | Human | UCHL1 | PE | BD |
| CD45RA | Mouse | Human | HI100 | APC | BD |
| TCRm | Armenian hamster | Mouse | H57-597 | FITC/PE | BD |
| MPO | Rabbit | Human | 1B10 | Purified | BD |
| IgG1 | Rabbit | Mouse | A85-1 | FITC | BD |
| HLA-B*07 | Mouse | Human | BB7.1 | PE | Millipore |

### References

Bassani-Sternberg M, Barnea E, Beer I, Avivi I, Katz T, Admon A. Feature Article: Soluble plasma HLA peptidome as a potential source for cancer biomarkers. Proc Natl Acad Sci U S A. 2010. Epub 2010/10/27.
Beer I, Barnea E, Ziv T, Admon A. Improving large-scale proteomics by clustering of mass spectrometry data. Proteomics. 2004;4(4):950-60.
Bonini C, Ferrari G, Verzeletti S, Servida P, Zappone E, Ruggieri L, et al. HSV-TK gene transfer into donor lymphocytes for control of allogeneic graft-versus-leukemia. Science. 1997;276(5319):1719-24. Epub 1997/06/13.
Boulter JM and Jakobsen BK. Stable, soluble, high-affinity, engineered T cell receptors: novel antibody-like proteins for specific targeting of peptide antigens. Clin Exp Immunol. 2005; 142(3):454-460.
Cohen CJ, Zhao Y, Zheng Z, Rosenberg SA, Morgan RA. Enhanced antitumor activity of murine-human hybrid T cell receptor (TCR) in human lymphocytes is associated with improved pairing and TCR/CD3 stability. Cancer Res. 2006;66(17):8878-86. Epub 2006/09/05.
Csoka M, Strauss G, Debatin KM, Moldenhauer G. Activation of T cell cytotoxicity against autologous common acute lymphoblastic leukemia (cALL) blasts by CD3xCD19 bispecific antibody. Leukemia 1996 Nov;10(11):1765-72.
Eggleton P, Gargan R, Fisher D. Rapid method for the isolation of neutrophils in high yield without the use of dextran or density gradient polymers. Journal of immunological methods. 1989;121(1):105-13.
Engels B, Cam H, Schüler T, Indraccolo S, Gladow M, Baum C, Blankenstein T, Uckert W. Retroviral vectors for high-level transgene expression in T lymphocytes. Hum Gene Ther. 2003 Aug 10;14(12):1155-68.
Falk K, Rotzschke O, Stevanovic S, Jung G, Rammensee HG. Allele-specific motifs revealed by sequencing of self-peptides eluted from MHC molecules. Nature. 1991;351(6324):290-6. Falk K, Rotzschke O, Takiguchi M, Gnau V, Stevanovic S, Jung G, et al. Peptide motifs of HLA-B51, -B52 and -B78 molecules, and implications for Behcet's disease. International immunology. 1995;7(2):223-8.
Fehse B, Kustikova OS, Li Z, Wahlers A, Bohn W, Beyer WR, et al. A novel 'sort-suicide' fusion gene vector for T cell manipulation. Gene Therapy 2002;9(23):1633-8.
Hamid O et al. Safety and tumor responses with lambrolizumab (anti-PD-1) in melanoma. N Engl JMed. 2013 Jul 11;369(2):134-44.
Han EQ, Li XL, Wang CR, Li TF, Han SY. Chimeric antigen receptor-engineered T cells for cancer immunotherapy: progress and challenges. J Hematol Oncol. 2013 Jul 8;6:47.
Hauck SM, Dietter J, Kramer RL, Hofmaier F, Zipplies JK, Amann B, et al. Deciphering membrane-associated molecular processes in target tissue of autoimmune uveitis by label-free quantitative mass spectrometry. Mol Cell Proteomics. 2010;9(10):2292-305. Epub 2010/07/06.
Klar R, Schober S, Rami M, Mall S, Merl J, Hauck SM, et al. Therapeutic targeting of naturally presented myeloperoxidase-derived HLA peptide ligands in myeloid leukemia cells by TCR-transgenic T cells. Leukemia. 2014:1-12. Epub 2014/05/13.
Knabel M, Franz TJ, Schiemann M, Wulf A, Villmow B, Schmidt B, et al. Reversible MHC multimer staining for functional isolation of T cell populations and effective adoptive transfer. Nat Med. 2002;8(6):631-7. Epub 2002/06/04.
Koristka S, Cartellieri M, Feldmann A, Arndt C, Loff S, Michalk I, Aliperta R, von Bonin M, Bornhäuser M, Ehninger A, Ehninger G, and Bachmann MP. Flexible Antigen-Specific Redirection of Human Regulatory T Cells Via a Novel Universal Chimeric Antigen Receptor System. 56th American Society of Hematology Annual Meeting and Exposition (December 6-9, 2014), Conference Program Book, Oral and Poster Abstracts, Session 801, No. 3494 (available also from https://ash.confex.com/ash/2014/webprogram/Paper72149.html).
Kuball J, Dossett ML, Wolfl M, Ho WY, Voss RH, Fowler C, et al. Facilitating matched pairing and expression of TCR chains introduced into human T cells. Blood. 2007;109(6):2331-8. Epub 2006/11/04.
Liang X, Weigand LU, Schuster IG, Eppinger E, van der Griendt JC, Schub A, et al. A single TCR alpha-chain with dominant peptide recognition in the allorestricted HER2/neu-specific T cell repertoire. J Immunol. 2010;184(3):1617-29. Epub 2010/01/01.
Linette GP, Stadtmauer EA, Maus MV, Rapoport AP, Levine BL, Emery L, et al. Cardiovascular toxicity and titin cross-reactivity of affinity-enhanced T cells in myeloma and melanoma. Blood. 2013;122(6):863-71. Epub 2013/06/19.
Livak KJ, Schmittgen TD. Analysis of relative gene expression data using real-time quantitative PCR and the 2(-Delta Delta C(T)) Method. Methods. 2001;25(4):402-8. Epub 2002/02/16.
Lunde E, Løset GÅ, Bogen B, Sandlie I. Stabilizing mutations increase secretion of functional soluble TCR-Ig fusion proteins. BMC Biotechnology 2010, 10:61.
Mandal PK, Ferreira LM, Collins R, Meissner TB, Boutwell CL, Friesen M, et al. Efficient ablation of genes in human hematopoietic stem and effector cells using CRISPR/Cas9. Cell Stem Cell 2014;15(5):643-52.
Manur PV, Wilson MH, Maiti SN, Mi T, Singh H, et al. piggyBac transposon/transposase system to generate CD19-specific T cells for the treatment of B-lineage malignancies. Hum Gen Ther. 2010 Apr;21(4):427-37.
Maiti SN, Huls H, Singh H, Dawson M, Figliola M, Olivares S, Rao P, Zhao YJ, Multani A, Yang G, Zhang L, Crossland D, Ang S, Torikai H, Rabinovich B, Lee DA, Kebriaei P, Hackett P, Champlin RE, Cooper LJ. Sleeping beauty system to redirect T cell specificity for human applications. J Immunother. 2013 Feb;36(2):112-23.
Miyoshi H, Blömer U, Takahashi M, Gage FH, Verma IM. Development of a self-inactivating lentivirus vector. J Virol. 1998 Oct;72(10):8150-7.
Niedermeyer TH, Strohalm M. mMass as a software tool for the annotation of cyclic peptide tandem mass spectra. PloS one. 2012;7(9):e44913.
Schirle M, Keilholz W, Weber B, Gouttefangeas C, Dumrese T, Becker HD, et al. Identification of tumor-associated MHC class I ligands by a novel T cell-independent approach. Europeanjournal of immunology. 2000;30(8):2216-25.
Scholten KB, Kramer D, Kueter EW, Graf M, Schoedl T, Meijer CJ, et al. Codon modification of T cell receptors allows enhanced functional expression in transgenic human T cells. Clin Immunol. 2006;119(2):135-45. Epub 2006/02/07.
Schuster IG, Busch DH, Eppinger E, Kremmer E, Milosevic S, Hennard C, et al. Allorestricted T cells with specificity for the FMNL1-derived peptide PP2 have potent antitumor activity against hematologic and other malignancies. Blood. 2007;110(8):2931-9.
Urbanska K, Lanitis E, Poussin M, Lynn RC, Gavin BP, Kelderman S, et al. A universal strategy for adoptive immunotherapy of cancer through use of a novel T-cell antigen receptor. Cancer Res. 2012 Apr 1;72(7):1844-52.
Volpe G, Cignetti A, Panuzzo C, Kuka M, Vitaggio K, Brancaccio M, et al. Alternative BCR/ABL splice variants in Philadelphia chromosome-positive leukemias result in novel tumor-specific fusion proteins that may represent potential targets for immunotherapy approaches. Cancer research. 2007;67(11):5300-7.
Voss RH, Thomas S, Pfirschke C, Hauptrock B, Klobuch S, Kuball J, et al. Coexpression of the T-cell receptor constant alpha domain triggers tumor reactivity of single-chain TCR-transduced human T cells. Blood. 2010;115(25):5154-63.
Wang X, Berger C, Wong CW, Forman SJ, Riddell SR, Jensen MC. Engraftment of human central memory-derived effector CD8+ T cells in immunodeficient mice. Blood. 2011;117(6):1888-98. Epub 2010/12/03.
Wang X, Naranjo A, Brown CE, Bautista C, Wong CW, Chang WC, et al. Phenotypic and functional attributes of lentivirus-modified CD19-specific human CD8+ central memory T cells manufactured at clinical scale. Journal of immunotherapy. 2012;35(9):689-701.
Weigand LU, Liang X, Schmied S, Mall S, Klar R, Stotzer OJ, et al. Isolation of human MHC class II -restricted T cell receptors from the autologous T cell repertoire with potent anti-leukaemic reactivity. Immunology. 2012;137(3):226-38. Epub 2012/10/03.
Wilde S, Sommermeyer D, Frankenberger B, Schiemann M, Milosevic S, Spranger S, et al. Dendritic cells pulsed with RNA encoding allogeneic MHC and antigen induce T cells with superior antitumor activity and higher TCR functional avidity. Blood. 2009;114(10):2131-9. Yu SF, von Rüden T, Kantoff PW, Garber C, Seiberg M, Rüther U, Anderson WF, Wagner EF, Gilboa E. Self-inactivating retroviral vectors designed for transfer of whole genes into mammalian cells. Proc Natl Acad Sci U S A 1986 May;83(10):3194-8.
Zhang J, Xin L, Shan B, Chen W, Xie M, Yuen D, et al. PEAKS DB: de novo sequencing assisted database search for sensitive and accurate peptide identification. Molecular & cellular proteomics : MCP. 2012;11(4):M111 010587.

## Claims

1. A T cell receptor (TCR) or a fragment thereof comprising:
(i) a T cell receptor α-chain or a fragment thereof comprising the amino acid sequence of SEQ ID NO: 3 *(CDR3 of T cell receptor α*-*chain*) or an amino acid sequence that has at least 75% identity, more preferably at least 83% identity, more preferably at least 91% identity with the amino acid sequence of SEQ ID NO: 3;
and
(ii) a T cell receptor β-chain or a fragment thereof comprising the amino acid sequence of SEQ ID NO: 6 *(CDR3 of T cell receptor β-chain)* or an amino acid sequence that has at least 76% identity, more preferably at least 84% identity, more preferably at least 92% identity with the amino acid sequence of SEQ ID NO: 6.

2. The T cell receptor or fragment thereof according to claim 1,
wherein said T cell receptor or said fragment thereof is capable of binding to a peptide having the amino acid sequence of SEQ ID NO: 7 *(MPO₄₆₆₋₄₇₄ peptide)* bound to HLA-B**07:02* (Human Leukocyte Antigen, allele B**07:02*) and/or to a peptide having the amino acid sequence of SEQ ID NO: 7 bound to HLA-B**07:04* and/or to a peptide having the amino acid sequence of SEQ ID NO: 7 bound to *HLA*-B**42:01,* more preferably to a peptide having the amino acid sequence of SEQ ID NO: 7 bound to HLA-B **07*:*02;*
and/or wherein said T cell receptor α-chain or said fragment thereof further comprises
- the amino acid sequence of SEQ ID NO: 1 (*CDR1 of T cell receptor α*-*chain*) or an amino acid sequence that has at least 83% identity with the amino acid sequence of SEQ ID NO: 1,
and/or
- the amino acid sequence of SEQ ID NO: 2 *(CDR2 of T cell receptor α*-*chain*) or an amino acid sequence that has at least 75% identity, more preferably at least 87% identity with the amino acid sequence of SEQ ID NO: 2;
and/or wherein said T cell receptor β-chain or said fragment thereof further comprises
- the amino acid sequence of SEQ ID NO: 4 *(CDR1 of T cell receptor β-chain)* or an amino acid sequence that has at least 80% identity with the amino acid sequence of SEQ ID NO: 4,
and/or
- the amino acid sequence of SEQ ID NO: 5 *(CDR2 of T cell receptor β-chain)* or an amino acid sequence that has at least 83% identity with the amino acid sequence of SEQ ID NO: 5;
and/or wherein
a) said T cell receptor α-chain (or said fragment thereof) and/or said T cell receptor β-chain (or said fragment thereof) comprise or are composed of human sequences or
b) said T cell receptor α-chain (or said fragment thereof) and/or said T cell receptor β-chain (or said fragment thereof) comprise or are composed of mouse sequences or
c) said T cell receptor α-chain (or said fragment thereof) and/or said T cell receptor β-chain (or said fragment thereof) are each composed of a combination of human and mouse sequences, wherein, preferably, the constant region of said T cell receptor α-chain and the constant region of said T cell receptor β-chain are composed of mouse sequences and, preferably, the variable region of said T cell receptor α-chain and the variable region of said T cell receptor β-chain are composed of human sequences.

3. The T cell receptor or fragment thereof according to claim 1 or 2, comprising
(i) a T cell receptor α-chain having an amino acid sequence comprising the amino acid sequence of SEQ ID NO: 8 or an amino acid sequence that has at least 80% identity, more preferably at least 90% identity, more preferably at least 95% identity, with the amino acid sequence of SEQ ID NO: 8,
and/or
(ii) a T cell receptor β-chain having an amino acid sequence comprising the amino acid sequence of SEQ ID NO: 9 or an amino acid sequence that has at least 80% identity, more preferably at least 90% identity, more preferably at least 95% identity, with the amino acid sequence of SEQ ID NO: 9;
or
said fragment of said T cell receptor comprises
(i) a fragment of a T cell receptor α-chain having the amino acid sequence of SEQ ID NO: 8 or of an amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 8;
and/or
(ii) a fragment of a T cell receptor β-chain having the amino acid sequence of SEQ ID NO: 9 or of an amino acid sequence that has at least 80% identity, more preferably at least 85% identity, more preferably at least 90% identity, more preferably at least 95% identity, more preferably at least 98% identity, more preferably at least 99% identity, with the amino acid sequence of SEQ ID NO: 9.

4. A soluble T cell receptor (sTCR) construct comprising
(1) a T cell receptor component comprising
at least one T cell receptor α-chain (or fragment thereof) as defined in any of claims 1-3 and
at least one T cell receptor β-chain (or fragment thereof) as defined in any of claims 1-3,
preferably covalently linked to each other to form a TCR heterodimer or multimer, and
(2) fusion component(s)
preferably selected from
- an Fc receptor,
- an Fc domain of an antibody,
- a cytokine, preferably IL-2 or IL-15,
- a toxin,
- an antibody, preferably selected from the group consisting of an anti-CD3 antibody, an anti-CD28 antibody, an anti-CD5 antibody, an anti-CD16 antibody and an anti-CD56 antibody,
- a linker specific chimeric antigen receptor (CAR),
or combinations thereof,
wherein said at least one T cell receptor α-chain (or said fragment thereof) of (1) and/or said at least one T cell receptor β-chain (or that fragment thereof) of (1) is bound to the fusion component(s) of (2),
and wherein, preferably, the soluble T cell receptor construct furthermore comprises a label.

5. A chimeric T cell receptor or fragment thereof comprising at least one T cell receptor or fragment thereof according to any of claims 1-3, wherein the T cell receptor α-chain (or fragment thereof) and/or the T cell receptor β-chain (or fragment thereof) is/are fused, preferably via a linker, to CD3-zeta chain(s) and/or one or more other T cell receptor stimulation domains, preferably selected from the group consisting of the intracellular CD28, CD137 or CD134 domains.

6. A bi-specific antibody comprising
(a) a T cell receptor component comprising the T cell receptor α-chain (or fragment thereof) and the T cell receptor β-chain (or fragment thereof) of a T cell receptor (or fragment thereof) according to any of claims 1-3,
wherein said T cell receptor α-chain (or fragment thereof) and said T cell receptor β-chain (or fragment thereof) are linked to each other
and fused, preferably via a linker, to
(b) an antibody or a single chain antibody fragment (scFv) or a linker specific chimeric antigen receptor (CAR), preferably an antibody, which is directed against an antigen or epitope on the surface of lymphocytes.

7. A nucleic acid encoding
- the amino acid sequence of a T cell receptor or fragment thereof according to any of claims 1-3,
- the amino acid sequence of a soluble T cell receptor construct according to claim 4,
- the amino acid sequence of a chimeric T cell receptor or fragment thereof according to claim 5, or
- the amino acid sequence of the T cell receptor component of a bi-specific antibody
according to claim 6;
or
- a set of nucleic acids, one of which encodes the amino acid sequence of the T cell receptor α-chain (or fragment thereof) and one of which the amino acid sequence of the T cell receptor β-chain (or fragment thereof) of a T cell receptor or fragment thereof according to any of claims 1-3;
- a set of nucleic acids, one of which encodes the amino acid sequence of the T cell receptor α-chain (or fragment thereof) and one of which the amino acid sequence of the T cell receptor β-chain (or fragment thereof) of a soluble T cell receptor construct as defined in claim 4;
- a set of nucleic acids, one of which encodes the amino acid sequence of the T cell receptor α-chain (or fragment thereof) and one of which the amino acid sequence of the T cell receptor β-chain (or fragment thereof) of a chimeric T cell receptor or fragment thereof as defined in claim 5; or
- a set of nucleic acids, one of which encodes the amino acid sequence of the T cell receptor α-chain (or fragment thereof) and one of which the amino acid sequence of the T cell receptor β-chain (or fragment thereof) of the T cell receptor component of a bi-specific antibody as defined in claim 6.

8. An expression construct for expressing the T cell receptor (or fragment thereof) according to any of claims 1-3, the soluble T cell receptor construct according to claim 4, the chimeric T cell receptor (or fragment thereof) according to claim 5 or the bi-specific antibody according to claim 6 in a host cell.

9. A cell comprising the T cell receptor (or fragment thereof) according to any of claims 1-3, the soluble T cell receptor construct according to claim 4, the chimeric T cell receptor (or fragment thereof) according to claim 5, the bi-specific antibody according to claim 6, the nucleic acid or set of nucleic acids according to claim 7 or the expression construct according to claim 8.

10. The cell according to claim9, wherein said cell is a lymphocyte, preferably a T cell, more preferably a CD3⁺ T cell or CD8⁺ T cell.

11. A pharmaceutical composition comprising one or more of:
(i) the T cell receptor (or fragment thereof) according to any of claims 1-3;
(ii) the soluble T cell receptor construct according to claim 4;
(iii) the chimeric T cell receptor (or fragment thereof) according to claim 5;
(iv) the bi-specific antibody according to claim 6;
(v) the nucleic acid or set of nucleic acids according to claim 7 or the expression construct according to claim 8; and/or
(vi) the cell according to claim 9 or 10,
and, optionally, pharmaceutical excipient(s).

12. Use of a T cell receptor (or fragment thereof) according to any of claims 1-3, a chimeric T cell receptor (or fragment thereof) according to claim 5, a bi-specific antibody according to claim 6, a nucleic acid or set of nucleic acids according to claim 7 or an expression construct according to claim 8 for generating genetically modified lymphocytes, preferably genetically modified T cells, more preferably genetically modified CD3⁺ T cells or CD8⁺ T cells, wherein, preferably, the generated genetically modified lymphocytes are for use in immunotherapy or donor lymphocyte infusions or purging in the context of induction or consolidation therapy with autologous modified cells.

13. The T cell receptor (or fragment thereof) according to any of claims 1-3, the soluble T cell receptor construct according to claim 4, the chimeric T cell receptor (or fragment thereof) according to claim 5, the bi-specific antibody according to claim 6, the nucleic acid or set of nucleic acids according to claim 7, the expression construct according to claim 8, the cell according to claim 9 or 10 or the pharmaceutical composition according to claim 11 for use in the detection, diagnosis or prognosis of a hematological neoplasia, preferably of leukemia, more preferably of a disease selected from the group consisting of myeloproliferative neoplasia (MPN), acute leukemia (preferably acute myeloid leukemia (AML)), chronic leukemia (preferably chronic myeloid leukemia (CML)), acute lymphoblastic leukemia (ALL), myelodysplastic syndrome and myeloproliferative syndrome, even more preferably of acute myeloid leukemia (AML).

14. The T cell receptor (or fragment thereof) according to any of claims 1-3, the soluble T cell receptor construct according to claim 4, the chimeric T cell receptor (or fragment thereof) according to claim 5, the bi-specific antibody according to claim 6, the nucleic acid or set of nucleic acids according to claim 7, the expression construct according to claim 8, the cell according to claim 9 or 10 or the pharmaceutical composition according to claim 11 for use as a medicament, preferably in the prevention and/or treatment of an MPO-positive hematological neoplasia, preferably of MPO-positive leukemia, more preferably of a disease selected from the group consisting of myeloproliferative neoplasia (MPN), MPO-positive acute leukemia (preferably acute myeloid leukemia (AML)), MPO-positive chronic leukemia (preferably chronic myeloid leukemia (CML)), MPO-positive acute lymphoblastic leukemia (ALL), myelodysplastic syndrome and myeloproliferative syndrome, even more preferably of acute myeloid leukemia (AML).

15. The T cell receptor (or fragment thereof), the soluble T cell receptor construct, the chimeric T cell receptor (or fragment thereof), the bi-specific antibody, the nucleic acid or set of nucleic acids, the expression construct, the cell or the pharmaceutical composition according to claim 14, wherein said treatment involves administration of said T cell receptor (or fragment thereof), soluble T cell receptor construct, chimeric T cell receptor (or fragment thereof), bi-specific antibody, nucleic acid or set of nucleic acids, expression construct, cell or pharmaceutical composition to a patient in need thereof, wherein said treatment involves allogeneic stem cell transplantation (SCT) from a donor to said patient and/or wherein said patient is HLA-B**07:02* positive and/or HLA-B**07:04* positive and/or *HLA-*B**42:01* positive, more preferably HLA-*B***07:02* positive.
